# EUROPEAN PATENT SPECIFICATION

(11) **EP 4 590 684 B1**
(45) Date of publication and mention of the grant of the patent: **05.08.2026**
(21) Application number: 23772502.3
(22) Date of filing: 18.09.2023
(51) Int. Cl.: C07H 1/00, C07H 21/00, C07H 21/02

(54) **IMPROVED OLIGONUCLEOTIDE SYNTHESIS**
VERBESSERTE OLIGONUKLEOTIDSYNTHESE
SYNTHÈSE AMÉLIORÉE D'OLIGONUCLÉOTIDES

(30) Priority: 19.09.2022 EP 22196404
(43) Date of publication of application: 30.07.2025
(73) Proprietor: Bachem Holding AG, 4416 Bubendorf (CH)
(72) Inventor: CREUSEN, Guido, 4127 Birsfelden (CH); MINUTH, Marco, 4416 Bubendorf (CH); SAMSON, Daniel, 4416 Bubendorf (CH)
(74) Representative: Jacobi, Markus Alexander
(86) International application number: PCT/EP2023/075684
(87) International publication number: WO 2024/061842

(56) References cited:
- WO-A1-2022/175211
- WO-A1-96/03417
- US-A1- 2021 309 690

## Description

The invention generally relates to the field of oligonucleotide synthesis, in particular solid-phase oligonucleotide synthesis, at an industrial or laboratory scale. Improved methods for solid-phase synthesis of oligonucleotides are disclosed. The invention is directed to methods of effectively removing a (temporary) protecting group comprising an optionally substituted triarylmethyl residue, preferably a di(p-methoxyphenyl)phenylmethyl protecting group, during solid-phase oligonucleotide synthesis. Further aspects of the invention are directed to compositions and the use thereof for removing a protecting group comprising an optionally substituted triarylmethyl residue, preferably a di(p-methoxyphenyl)phenylmethyl protecting group, from a hydroxyl moiety during the chemical synthesis, in particular solid-phase synthesis, of an oligonucleotide.

Oligonucleotide synthesis typically comprises one or more coupling steps, during each of which a free (i.e. unprotected) hydroxyl group of a first nucleoside or oligonucleotide is reacted with a phosphorus moiety of second nucleoside or oligonucleotide, which typically comprises a protected backbone hydroxyl moiety. Protection of this backbone hydroxyl moiety is required to avoid double insertion of said second nucleoside or oligonucleotide and/or multimerization. Prior to the next coupling step, this hydroxyl moiety is typically deprotected to then engage in the next condensation reaction with the phosphorus moiety of a third nucleoside or oligonucleotide, and so on. The (hydroxyl) protecting groups cleaved prior to each condensation reaction (i.e. once per coupling cycle) are commonly referred to as temporary protecting groups. Typically, functional groups (e.g. exocyclic amino groups) in the nucleobases and other functional groups within the carbohydrate (e.g. ribose or 2'-deoxyribose) moieties or internucleosidic linkage groups are also protected during chemical oligonucleotide synthesis. However, since these protecting groups are typically only removed once all coupling cycles have been performed, they are commonly referred to as permanent protecting groups.

The temporary and the permanent protecting groups are typically orthogonal to each other, meaning that one type can be removed under conditions, which do not affect the other type of protecting group. Typically, the permanent protecting groups are cleavable under alkaline conditions (i.e. by treatment with a base) and the temporary protecting groups are cleavable under acidic conditions (i.e. by treatment with an acid).

To facilitate separation from the reagents after each step, the growing oligonucleotide chain is commonly bound to a solid support (vide infra).

Typically, protecting groups comprising an optionally substituted triarylmethyl residue, herein also referred to as triarylmethyl type protecting groups, in particular the di(*p*-methoxyphenyl)phenylmethyl (DMT) group, are used as temporary (hydroxyl) protecting groups. The step of removing the (temporary) triarylmethyl type protecting groups (also referred to as detritylation step) has proven surprisingly challenging for the synthesis of high quality oligonucleotides. One side reaction associated with acid-induced cleavage of triarylmethyl type (hydroxyl) protecting groups is acid-induced nucleobase cleavage (i.e. one or more nucleobases are cleaved from the backbone of the oligonucleotide). This side reaction is particularly pronounced with purine type nucleobases such as guanine and adenine. Nucleobase cleavage of purine type nucleobases (i.e. nucleobases comprising an optionally substituted purine residue) is typically referred to as depurination. To avoid nucleobase cleavage, in particular depurination, various strategies have been explored. Nucleobase protecting groups reducing the basicity, alternative temporary protecting groups, and coupling schemes using di- and trinucleotides have been tested, but none of these approaches has proven its value for routine oligonucleotide synthesis.

Habus and Agrawal (WO 96/03417 and Nucleic Acids Research 1994, 22(20), 4350-4351) teach that a detritylation agent comprising 2 % dicholoroacetic acid (DCA) and 0.1 % of a lower alcohol (i.e. a C₁-C₆₋alcohol) such as ethanol or methanol and/or 0.1 to 1 % 1*H*-pyrrole in dichloromethane (DCM) suppresses depurination during solid-phase oligonucleotide synthesis. It was speculated that said lower alcohol and/or 1*H*-pyrrole acted bifunctionally - as proton scavenger decreasing the acidity of the acid and as carbocation scavenger suppressing the reaction of the triarylmethyl cation, in particular the DMT cation, with the deprotected hydroxyl group (WO 96/03417). The use of methanol or ethanol alongside a protic acid for detritylation of oligonucleotides has, for example, also been suggested by Krotz and Ravikumar (US7273933B1) as well as by Hargreaves and Leproust (US20080058511), albeit not in the context of undesired nucleobase cleavage. The polyfluorinated alcohols trifluoroethanol, hexafluoroisopropanol, pentafluoropropanol, 1,1,1,3,3,3-hexafluoro-2-methyl-2-propanol, and nonafluoro tertiary butyl alcohol have also been used in detritylation cocktails for the suppression of depurination (WO 2022/195111 A1).

H. Takaku et al. (Chemistry Letters 1983, 12(11), 1661-1664) found that a detritylation agent consisting of 3 % trichloroacetic acid (TCA) in nitromethane/methanol (95:5) gave less depurination than a detritylation agent consisting of 3 % TCA in DCM.

Hirai and Katayama (EP2711370A1) investigated the cleavage of a DMT protecting group from the 5'-hydroxyl moiety of a nucleoside bonded to a soluble support (a pseudo solid-phase protecting group) via its 3'-hydroxyl moiety using trifluoroacetic acid (TFA, 394 µmol) in DCM (350 µL), in the presence of various cation scavengers (each 162 µmol), including methanol and *m*-cresol (cf., e.g., [0199]-[0205] of this reference). In contrast to methanol, *m*-cresol was found not to sufficiently function as cation scavenger.

Arya et al. (Bioconjugate Chemistry 2007, 18(1), 160-169) reported a synthesis of an oligonucleotide-neomycin conjugate through solid-phase oligonucleotide synthesis (SPOS) using 4 % trichloroacetic acid in DCM for 5'-O-detritylation in all coupling cycles. After cleavage from the support, the 5'-terminal DMT group was removed alongside the *tert*-butyloxycarbonyl (Boc) amino protecting groups at the neomycin moiety. For this specific reaction, a mixture of 1,4-dioxane, trifluoroacetic acid (TFA), and *m*-cresol (96:3:1, v/v/v; approx. 95 mmol/L of *m*-cresol) was used.

Cheruvallath (Organic Process Research & Development 2003, 7, 917-920) and Septak (Nucleic Acids Research, 1996, 24(15), 3053-3058) both teach to increase the concentration of dichloroacetic acid in the detritylation reagent from 3 % to 10 % or 15 %, respectively, and to keep the contact time between detritylation reagent and oligonucleotide minimal in order to favor detritylation over depurination. This approach is still used for the industrial scale production of oligonucleotides such as those exemplarily usable as active pharmaceutical ingredients (APIs). However, having to limit the contact time between the detritylation reagent and the oligonucleotide severely limits the scalability of the process, because the maximum pumping capacity of the liquid handling systems is limited.

There is a long felt, still unsatisfied need for a solid-phase synthesis method to deprotect the (backbone-) hydroxyl group of a growing oligonucleotide chain, while suppressing undesired nucleobase cleavage, in particular depurination, wherein this method is preferably scalable and amenable to the large scale production of oligonucleotides, for example in column reactors and/or in batch reactors. To be of use in industrial production, such a method should not only suppress undesired nucleobase cleavage, in particular depurination, but should also result in an acceptable product yield. Preferably such a method would not rely on the use of halogenated solvents such as DCM.

The present invention provides a solution to this problem.

One aspect of the invention pertains to a method for the solid-phase synthesis of a target oligonucleotide O^{T} comprising a step (b) of incubating a nucleoside or oligonucleotide, which is covalently linked to a solid support and comprises a backbone hydroxyl moiety protected by a protecting group PG-0 comprising an optionally substituted triarylmethyl residue, with a deprotection mixture M-b, thereby cleaving the protecting group PG-0 from the nucleoside or oligonucleotide, wherein said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety.

One aspect of the invention pertains to a method for the solid-phase synthesis of a target oligonucleotide O^{T} comprising a step (b) of incubating a nucleoside or oligonucleotide, which is covalently linked to a solid support and comprises a backbone hydroxyl moiety protected by a protecting group PG-0 comprising an optionally substituted triarylmethyl residue, with a deprotection mixture M-b, thereby cleaving the protecting group PG-0 from the nucleoside or oligonucleotide, wherein said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety, and wherein said nucleoside or oligonucleotide comprises at least one purine type nucleobase, preferably a nucleobase selected from the group consisting of adenine and guanine, in particular adenine.

One aspect of the invention pertains to a method for the solid-phase synthesis of a target oligonucleotide O^{T} comprising a step (b) of incubating a nucleoside or oligonucleotide, which is covalently linked to a solid support and comprises a backbone hydroxyl moiety protected by a protecting group PG-0 comprising an optionally substituted triarylmethyl residue, with a deprotection mixture M-b, thereby cleaving the protecting group PG-0 from the nucleoside or oligonucleotide, wherein said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety, and wherein said backbone hydroxyl moiety protected by PG-0 is part of a nucleoside moiety comprising a purine type nucleobase, preferably a nucleobase selected from the group consisting of adenine and guanine, in particular adenine.

As used herein, a purine type nucleobase is a nucleobase comprising an optionally substituted purine residue. Preferred examples of purine type nucleobases are adenine and guanine. Purine type nucleobases may carry one or more protecting groups. In particular, exocyclic amino groups, as e.g. present in adenine and guanine, may be protected. Preferred examples of suitable amine protecting groups are compiled in Table T-1 (vide infra). In some embodiments of the method of the invention, said nucleoside or oligonucleotide, which is covalently linked to a solid support and comprises a backbone hydroxyl moiety protected by a protecting group PG-0 comprising an optionally substituted triarylmethyl residue is a component C-0 as defined herein. This means that any definitions, explanations, and embodiments pertaining to said component C-0 may likewise apply to said nucleoside or oligonucleotide, which is covalently linked to a solid support and comprises a backbone hydroxyl moiety protected by a protecting group PG-0 comprising an optionally substituted triarylmethyl residue.

In some embodiments of the method of the invention, the target oligonucleotide O^{T} comprises a first cycle oligonucleotide O-1, and the method comprises the following step (a) and a first coupling cycle comprising the following steps (b) to (e):
(a) providing a component C-0 selected from the group consisting of a nucleoside and an oligonucleotide, wherein the component C-0 is covalently linked to a solid support and comprises a backbone hydroxyl moiety protected by a protecting group PG-0 comprising an optionally substituted triarylmethyl residue;
(b) incubating the component C-0 of step (a) with a deprotection mixture M-b, thereby cleaving the protecting group PG-0 from the component C-0, so as to obtain a component C-0^{#} having a free backbone hydroxyl group;
(c) providing a building block B-1 selected from the group consisting of a nucleoside and an oligonucleotide, wherein the building block B-1 comprises a backbone hydroxyl moiety protected by a protecting group PG-1 comprising an optionally substituted triarylmethyl residue, and a phosphorus moiety covalently bonded via its phosphorus atom to an oxygen atom of the backbone of the building block B-1;
(d) reacting the component C-0^{#} of step (b) with the building block B-1 of step (c) under conditions suitable to form a covalent bond between said free backbone hydroxyl group of the component C-0^{#} and the phosphorus atom of said phosphorus moiety of the building block B-1, thereby obtaining a first cycle oligonucleotide O-1;
(e) optionally, incubating the first cycle oligonucleotide O-1 obtained in step (d) with an oxidizing or sulfurizing agent, thereby converting any P (III) atoms within said first cycle oligonucleotide O-1 to P (V) atoms;
wherein in step (b), said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety.

In some embodiments of the method of the invention, the target oligonucleotide O^{T} comprises a n-th cycle oligonucleotide O-n, and the method further comprises performing (n-1) iterations of a coupling cycle comprising the following steps (b') to
(e'), wherein n is an integer in the range of 2 to 99, which denotes the total number of coupling cycles performed to obtain the n-th cycle oligonucleotide O-n, and each individual coupling cycle comprising the following steps (b') to (e') is identified by a serial number x, which runs in steps of 1 from 2 to n:
(b') incubating the (x-1)-th cycle oligonucleotide O-(x-1) obtained in the previous coupling cycle with a deprotection mixture M-b', thereby cleaving the protecting group PG-(x-1) from the (x-1)-th cycle oligonucleotide O-(x-1), so as to obtain a (x-1)-th cycle oligonucleotide (O-(x-1))^{#} having a free backbone hydroxyl group;
(c') providing a building block B-x selected from the group consisting of a nucleoside and an oligonucleotide, wherein the building block B-x comprises a backbone hydroxyl moiety protected by a protecting group PG-x comprising an optionally substituted triarylmethyl residue, and a phosphorus moiety covalently bonded via its phosphorus atom to an oxygen atom of the backbone of the building block B-x;
(d') reacting the (x-1)-th cycle oligonucleotide (O-(x-1))^{#} obtained in step (b') with the building block B-x of step (c') under conditions suitable to form a covalent bond between said free backbone hydroxyl group of the (x-1)-th cycle oligonucleotide (O-(x-1))^{#} and the phosphorus atom of said phosphorus moiety of the building block B-x, thereby obtaining a x-th cycle oligonucleotide O-x;
(e') optionally, incubating the x-th cycle oligonucleotide O-x obtained in step (d') with an oxidizing or sulfurizing agent, thereby converting any P (III) atoms within said x-th cycle oligonucleotide O-x to P (V) atoms;
wherein in at least one iteration of step (b'), said deprotection mixture M-b' is a liquid composition C comprising a solvent, a protic acid having a pKa equal to or smaller than 4, and at least one alcohol, in which one or more hydroxyl groups are covalently bonded directly to an optionally substituted phenyl moiety.

As used in the context of the present invention, expressions like "target oligonucleotide O^{T}" or "liquid composition C" may be understood as synonymous with "target oligonucleotide (O^{T})" or "liquid composition (C)". This means. "O^{T} ", "(O^{T})" "C" and "(C)" may be understood as a reference marks, which do not imply any further limitation. The same applies for similar expressions such as the first cycle oligonucleotides O-1 and (O-1)^{#}, the (x-1)-th cycle oligonucleotides O-(x-1) and (O-(x-1))^{#}, the n-th cycle oligonucleotide O-n, the components C-0 and C-0^{#}, the building blocks B-1 and B-x, the protecting groups PG-0, PG-1, PG-x and PG-(x-1), and the deprotection mixtures M-b, M-b', M-g, and M-g'.

As used herein, the indefinite articles "a" and "an" may mean "one or more", unless indicated differently.

As used herein, the terms "solid-phase synthesis of an (target) oligonucleotide" may be understood in the broadest sense to refer to any synthesis of an oligonucleotide, wherein an oligonucleotide bound to, preferably covalently linked to, a solid support is subjected to at least one chemical reaction. Preferably, the growing oligonucleotide chain is covalently linked to a solid support throughout at least one, preferably all, coupling cycles. Herein, the terms "solid-phase synthesis of an oligonucleotide" and "solid-phase oligonucleotide synthesis" (SPOS) are used interchangeably.

As used herein, the term "target oligonucleotide O^{T}" refers to any specific oligonucleotide which is to be synthesized by the method of the invention. In other words, the "target oligonucleotide O^{T}" is generally the final oligonucleotide product of the method of the invention. For example, the "target oligonucleotide O^{T} may have the same sequence as the n-th cycle oligonucleotide, or may comprise one or more further nucleoside moieties, and/or may be obtained by conjugating the n-th cycle oligonucleotide to another compound.

Herein, the term "oligonucleotide" is used in a most general way to relate to any oligomers comprising at least two nucleoside subunits interconnected via an internucleosidic linkage group of any one of Formulae A and B: 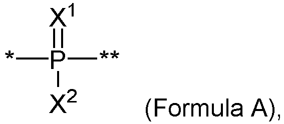 wherein in Formula A:
X¹ is selected from the group consisting of O and S;
X² is selected from the group consisting of O-R¹, S-R¹, and H; and
R¹ may be any conceivable residue, and is preferably selected from the group consisting of H and a protecting group (preferably a protecting group removable under alkaline conditions, in particular the 2-cyanoethyl group, i.e. CH₂-CH₂-CN)); 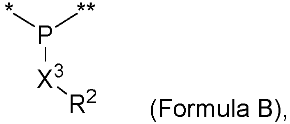
wherein in Formula B:
   X³ is selected from the group consisting of O and S; and
   R² is a protecting group, preferably a protecting group removable under alkaline conditions, in particular the 2-cyanoethyl group; and
   wherein in Formulae A and B each of * and ** independently of each other denotes a binding site (i.e. the point of attachment) of a nucleoside subunit, which is to say that each of * and ** represents the atom of the respective nucleoside subunit with which said nucleoside subunit binds to the internucleosidic linkage group, wherein said atom represented by * and said atom represented by ** are both O.

It will be understood that the internucleosidic linkage groups of Formulae A or B may be protonated (e.g. at a carbonyl or thiocarbonyl group) or deprotonated (e.g. OH or SH groups may be deprotonated), without this being indicated specifically in Formulae A and B. Likewise, Formulae A and B will be understood to embrace any salts, stereoisomeric and tautomeric forms of the respective internucleosidic linkage groups, without this being indicated specifically in Formulae A and B.

Internucleosidic linkage groups, also referred to as internucleoside linkage groups or linkage groups, may be classified depending on the oxidation state of the respective phosphorus (P) atom. Internucleosidic linkage groups typically comprise a P (III) atom or a P (V) atom. Throughout this text, the terms P (III) or phosphorus (III) atom are used interchangeably to denote a P atom of a certain oxidation state, namely with the oxidation state III (i.e. +3). Along the same lines, the terms P (V) or phosphorus (V) atom are used interchangeably to denote a P atom of a certain oxidation state, namely with the oxidation state V (i.e. +5). Internucleosidic linkage groups whose P atom is a P (III) atom are herein referred to as P (III) or phosphorus (III) linkage groups. Internucleosidic linkage groups whose P atom is a P (V) atom are herein referred to as P (V) or phosphorus (V) linkage groups.

Examples of P (V) linkage groups are:
- phosphate diester (i.e. phosphodiester) groups (i.e. groups of Formula A with X¹ being O and X² being OH),
- thiophosphate diester (i.e. phosphorothioate) groups (i.e. groups of Formula A with X¹ being S and X² being OH),
- dithiophosphate diester (i.e. phosphorodithioate) groups (i.e. groups of Formula A with X¹ being S and X² being SH),
- phosphate triester groups (i.e. groups of Formula A with X¹ being O and X² being OR¹, where R¹ is not H, and preferably is a protecting group such as the 2-cyanoethyl group),
- thiophosphate triester groups (i.e. groups of Formula A with X¹ being S and X² being OR¹, where R¹ is not H, and preferably is a protecting group such as the 2-cyanoethyl group), and
- dithiophosphate triester groups (i.e. groups of Formula A with X¹ being S and X² being SR¹, where R¹ is not H, and preferably is a protecting group such as the 2-cyanoethyl group).

Examples of P (III) linkage groups are:
- phosphite triester groups (i.e. groups of Formula B with X³ being O),
- thiophosphite triester groups (i.e. groups of Formula B with X³ being S), and
- H-phosphonate diester groups (i.e. groups of Formula A with X¹ being O and X² being H).

It will be understood by those skilled in the art, that, if an oligonucleotide comprises more than one internucleosidic linkage groups, these internucleosidic linkage groups may be the same or different (i.e. have the same or different chemical structures). An oligonucleotide may also comprise one or more P(III) linkage groups and one or more P(V) linkage groups. As known to the skilled artisan, internucleosidic linkage groups may be modified in the course of oligonucleotide synthesis. For example, P(III) linkage groups may be converted to P (V) linkage groups by incubation with an oxidizing or sulfurizing agent, and/or protecting groups may be removed.

Herein, the term "substituent" may be understood in the broadest sense and may denote any chemical residue or moiety. Thus, herein, unless indicated differently, the terms "substituent", "residue" and "moiety" may be used interchangeably.

The term "group" is also used to denote certain substituents (i.e. residues or moieties). However, the terms "substituent", "moiety" or "residue" may be broader than the term "group" in the cases laid out below:
Herein, the term hydroxyl group refers to a OH residue. In other words, a hydroxyl group is always a free hydroxyl group. However, the term hydroxyl moiety additionally encompasses a hydroxyl residue, which results from a reaction of a hydroxyl group with another chemical group, e.g. a hydroxyl residue involved in an internucleosidic linkage group or a hydroxyl residue bound to a protecting group. The expressions sulfhydryl group, SH, and sulfhydryl moiety are used analogously. Herein, the terms amino group and amine group are used interchangeably to denote a substituent bonded to the respective chemical structure via a nitrogen atom, wherein said nitrogen atom is further bonded to two or three residues selected from the group consisting of H, alkyl, heteroalkyl, alkenyl, heteroalkenyl, alkynyl, heteroalkynyl, aryl, and heteroaryl. These residues may be further substituted and may be further specified, for example when denoting the amino group as di(C₁-C₆-alkyl)amino group (e.g. N(C₁-C₆-alkyl)₂). Said di(C₁-C₆-alkyl)amino group may also be a cyclic amino group in which formally two alkyl residues are bonded to each other to form a cyclic structure. Examples of such cyclic amino groups comprise a pyrrolidine group and a piperidine group. The term amine moiety additionally encompasses an amine residue, which results from a reaction of an amine group with another chemical group, e.g. an amine residue involved in a bond to a protecting group.

As used herein, the term "optionally substituted" may be understood in the broadest sense to mean that in the respective structure, which is optionally substituted, e.g. in said optionally substituted phenyl moiety or in said optionally substituted triarylmethyl residue, one or more hydrogen residues may optionally and independently of each other be substituted by another residue, also referred to as substituent. If a "substituent" (i.e. residue or moiety) is not further specified it may be any conceivable stable atom or atom group and may preferably be selected from the group consisting of an alkyl residue, O-alkyl, a halogen residue (F, Cl, Br, I), a cyano (i.e. CN) residue, a heteroalkyl residue, an alkenyl residue, a heteroalkenyl residue, an alkynyl residue, a heteroalkynyl residue, an aryl residue, a heteroaryl residue, a ketone residue (in particular C(O)alkyl), an aldehyde residue (CHO), a carboxylic acid residue or ester (in particular C(O)O-alkyl) or amide thereof, an amine group or moiety, a boryl residue (i.e. a substituent bonded via a B atom), a silyl residue (i.e. a substituent bonded via a silicon atom), a hydroxyl group or moiety, a sulfhydryl group or moiety, and combinations thereof. Unless indicated differently, these substituents may be further substituted, i.e. one or more hydrogen atoms in these substituents may be substituted by further substituents.

As used throughout the present invention, the term "alkyl" may be understood in the broadest sense and may be used to denote any aliphatic group (in other words: residue, moiety or substituent) which may be composed of atoms of the chemical elements carbon (C) and hydrogen (H), but does not comprise any heteroatoms. Additionally, an alkyl group as defined herein may be characterized in that it comprises at least one carbon atom and in that the one or more carbon atoms may only be bonded to each other via direct single bonds. Preferably, unless indicated differently in the context of specific embodiments, an alkyl group may comprise 1 to 20, 1 to 6, or 1 to 5 carbon atoms. Unless stated differently, the term "alkyl" may generally embrace unbranched, branched, and cyclic groups. Non-limiting examples of alkyl groups comprise methyl, ethyl, *n*-propyl (propane-1-yl), isopropyl (propane-2-yl), *n*-butyl (butan-1-yl), *sec*-butyl (butan-2-yl), *tert*-butyl (2-methylpropan-2-yl), isobutyl (2-methylpropan-1-yl), cyclohexyl, and cyclopentyl.

As used throughout the present invention, the term "heteroalkyl" may be understood in the broadest sense and may be used to denote any alkyl group, in which one or more carbon or hydrogen atoms are substituted by a heteroatom, with the proviso that a heteroalkyl group must comprise at least one carbon atom. A heteroatom in this context may be any atom of a chemical element other than carbon and hydrogen, and, unless indicated differently in the context of specific embodiments, may preferably be an atom which is at each occurrence independently selected from the group consisting of N (nitrogen), O, (oxygen), S (sulfur), P (phosphorus), F (fluorine), Cl (chlorine), Br (bromine), I (iodine), and Si (silicon). Preferably, unless indicated differently in the context of specific embodiments, a heteroalkyl group may comprise 1 to 19, 1 to 6, or 1 to 5 carbon atoms. Unless stated differently, the term "heteroalkyl" may generally embrace unbranched, branched, and cyclic groups. Non-limiting examples of heteroalkyl groups comprise halogenated alkyl groups such as trifluoromethyl groups, oxygenated alkyl groups (i.e. alkyloxy groups), aminated alkyl groups (i.e. alkylamine groups, e.g. dimethylamine groups), and cyclic groups such as e.g. piperidine groups, pyrrolidine groups or morpholine groups. A heteroalkyl-substituent may typically be bonded via one or more of its carbon atoms.

As used throughout the present invention, the term "alkenyl" may be understood in the broadest sense and may be used to denote any aliphatic group which may be derived from an alkyl group by introducing one or more C=C-double bonds between carbon atoms. Likewise, the term "heteroalkenyl" may be understood in the broadest sense and may be used to denote any aliphatic group which may be derived from a heteroalkyl group by introducing one or more C=C-double bonds between carbon atoms. Herein, unless stated differently in the context of specific embodiments, the terms "alkenyl" and "heteroalkenyl" generally embrace both, the (E)- and the (Z)-isomers, and mixtures thereof. As used throughout the present invention, the term "alkynyl" may be understood in the broadest sense and may be used to denote any aliphatic group which may be derived from an alkyl group by introducing one or more C=C-triple bonds between carbon atoms. Likewise, the term "heteroalkynyl" may be understood in the broadest sense and may be used to denote any aliphatic group which may be derived from a heteroalkyl group by introducing one or more C≡C-triple bonds between carbon atoms. It will be understood that an alkenyl group, a heteroalkenyl group, an alkynyl group, and a heteroalkynyl group comprise at least two carbon atoms.

As used herein, the term "hydrocarbon" may be understood in the broadest sense and may be used to denote any compound or residue which is composed of atoms of the chemical elements carbon (C) and hydrogen (H), but does not comprise any heteroatoms. The term "aliphatic hydrocarbon" embraces alkyl groups, alkenyl groups, and alkynyl groups as defined herein, unless indicated differently in the context of specific embodiments.

As used throughout the present invention, the term "aryl" may be understood in the broadest sense and may be used to denote any mono- or polycyclic aromatic group, with the proviso that all aromatic ring atoms are carbon atoms. Unless indicted differently in the context of specific embodiments, an aryl group may preferably comprise 6 to 30, 6 to 20, or 6 to 15, in particular 6 aromatic carbon atoms. Benzene (i.e. the phenyl group, Ph) may be a preferred example of a monocyclic aromatic (i.e. aryl) group. It will be understood by those skilled in the art that the expression "polycyclic" in this regard may refer to condensed aromatic ring systems in which two or more aromatic rings are fused together. This is to say that the condensed aromatic rings share at least one bond between aromatic carbon atoms, so that this shared bond as well as the carbon atoms forming the bond are part of two or more monocyclic aromatic groups which build up the polycyclic aromatic group. Non-limiting examples of polycyclic aromatic (i.e. aryl) groups are naphthalene (i.e. napthyl), anthracene (i.e. anthracenyl), and phenanthrene (i.e. phenanthryl).

As used throughout the present invention, the term "heteroaryl" may be understood in the broadest sense and may be used to denote any mono- or polycyclic heteroaromatic group, which differs from an aromatic (i.e. aryl) group in that at least one, preferably 1 to 5 or 1 to 3, aromatic ring atoms are heteroatoms. A heteroatom in this context is any atom of a chemical element other than carbon and hydrogen, and may, unless indicated differently in the context of specific embodiments, preferably be an atom which is at each occurrence independently selected from the group consisting of N, O, and S. Non-limiting examples of monocyclic heteroaromatic (i.e. heteroaryl) groups comprise pyridine (i.e. pyridyl), pyridazine (i.e. pyridazinyl), pyrimidine (i.e. pyrimidinyl), pyrazine (i.e. pyrazinyl), 1,2,3-triazine (i.e. 1,2,3-triazinyl), 1,2,4- triazine (i.e. 1,2,4-triazinyl), 1,3,5- triazine (i.e. 1,3,5-triazinyl), 1,2,3,4- tetrazine (i.e. 1,2,3,4-tetrazinyl), 1,2,4,5- tetrazine (i.e. 1,2,4,5-tetrazinyl), pyrrole (i.e. pyrrolyl), pyrazole (i.e. pyrazolyl), imidazole (i.e. imidazolyl), 1,2,3-triazole (i.e. 1,2,3-triazolyl), 1,2,4-triazole (i.e. 1,2,4-triazolyl), thiophene (i.e. thiophenyl), and furan (i.e. furanyl). Non-limiting examples of polycyclic heteroaromatic (i.e. heteroaryl) groups comprise quinoline (i.e. quinolinyl), quinazoline (i.e. quinazolinyl), quinoxaline (i.e. quinoxalinyl), benzofuran (i.e. benzofuranyl), benzothiophene (i.e. benzothiophenyl), benzimidazole (i.e. benzimidazolyl), benzothiazole (i.e. benzothiazolyl), benzoxazole (i.e. benzoxazolyl), dibenzofuran (i.e. dibenzofuranyl), and acridine (i.e. acridinyl).

As used herein, the term "aliphatic" may be used to denote any compounds or residues which are not aromatic, i.e. which do not comprise an aromatic or heteroaromatic ring system. It is understood that such aliphatic compounds may be unbranched, branched or cyclic, unless indicated differently in the context of specific embodiments. For example, an aliphatic cyclic amine moiety may, e.g., be a piperidine moiety, a pyrrolidine moiety or a morpholine moiety, but may not be a pyridine moiety, which instead is a heteroaromatic amine moiety or, more general, a heteroaryl moiety or heteroaromatic moiety.

In line with common practice, the names of substituents (i.e. residues or moieties and also groups) may herein typically be derived from the chemical name and indicated by the last syllable "-yl". However, herein, this wording may be used interchangeably with the chemical name of the respective chemical atom or atom group as if it was not a substituent, with common abbreviations, and with the element symbol(s) themselves. For example, a CH₃-substituent may also be referred to as methyl or Me, a Cl-substituent may also be referred to as chlorine or simply Cl, a phenyl substituent may also be denoted as Ph, and a cyano group may also be referred to as CN. If a substituent is denoted using element symbols, e.g. CN, O(C₁-C₆-alkyl), C(O)(C₁-C₆-alkyl), C(O)O(C₁-C₆-alkyl and comprises more than one atom, the point of attachment of the respective residue (i.e. the atom from which the chemical bond to the parent structure originates) is herein generally the atom denoted to the very left, e.g. the carbon atom in CN and the oxygen atom in O(C₁-C₆-alkyl), unless indicated differently. The same rationale applies if a substituent is denoted using generic residues R such as in O-R^{z-1}, where the oxygen atom constitutes the point of attachment of the residue to the parent structure. Herein, unless stated differently in the context of specific embodiments, when referring to a specific substituent in general terms such as "butyl", this wording embraces all isomers having a chemical structure falling under the respective general substituent name such as "butyl", including for example regioisomers (such as *n*-butyl, *sec*-butyl, *tert*-butyl, isobutyl, and the like), diastereomers, and enantiomers, where applicable.

As used throughout the present invention, the number of carbon atoms of residues (also moieties or substituents or groups) is occasionally indicated in the form of subscript numbers, for example in the form of C₁-C₆-alkyl or C₆-C₂₀-aryl. These subscript numbers refer to the range of allowable numbers of carbon atoms for the specific residue and in the context of the specific embodiment. When referring to aryl or heteroaryl residues, these subscript numbers refer to aromatic ring carbon atoms. For example, a C₁-C₆-alkyl group comprises 1 to 6 carbon atoms, but not less than 1 or more than 6 carbon atoms. Along the same lines, a C₆-C₂₀₋aryl group comprises 6 to 20 aromatic ring carbon atoms, but not less than 6 or more than 20 aromatic ring carbon atoms.

Herein, unless stated differently in the context of specific embodiments, when referring to an oligonucleotide, said oligonucleotide may generally be present as a mixture of isomers, in particular as a mixture of stereoisomers. A person skilled in the art understands that a molecule of an oligonucleotide may (typically) only be present in a single (stereo)isomeric form at a certain point in time. Thus, when referring to an oligonucleotide as optionally being present as a mixture of (stereo)isomers, this may refer to a population of oligonucleotide molecules having essentially the same nucleoside sequence, wherein the molecules of said population may be present in different (stereo)isomeric forms. Thus, said population of oligonucleotide molecules may be a mixture of numerous discrete stereoisomers, or may be enriched in one specific stereoisomeric form, or may essentially consist of molecules of a specific stereoisomeric form.

It will be understood that certain groups, for example OH, SH, and SeH groups within an oligonucleotide may be deprotonated. Likewise, it will be understood that certain groups, for example amino groups, within an oligonucleotide may be protonated. It will be understood by a person skilled in the art that an oligonucleotide as defined herein may optionally bear any counter ions known in the art, in particular cations such as sodium cations, potassium cations, magnesium cations, ammonium cations as well as in general cations derived from amines such as trimethylamine (TEA), diisopropylamine (DIPEA) or derived from heteroaromatics such as pyridine or collidine and/or anions such as chloride anions, bromide anions, acetate anions, trifluoroacetate anions, carbonate anions, hydrocarbonate anions, phosphate anions, hydrogen phosphate anions, dihydrogen phosphate anions, perchlorate anions, and combinations thereof. Deprotonation or protonation of an oligonucleotide as well as the (non-covalent) attachment of one or more cations and/or anions may for example occur during the synthesis, isolation, and purification of an oligonucleotide. In general, it will be understood by those skilled in the art that an oligonucleotide as defined herein may be non-covalently bonded or associated to/with species with whom the oligonucleotide was contacted in the course of its synthesis, isolation or purification, for example cations and/or anions or residuals of protecting groups as well as traces of one or more cation scavengers, such as, e.g., thiols or silanes. It will also be understood that such non-covalently bonded or associated species will typically not be depicted in chemical formulas, especially generic formulas, referring to the oligonucleotide or oligonucleotides.

Non-limiting examples of oligonucleotides of the invention are 2'-deoxynucleic acids (DNA), ribonucleic acids (RNA), locked nucleic acids (LNA), constrained ethyl nucleic acid analogs (cET), bridged nucleic acids (BNA), tricycloDNA, unlocked nucleic acids (UNA), small interfering RNA (siRNA), microRNA, antisense oligonucleotides (ASO), gapmers, glycerol nucleic acids, phosphorothioate oligonucleotides, phosphorodithioate oligonucleotides, as well as derivatives and analogs thereof, all of which are known to those skilled in the art.

An oligonucleotide preferably is a linear sequence of nucleoside subunits, wherein any two adjacent nucleoside subunits are interconnected by an internucleosidic linkage group as defined above. Such a linear sequence may also be referred to as an oligonucleotide strand. In line with common practice, the number of nucleoside subunits in an oligonucleotide may be denoted by a number (an integer equal to or larger than 2) followed by the syllable "mer" or by using a suitable prefix (e.g. di for 2, tri for 3, and the like). For example, an oligonucleotide comprising exactly two nucleoside subunits may be denoted as a 2mer or 2mer oligonucleotide or a dinucleotide and an oligonucleotide comprising exactly 20 nucleoside subunits may be denoted as a 20mer or a 20mer oligonucleotide.

Such an oligonucleotide strand will comprise a first terminal nucleoside subunit and a second terminal nucleoside subunit, both of which only have exactly one adjacent nucleoside subunit. In case of a dinucleotide (i.e. a 2mer oligonucleotide) the two terminal nucleoside subunits are interconnected by a first and only internucleosidic linkage group. An oligonucleotide comprising more than two nucleoside subunits, will comprise exactly two terminal nucleoside subunits and one or more non-terminal nucleoside subunits, wherein non-terminal nucleoside subunits are characterized in that they have exactly two adjacent (i.e. one antecedent and one following) nucleoside subunits. The term "adjacent" when referring to nucleoside subunits of an oligonucleotide (strand) refers to any two nucleoside subunits which are interconnected by an internucleosidic linkage group.

It is understood that a nucleoside moiety which forms part of an oligonucleotide is herein referred to as a nucleoside subunit of said oligonucleotide. Herein, the term nucleoside moiety embraces both, nucleosides as such and nucleoside subunits of an oligonucleotide as defined herein. The term "nucleoside" (without the addition "moiety" or "subunit") is however only used to denote a mononucleoside, i.e. a nucleoside moiety which is not a nucleoside subunit of an oligonucleotide. The possible chemical structures of nucleoside moieties are known to those skilled in the art.

A nucleoside moiety may be composed of:
- a carbohydrate moiety (in other words: a sugar moiety), preferably a monosaccharide moiety, more preferably a pentose moiety, in particular a ribose moiety (such as typically present in RNA) or a 2'-deoxyribose moiety (such as typically present DNA); and
- a nucleobase,
wherein said carbohydrate moiety and said nucleobase may typically be covalently bonded to each other via a direct covalent bond, typically an N-glycosidic bond. The nature and the chemistry of carbohydrates and nucleobases form part of the common knowledge of those skilled in the art, who are able to select a carbohydrate moiety as well as a nucleobase for a nucleoside moiety depending on the structure of the oligonucleotide to be synthesized.

Herein, unless indicated differently in the context of specific embodiments, the term "nucleoside moiety" may comprise naturally occurring nucleoside moieties as well as non-natural nucleoside moieties, in which the carbohydrate moiety and/or the nucleobase have been chemically modified or in which the nucleobase may even be absent (i.e. an abasic site). Non-limiting examples of naturally occurring nucleoside moieties may comprise adenosine, 2'-deoxyadenosine, guanosine, 2'-deoxyguanosine, cytidine, 2'-deoxycytidine, uridine, (2'-deoxy)thymidine, ribothymidine, inosine, and methylated derivatives thereof, all of which are known to those skilled in the art. Further examples may comprise queuosine, archaeosine, wybutosine, lysidine, and N⁶-threonylcarbamoyladenosine.

The term "derivative" as used herein may be understood in the broadest sense and may refer to a compound obtainable from a first compound (i.e. a parent compound) by means of one or more, preferably one, chemical reaction. As a result, a derivative may differ from the first (parent) compound for example with regards to the substitutional pattern or with regards to the presence or absence of one or more atom, atom groups, functional groups, or protecting groups.

For example, chemical modifications of (non-natural) nucleoside moieties may comprise modifications of the carbohydrate, in particular of the ribose or 2'-deoxyribose moiety. Such carbohydrate-modifications may exemplarily be selected from the group consisting of:
- introducing an O-CH₃ (i.e. O-methyl or OMe) group, an O-CH₂-CH₂-O-CH₃ (i.e. O-methoxyethyl or O-MOE) group or an F-substituent (i.e. a fluorine substituent) at the 2'-carbon atom;
- introducing a methylene or ethylene bridge between the 2'-oxygen and 4'-carbon ("locked" derivatives) of a ribose moiety;
- interconnecting the 3'-carbon atom and the 5'-carbon atom of a ribose or 2'-deoxyribose moiety (e.g. in tricycloDNA, tcDNA);
- replacing the ribose or 2'-deoxyribose moiety by another pentose such as arabinose or a hexose such as mannose; and
- using the L-enantiomer of the carbohydrate instead of the naturally occurring D-enantiomer;
and/or combinations of these modifications.

Herein, the term "nucleobase" encompasses both non-natural nucleobases as well as naturally occurring nucleobases such as adenine, guanine, cytosine, thymine, and uracil. A person skilled in the art knows how to select a non-natural nucleobase suitable to replace a naturally occurring nucleobase in a nucleoside moiety, so that the non-natural nucleobase may still be capable of a specific interaction with a complementary nucleobase. The interaction between complementary nucleobases may be mediated by hydrogen bonds (cf. Watson-Crick base pairing). Non-natural nucleobases may preferably be derivatives of purine or pyrimidine, which are capable of a specific interaction with another nucleobase.

Non-natural nucleoside moieties may for example be formed from a naturally occurring carbohydrate and a non-natural nucleobase or from a non-natural carbohydrate and a naturally occurring nucleobase or from a non-natural carbohydrate and a non-natural nucleobase.

Examples of nucleoside moieties are nucleoside moieties of Formula C: 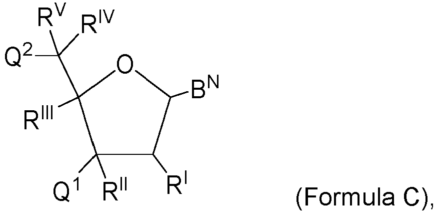 wherein in Formula C:
B^{N} is a nucleobase which may carry one or more protecting groups;
Q¹ is selected from the group consisting of OR³ (if the nucleoside moiety is the 3'-terminal nucleoside moiety of an oligonucleotide or if the nucleoside moiety is a nucleoside) and an oxygen atom covalently bonded to an internucleosidic linkage group (if the respective nucleoside moiety is part of an oligonucleotide and not the 3'-terminal nucleoside moiety thereof);
R³ is selected from the group consisting of H, a protecting group, and a conjugated moiety which is not a nucleoside, nucleotide or oligonucleotide;
Q² is selected from the group consisting of OR⁴ (if the nucleoside moiety is the 5'-terminal nucleoside moiety of an oligonucleotide or if the nucleoside moiety is a nucleoside) and an oxygen atom covalently bonded to an internucleosidic linkage group (if the respective nucleoside moiety is part of an oligonucleotide and not the 5'-terminal nucleoside moiety thereof);
R⁴ is selected from the group consisting of H, a protecting group, and a conjugated moiety which is not a nucleoside, nucleotide or oligonucleotide;
R^{I} is selected from the group consisting of H, F, and OR⁵, where R⁵ may be H, a protecting group, any conceivable substituent, or a conjugated moiety which is not a nucleoside, nucleotide or oligonucleotide;
R^{III} is H or R^{III} and R^{I} are bonded together to form a cyclic structure;
R^{II}, R^{IV}, and R^{V} may independently of each other be any conceivable substituent, wherein two or three of these residues may optionally be bonded together to form a cyclic structure.

As laid out above, unless indicated differently in the context of specific embodiments, an oligonucleotide may herein generally be present as a mixture of isomers. No stereochemical information may be deduced from Formula C. In some preferred embodiments, a nucleoside moiety of Formula C is a nucleoside moiety of the following Formula C-a: 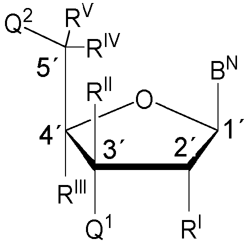 (Formula C-a; carbon atoms labelled from 1' to 5'),
wherein in Formula C-a, the carbon atoms have been numbered (in line with common practice) from 1' to 5', which merely serves illustrative purposes and should not be construed to be limiting in any kind. In Formula C-a, Q¹, Q², B^{N}, R^{I}, R^{II}, R^{III}, R^{IV}, R^{V}, R³, R⁴, and R⁵ are defined as for Formula C. It is understood that the carbon atom numbers will be identical in any ribose or 2'-deoxyribose based nucleoside moieties.

From what has been laid out above, it will be understood that any nucleoside subunits comprising a ribose or 2'-deoxyribose moiety, e.g. nucleoside moieties of Formula C and/or C-a, will preferably all be incorporated into an oligonucleotide in 3' → 5' direction or 5' → 3'direction (all in the same direction). Such nucleoside subunits will bond to one (if it is a terminal nucleoside subunit) or two (if it is a non-terminal nucleoside subunit) internucleosidic linkage groups, wherein, preferably, bonding to said internucleosidic linkage group(s) occurs through the 3'-hydroxyl moiety (i.e. the hydroxyl moiety bonded to the 3' -carbon atom) and/or the 5'-hydroxyl moiety (i.e. the hydroxyl moiety bonded to the 5'-carbon atom) exclusively. Since all nucleoside subunits are preferably incorporated in the same direction, any internucleosidic linkage groups between nucleoside subunits comprising a ribose or 2'-deoxyribose moiety are preferably bonded to the 5'-hydroxyl moiety of one nucleoside subunit and the 3'-hydroxyl moiety of another nucleoside subunit. Obviously, the 3'-hydroxyl moiety of the 3'-terminal nucleoside subunit does not engage in bonding to an internucleosidic linkage group. Likewise, the 5'-hydroxyl moiety of the 5'-terminal nucleoside subunit does not engage in bonding to an internucleosidic linkage group.

It is understood from what has been laid out above, that an oligonucleotide comprises two or more nucleoside subunits (nucleoside moieties). It will further be understood that these two or more nucleoside subunits of an oligonucleotide may be the same or different (i.e. have the same or different chemical structures).

As used herein, the term "nucleotide" may be understood in the broadest sense and may preferably refer to a conjugate of a nucleoside moiety and a phosphate group or derivative thereof, wherein a hydroxyl moiety of said nucleoside moiety bonds via its oxygen atom to the phosphorus atom of the phosphate moiety or derivative thereof.

As will be understood by those skilled in the art, the internucleosidic linkage groups and any carbohydrate moieties, e.g. ribose- or 2'-deoxyribose moieties, are herein referred to as the "backbone" of an oligonucleotide. Thus, the term "backbone" of an oligonucleotide excludes the nucleobases. It will also be understood that in a (mono-)nucleoside, the backbone is the carbohydrate moiety, e.g. the ribose- or 2'-deoxyribose moiety, since a nucleoside does not contain any internucleosidic linkage groups.

It forms part of the common knowledge of those skilled in the art that oligonucleotides may be conjugated to additional moieties, which are not a nucleoside, nucleotide or oligonucleotide as defined herein, for various purposes. In particular, free OH-groups (e.g. 3'-OH and/or 5'-OH groups of ribose or 2'-deoxyribose moieties), preferably at the terminal nucleosides of an oligonucleotide strand, may engage in such conjugation. For example, the 5'-OH group of the antisense strand of siRNA (small interfering RNA, known to those skilled in the art) may be modified by a vinyl phosphonate to inhibit cellular degradation and improve potency. A further example is conjugation to *N*-acetylgalactosamine (GalNAc), which may be of interest for targeted oligonucleotide delivery to hepatocytes. GalNAc structures, often branched to accommodate 3 or 4 GalNAc moieties, may be conjugated, e.g. to the 5'-OH group of an oligonucleotide (cf., e.g., WO2016055601), to the 3'-OH group (cf., e.g., WO2009073809), or monovalent GalNAc moieties may be conjugated via a linker to the 2'-position of subsequent ribose moieties within the oligonucleotide strand (cf., e.g., WO2019075419). For example, the target oligonucleotide O^{T} may be an oligonucleotide conjugate, unless indicated differently in the context of specific embodiments. A used herein, the term "oligonucleotide conjugate" may refer to any oligonucleotide comprising at least one nucleoside-subunit which is covalently bonded to another moiety, which is not a nucleoside, nucleotide or oligonucleotide, e.g. to a moiety comprising a peptide, protein, lipid, carbohydrate, or a hydrocarbon moiety, among which carbohydrate moieties may be preferred. In some embodiments of the method of the invention, the target oligonucleotide O^{T} is not an oligonucleotide conjugate.

The term "target oligonucleotide O^{T} comprising a first cycle oligonucleotide O-1" (in other words: the target oligonucleotide O^{T} comprises a first cycle oligonucleotide O-1), means that the target oligonucleotide O^{T} comprises the nucleoside sequence of said first cycle oligonucleotide O-1. The term "target oligonucleotide O^{T} comprising a n-th cycle oligonucleotide O-n" (in other words: the target oligonucleotide O^{T} comprises a n-th cycle oligonucleotide O-n), means that the target oligonucleotide O^{T} comprises the nucleoside sequence of said n-th cycle oligonucleotide O-n.

As used herein, the term "nucleoside sequence" refers to an array of the nucleoside subunits within an oligonucleotide. For a linear oligonucleotide, the nucleoside sequence is usually given starting from a first terminal nucleoside subunit, optionally continuing with one or more non-terminal nucleoside subunits, and ending with a second terminal nucleoside subunit. As used herein, the term "nucleoside sequence" does not specify one or more internucleosidic linkage groups and does not take into account the presence or absence of any protecting groups. Thus, two oligonucleotides comprising the same nucleoside subunits in the same order are herein considered to comprise the same nucleoside sequence, regardless of whether or not the internucleosidic linkage groups interconnecting these nucleoside subunits are the same or not (i.e. have the same chemical structure or not) and regardless of whether or not any atoms or functional groups within the carbohydrate moieties, the nucleobases, and the internucleosidic linkage groups are protected. A solid support is herein regarded a (permanent) protecting group and thus comprised in the general term "protecting group", unless indicated differently in the context of specific embodiments. For example, the internucleosidic linkage groups may differ with regards to the oxidation state of the phosphorus atom and/or the presence or absence of protecting groups such as the 2-cyanoethyl group. As another example, the exocyclic amino groups of nucleobases such as cytosine, 5-methylcytosine, guanine, and adenine may be protected or may not be protected, and a 5'-terminal hydroxyl moiety may or may not be protected without having any impact on what is herein referred to as the nucleoside sequence.

It will be understood that, unless indicated differently in the context of specific embodiments, the target oligonucleotide O^{T} may comprise more nucleoside subunits than the first cycle oligonucleotide O-1, for example, if the first coupling cycle comprising steps (b) to (e) is followed by further steps including further coupling cycles, e.g. further coupling cycles comprising steps (b') to (e'). It will be understood that, unless indicated differently in the context of specific embodiments, the target oligonucleotide O^{T} may comprise more nucleoside subunits than the n-th cycle oligonucleotide O-n, for example, if the (n-1) iterations of coupling cycles comprising steps (b') to (e') are followed by further steps including one or more condensation reactions with another nucleoside or oligonucleotide. Additionally, or alternatively, the target oligonucleotide O^{T} may also be a conjugate as defined herein, i.e. covalently bonded to a moiety which is not a nucleoside, nucleotide or oligonucleotide, for example, to a carbohydrate moiety. Such conjugation may, for example, be achieved after the final iteration of the coupling cycle has been carried out.

In some embodiments of the method of the invention, the target oligonucleotide O^{T} consists of the same nucleoside sequence as the first cycle oligonucleotide O-1. In such embodiments, the target oligonucleotide O^{T} may only differ from the first cycle oligonucleotide O-1 with regards to the internucleosidic linkage groups and the absence or presence of protecting groups. In some embodiments of the method of the invention, the target oligonucleotide O^{T} consists of the same nucleoside sequence as the n-th cycle oligonucleotide O-n. In such embodiments, the target oligonucleotide O^{T} may only differ from the n-th cycle oligonucleotide O-n with regards to the internucleosidic linkage groups and the absence or presence of protecting groups. For example, the target oligonucleotide may preferably only comprise phosphorus (V) linkage groups while the first cycle oligonucleotide O-1 or the n-th cycle oligonucleotide O-n may comprise one or more phosphorus (III) linkage groups. For example, the target oligonucleotide O^{T} may preferably not comprise any protecting groups, while the first cycle oligonucleotide O-1 and the n-th cycle oligonucleotide O-n typically comprise several protecting groups and are at least covalently linked to a solid support, which is herein considered a (permanent) protecting group.

In some embodiments of the method of the invention, the target oligonucleotide O^{T} comprises 2-200, 2-150, 2-100, 2-90, 2-80, 2-70, 2-60, 2-50, 2-40, 2-30, or 2-20 nucleoside subunits. In some embodiments of the method of the invention, the target oligonucleotide O^{T} comprises at least one purine type nucleobase, preferably a nucleobase selected from the group consisting of adenine and guanine, in particular adenine.

Herein, the terms "removing a protecting group", "cleaving a protecting group", and "deprotecting" a functional group are used interchangeably. Likewise, herein, the terms "cleavage of a protecting group", "removal of a protecting group", and "deprotection" of a functional group are used interchangeably. To highlight that a specific atom or functional group does not carry a protecting group, the adjective "free" may be used, which does not imply that any groups not denoted as "free" carry a protecting group. Herein, the process of removing a protecting group comprising an optionally substituted triarylmethyl residue, e.g. a DMT group, may be referred to as "detritylation" and the reaction mixture in which such detritylation occurs may be referred to as detritylation cocktail.

As used herein, the term "protecting group" may be understood in the broadest sense as any group which is introduced into a molecule by means of chemical modification (i.e. through one or more, typically one, chemical reaction) of an atom or functional group (i.e. the atom or functional group which is to be protected) and which, once introduced, prevents said atom or functional group from engaging in chemical reactions in subsequent process steps. The terms "protected" and "carrying a protecting group" are herein used interchangeably to denote an atom or functional group covalently bonded to a protecting group. A protecting group used for protecting an amine moiety may herein be referred to as an amine protecting group or amino protecting group. Along the same lines, a protecting group used for protecting a hydroxyl moiety may herein be referred to as a hydroxyl protecting group. It will be understood by those skilled in the art that a protecting group may substitute a hydrogen residue from the atom or functional group to be protected. For example, a hydroxyl protecting group may covalently bind to the oxygen atom of the hydroxyl moiety to be protected, thereby substituting the hydrogen residue.

As used herein, the two interchangeable terms "protecting group comprising an optionally substituted triarylmethyl residue" and "triarylmethyl type protecting group" may be understood in the broadest sense as any protecting group which covalently binds to the atom or functional group to be protected (e.g. to the oxygen atom of the hydroxyl moiety) via a carbon atom to which three optionally substituted aryl moieties are bonded. The basic triarylmethyl type protecting group is the triphenylmethyl group (i.e. the trityl group). As known to those skilled in the art, substituents, e.g. alkyl or alkoxy substituents, capable of stabilizing a triarylmethyl-cation formed during acid-catalyzed removal of a triarylmethyl type protecting group, may facilitate acid-catalyzed cleavage of the respective protecting group.

Examples of triarylmethyl type protecting groups are the trityl group, the (p-methylphenyl)diphenylmethyl group (i.e. the 4-methyltrityl group), the di(p-methylphenyl)phenylmethyl group (i.e. the 4,4'-dimethyltrityl group), the tri(p-methylphenyl)methyl group (i.e. the 4,4',4"-trimethyltrityl group), the (p-methoxyphenyl)diphenylmethyl group (i.e. the MMT group), the di(p-methoxyphenyl)phenylmethyl group (i.e. the DMT group), the tri(p-methoxyphenyl)methyl group (i.e. the TMT group), the 4,4'-dimethoxy-3"-[N-(imidazolylmethyl)]trityl group (i.e. the IDT group), the 4,4'-dimethoxy-3"-[N-(imidazolylethyl)carbamoyl]trityl group (i.e. the IET group), the bis(4-methoxyphenyl)-1'-pyrenylmethyl group (i.e. the Bmpm group), and the 4-(17-tetrabenzo[a,c,g,i]fluorenylmethyl)-4',4"-dimethoxytrityl group (i.e. the Tbf-DMT group). The trityl group, the MMT group, and the DMT group may be preferred. The DMT group is herein most preferred as temporary hydroxyl protecting group. Thus, in some embodiments, a protecting group comprising an optionally substituted triarylmethyl residue is at each occurrence a DMT protecting group.

As used herein, the term "liquid composition" may be understood in the broadest sense to refer to any composition comprising a solvent (which may be a single solvent or a mixture of solvents) and one or more components which are dissolved in said solvent. A liquid composition may be further categorized, e.g. as a liquid composition C, if it fulfills certain conditions.

As used herein, the term "coupling cycle" may be understood in the broadest sense and may refer to a sequence of two or more process steps, including a deprotection step used to provide a free hydroxyl group at a nucleoside or oligonucleotide, and a subsequent coupling step (also referred to as condensation step), in which said free hydroxyl group engages in a bond forming reaction with a phosphorus moiety of another nucleoside or oligonucleotide. Several iterations (i.e. rounds of) coupling cycles may be carried out. During each of these iterations, an elongated oligonucleotide is obtained. A coupling cycle may be further characterized in embodiments of the invention, for example by stating that it comprises steps (b) to (e) or steps (b') to (e'). In the aforementioned coupling cycle comprising steps (b) to (e), i.e. steps (b), (c), (d), and (e), these steps may be carried out in this order, wherein step (e) is optional, unless indicated differently in the context of specific embodiments. It will be understood that stating that a coupling cycle comprises steps (b) to (e) does not alter the fact that step (e) is optional and may or may not be performed. In the aforementioned coupling cycle comprising steps (b') to (e'), i.e. steps (b'), (c'), (d'), and (e'), these steps may be carried out in this order, wherein step (e') is optional, unless indicated differently in the context of specific embodiments. It will be understood that stating that a coupling cycle comprises steps (b') to (e') does not alter the fact that step (e') is optional and may or may not be performed. It will further be understood that a coupling cycle may comprise further steps, which may be inserted before, between, or after the steps specified.

The method of the invention may comprise a first coupling cycle, which is performed after step (a) and which comprises steps (b) to (e). Optionally, this first coupling cycle is followed by (n-1) further iterations of a coupling cycle comprising steps (b') to (e'), wherein n is an integer in the range of 2 to 99 and denotes the total number of coupling cycles performed to obtain the n-th cycle oligonucleotide O-n. This total number of performed coupling cycles includes the first coupling cycle comprising steps (b) to (e) as well as the (n-1) iterations of the coupling cycle comprising steps (b') to (e'), but does not include any coupling cycles optionally performed, e.g. to prepare component C-0 or any of the building blocks used. Each coupling cycle comprising steps (b') to (e') is herein identified by a serial number x, which runs in steps of 1 from 2 to n, with n being said integer representing the total amount of coupling cycles performed to obtain the n-th cycle oligonucleotide O-n.

In some embodiments of the method of the invention, n is an integer in the range of 2-89, 2-79, 2-69, 2-59, 2-49, 2-39, 2-29, or 2-19.

One will understand that for, e.g. n=2, the method according the invention comprises a total of 2 coupling cycles: The first coupling cycle comprising the steps (b) to (e), and (n-1)=1, i.e. one, iteration of the coupling cycle comprising the steps (b') to (e') the latter being numbered with x=2=n. For n=30, the method according to the invention comprises a total of 30 coupling cycles: The first coupling cycle comprising the steps (b) to (e), and (n-1), i.e. 29, iterations of the coupling cycle comprising the steps (b') to (e'), the latter being numbered with consecutive integers x running from 2 to 30.

The final iteration of the coupling cycle comprising steps (b') to (e') is referred to as the the n-th cycle, i.e. x=n. Thus, the serial number x is herein used to identify any specific coupling cycle comprising the steps (b') to (e'), and the building blocks employed in, as well as the oligonucleotides obtained from said coupling cycle. It will be understood that, for example, said building block B-x is, e.g., the building block B-5 in the fifth coupling cycle (x=5). It will also be understood that, for example, said x-th cycle oligonucleotide O-x is, e.g., the fifth cycle oligonucleotide O-5 in the fifth coupling cycle. One will readily understand that, as the first coupling cycle comprising the steps (b) to (e) is counted in the total number of coupling cycles, the fifth coupling cycle is the fourth iteration of the coupling cycle comprising the steps (b') to (e'). Thus, the serial number x does not denote the *number of iterations* of the coupling cycle comprising the steps (b') to (e'). Instead, x is simply used to consecutively number the coupling cycles comprising the steps (b') to (e'). In other words: x is used to consecutively number the coupling cycles from the second coupling cycle onwards.

It will further be understood that, because x starts at 2 and runs in steps of 1, the coupling cycle previous to any given coupling cycle x may be referred to as the coupling cycle (x-1) and the oligonucleotide obtained in this previous coupling cycle may be referred to as the (x-1)-th cycle oligonucleotide O-(x-1). Thus, the x-th coupling cycle will start with removing the protecting group PG-(x-1) from the (x-1)-th-cycle oligonucleotide O-(x-1) of the previous coupling cycle. The x-th cycle oligonucleotide O-x of the coupling cycle x will then be obtained by reacting the deprotected (x-1)-th-cycle oligonucleotide (O-(x-1))^{#} with the building block B-x. For example, in the second coupling cycle, the (x-1)-th cycle oligonucleotide O-(x-1) will be the first cycle oligonucleotide O-1 of step (d) or (e). As another example, in the third coupling cycle, the (x-1)-th cycle oligonucleotide O-(x-1) will be the second cycle oligonucleotide O-2 of step (d') or (e'). It will further be understood that the n-th cycle is the final coupling cycle yielding the n-th cycle oligonucleotide O-n.

Step (a) of the method of the invention is: providing a component C-0 selected from the group consisting of a nucleoside and an oligonucleotide, wherein the component C-0 is covalently linked to a solid support and comprises a backbone hydroxyl moiety protected by a protecting group PG-0 comprising an optionally substituted triarylmethyl residue.

The terms "nucleoside" and "oligonucleotide" have been explained above. In some embodiments of the method of the invention, the component C-0 is a nucleoside. In some embodiments of the method of the invention, the component C-0 is an oligonucleotide. In some embodiments of the method of the invention, the component C-0 is an oligonucleotide comprising not more than 50, 40, 30, 25, 20, 15, 10, or 5 nucleoside subunits. In some embodiments of the method of the invention, the component C-0 is selected from the group consisting of a nucleoside and an oligonucleotide comprising not more than 50, 40, 30, 25, 20, 15, 10, or 5 nucleoside subunits.

The term "backbone hydroxyl moiety" refers to a hydroxyl moiety which is part of the backbone of the component C-0 and will be understood based on the above explanations of the terms "hydroxyl moiety" and "backbone". Preferably, said hydroxyl moiety is a hydroxyl moiety of a carbohydrate moiety, in particular of an optionally substituted and/or protected ribose or 2'-deoxyribose moiety. Preferably, said backbone hydroxyl moiety is comprised in a terminal nucleoside subunit of the component C-0. In some preferred embodiments, the component C-0 comprises exactly one backbone hydroxyl moiety protected by a protecting group PG-0 comprising an optionally substituted triarylmethyl residue. In some preferred embodiments, the component C-0 comprises exactly one protecting group comprising an optionally substituted triarylmethyl residue, which is said protecting group PG-0.

In some embodiments of the method of the invention, said nucleoside or oligonucleotide, which is covalently linked to a solid support and comprises a backbone hydroxyl moiety protected by a protecting group PG-0, e.g. the component C-0, is a compound of the following Formula I: 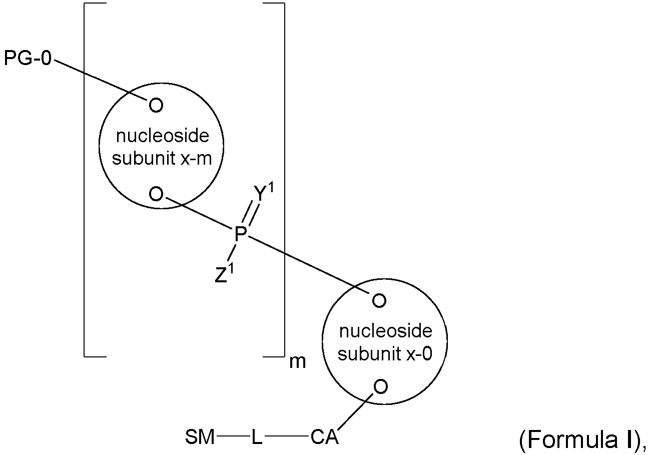 wherein in in Formula I:
each oxygen atom (O) depicted within each nucleoside subunit x-0 to x-m represents the oxygen atom of a hydroxyl moiety of the respective nucleoside subunit;
each of the nucleoside subunits x-0 to x-m may be the same or different (i.e. have the same or a different chemical structure);
PG-0 is a protecting group comprising an optionally substituted triarylmethyl residue, preferably a di(p-methoxyphenyl)phenylmethyl (DMT) protecting group;
m is an integer equal to or larger than 0;
Y¹ is selected independently for each repetitive unit m from the group consisting of O and S;
Z¹ is selected independently for each repetitive unit m from the group consisting of O-R^{z-1} and S-R^{z-1};
R^{z-1} is a protecting group, which may be the same or different for each repetitive unit m;
CA is a capping moiety or a covalent chemical bond;
L is a linker moiety or a covalent chemical bond; and
SM is a solid support.

In some embodiments of the method of the invention, said nucleoside or oligonucleotide, which is covalently linked to a solid support and comprises a backbone hydroxyl moiety protected by a protecting group PG-0, e.g. the component C-0, of Formula I is a compound of the following Formula I-a: 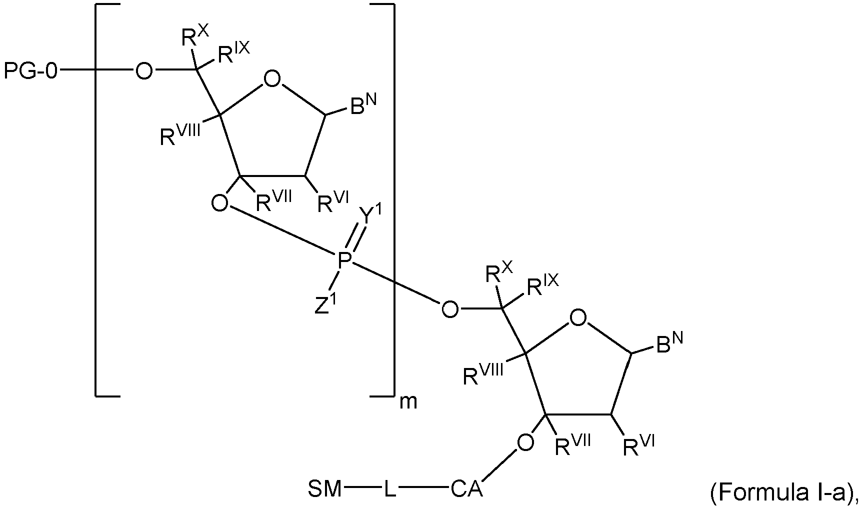 wherein in Formula I-a:
m, PG-0, Y¹, Z¹, R^{z-1}, CA, L, and SM are defined as for Formula I;
B^{N} is a nucleobase, which may be the same or different at each occurrence;
R^{VI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the following Formula I-a-tc: 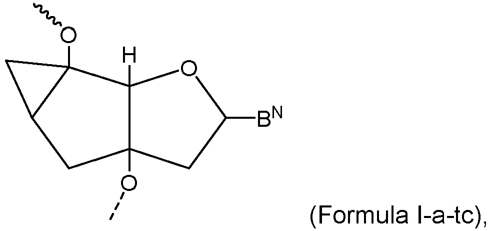
wherein in Formula I-a-tc:
   the oxygen atom from which the dashed line originates represents the oxygen atom bonded to the 3'-carbon atom, i.e. the carbon atom to which R^{VII} is bonded in Formula I-a;
   the dashed line indicates the covalent chemical bond interconnecting the respective oxygen atom of the nucleoside subunit of Formula I-a-tc and either CA-L-SM or P(Y¹)(Z¹) in Formula I-a;
   the oxygen atom from which the wavy line originates represents the oxygen atom bonded to the 5'-carbon atom, i.e. the carbon atom to which C(R^{IX})(R^{X}) is bonded in Formula I-a; and
   the wavy line indicates the covalent chemical bond interconnecting the respective oxygen atom of the nucleoside subunit of Formula I-a-tc and either PG-0 or P(Y¹)(Z¹) in Formula I-a.

In some embodiments of the method of the invention, said nucleoside or oligonucleotide, which is covalently linked to a solid support and comprises a backbone hydroxyl moiety protected by a protecting group PG-0, e.g. the component C-0 of Formula **I,** in particular of Formula I-a, is a compound of the following Formula I-b: 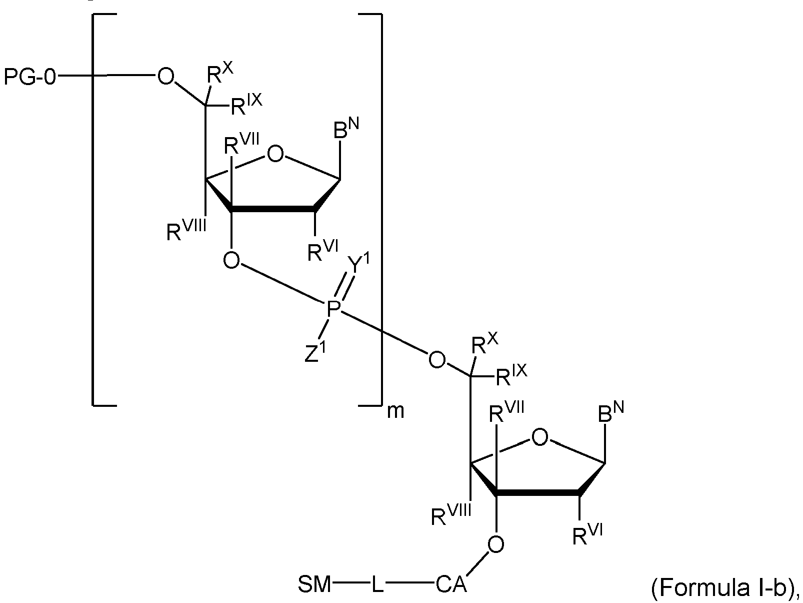 wherein in Formula I-b:
m, PG-0, Y¹, Z¹, R^{z-1}, B^{N}, CA, L, SM, R^{VI} , and R^{VIII} are defined as for Formula I-a, and
for each nucleoside subunit independently, R^{VII}, R^{IX}, and R^{X} are either all H or they are bonded together so that the respective nucleoside subunit has a structure of the following Formula I-b-tc: 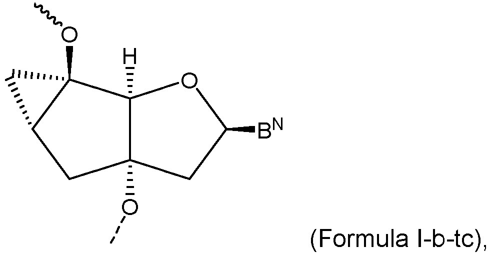
wherein in Formula I-b-tc:
   the oxygen atom from which the dashed line originates represents the oxygen atom bonded to the 3'-carbon atom, i.e. the carbon atom to which R^{VII} is bonded in Formula I-b;
   the dashed line indicates the covalent chemical bond interconnecting the respective oxygen atom of the nucleoside subunit of Formula I-b-tc and either CA-L-SM or P(Y¹)(Z¹) in Formula I-b;
   the oxygen atom from which the wavy line originates represents the oxygen atom bonded to the 5'-carbon atom, i.e. the carbon atom to which C(R^{IX})(R^{X}) is bonded in Formula I-b; and
   the wavy line indicates the covalent chemical bond interconnecting the respective oxygen atom of the nucleoside subunit of Formula I-b-tc and either PG-0 or P(Y¹)(Z¹) in Formula I-b.

In some embodiments, in said nucleoside or oligonucleotide, which is covalently linked to a solid support and comprises a backbone hydroxyl moiety protected by a protecting group PG-0, e.g. the component C-0, of any one of Formulae I, I-a, and I-b, CA is a covalent chemical bond and L is a linker moiety. It will be understood that in such embodiments, CA in any one of Formulae I, I-a, and I-b is a covalent chemical bond interconnecting the 3'-O atom of the respective nucleoside subunit (e.g. nucleoside subunit x-0 in Formula I) and the linker moiety L. In some embodiments, in said nucleoside or oligonucleotide, which is covalently linked to a solid support and comprises a backbone hydroxyl moiety protected by a protecting group PG-0, e.g. the component C-0, of any one of Formulae I, I-a, and I-b, L is a covalent chemical bond and CA is a capping moiety. It will be understood that in such embodiments, L in any one of Formulae I, I-a, and I-b is a covalent chemical bond interconnecting the capping moiety CA and the solid support SM. In some embodiments in said nucleoside or oligonucleotide, which is covalently linked to a solid support and comprises a backbone hydroxyl moiety protected by a protecting group PG-0, e.g. the component C-0, of any one of Formulae I, I-a, and I-b, CA and L are a covalent chemical bond. It will be understood that in such embodiments, both CA and L in any one of Formulae I, I-a, and I-b together represent the same covalent chemical bond interconnecting the 3'-O atom of the respective nucleoside subunit (e.g. nucleoside subunit x-0 in Formula I) and the solid support SM. Hence, in such embodiments, CA and L do not represent two adjacent covalent chemical bonds.

As used herein, the term "solid support" refers to a macroscopic insoluble, solid or gel-like substrate, wherein the term "insoluble" refers to the solvents used throughout the oligonucleotide synthesis. Herein, in line with common practice, the terms "solid support", "insoluble support", and "solid phase" or "solid-phase" are used interchangeably. These terms encompass the terms "resin" and "[resin]", which may be understood in the broadest sense as a particulate structure usable for solid-phase oligonucleotide synthesis. Widely used examples of suitable solid supports are controlled pore glass (CPG), e.g. long-chain alkylamine-CPG bead supports, cross-linked polystyrene beads, and cross-linked polystyrene-PEG composites, e.g. Tentagel^{™} resins. Since the solid support is insoluble in the solvents used for oligonucleotide synthesis, the conjugates of the solid support and the growing oligonucleotide chains are insoluble as well, so that they may be isolated from a reaction mixture (a liquid phase) by simple filtration, e.g. by draining the solvent through a filter or membrane or frit, which retains the conjugates of the solid support and the oligonucleotides inside the reactor. In oligonucleotide synthesis, the components of the reaction mixture will typically differ for the different steps of a coupling cycle, e.g. a coupling cycle comprising steps (b) to (e). Thus, being able to drain the reaction mixture while retaining the growing oligonucleotide chains inside the reactor significantly facilitates the process. Additionally, washing steps, in which the conjugates of the solid support and the growing oligonucleotide chains are typically treated with neat solvent, followed by draining of said solvent, may be implemented in between the steps of a coupling cycle, so as to remove excess reagents or reactants as well as any soluble byproducts or side products. The growing oligonucleotide chains are typically bonded (directly or via a linker) to the solid support via a functional group, typically a hydroxyl moiety, for which the solid support serves as permanent protecting group. Thus, herein, the terms "protecting group" and "permanent protecting group" (but not the term "temporary protecting group") embrace a solid support, unless indicated differently in the context of specific embodiments. Oligonucleotide synthesis using a solid support is herein also referred to as solid-phase oligonucleotide synthesis (SPOS). Any solid support employed in solid-phase oligonucleotide synthesis may be used in the method of the invention.

A "linker moiety" may be used to establish the linkage between the solid support and either a nucleoside subunit of the oligonucleotide to be synthesized or a capping moiety bonded to it. As used herein, the term "linker moiety" refers to a moiety, which exhibits at least two functional groups, wherein one of said functional groups is capable of engaging in a covalent chemical bond with a functional group on the solid support and the other of said functional groups is capable of engaging in a covalent chemical bond with a functional group on a nucleoside moiety of the oligonucleotide to be synthesized or a capping moiety bonded to it. Thus, the expressions "nucleoside or oligonucleotide (e.g. the component C-0) is covalently linked to a solid support" will be understood to embrace both a direct covalent bond between said nucleoside or oligonucleotide and said solid support as well as a covalent interconnection of said nucleoside or oligonucleotide and said solid support in form of a linker moiety.

Typically, solid supports may bear amino groups (e.g. aminomethyl groups of a polymer resin obtained from co-polymerization of poly(styrene) with divinylbenzene, followed by introduction of amino groups, e.g. by means of an aminomethylation reaction) and the linker may have a functional group, capable of forming an amide bond with such an amino group on the support. The functional group at the nucleoside moiety or the capping moiety which engages in the covalent chemical bond with the linker moiety may, for example, be a hydroxyl group or an amino group, preferably a hydroxyl group, in particular the 3'-hydroxyl group of a ribose or 2'-deoxyribose moiety. For example, a succinate-type linker moiety or a hydroquinone-O,O'-diacetic acid linker moiety, also referred to as "Q-linker", may be used, both of which are known to those skilled in the art. Other commonly used linkers are of the so-called universal linkers, which typically comprise a DMT-protected hydroxyl moiety, to which the first nucleoside subunit may be coupled (after initial detritylation). Non-limiting examples of suitable universal linkers are linkers of the "Unylinker" type described in US7202264.

In some embodiments, in said nucleoside or oligonucleotide, which is covalently linked to a solid support and comprises a backbone hydroxyl moiety protected by a protecting group PG-0, e.g. the component C-0, according to any one of Formulae I, I-a, and I-b, if L is a linker moiety, L is a moiety according to any one of the following Formulae L-I, L-II, L-III, and L-IV: 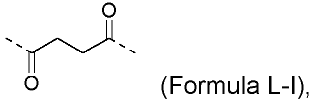 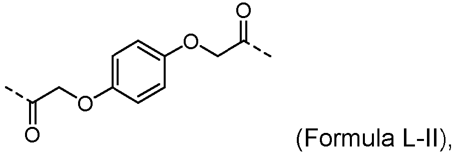 wherein in Formulae L-I and L-II:
one of the two dashed lines indicates a covalent chemical bond to a hydroxyl moiety of the capping moiety CA or to the respective hydroxyl moiety of the respective nucleoside subunit (e.g. nucleoside subunit x-0 in Formula I); and
the other of the two dashed lines indicates a covalent chemical bond to a functional group, preferably an amine or hydroxyl moiety, of the solid support; 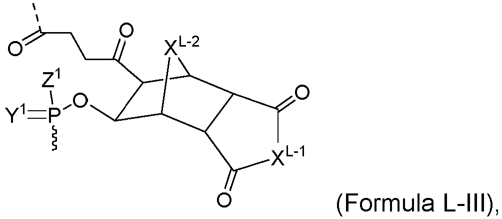 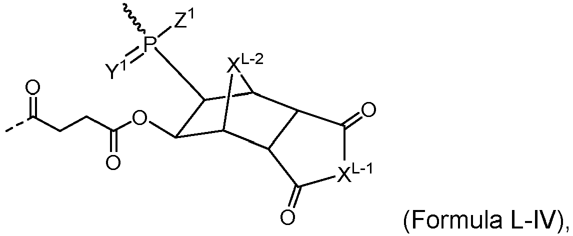
wherein in Formulae L-III and L-IV:
   Y¹ and Z¹ are defined as for Formula I;
   the wavy line indicates a covalent chemical bond to a hydroxyl moiety of the capping moiety CA or to the respective hydroxyl moiety of the respective nucleoside subunit (e.g. nucleoside subunit x-0 in Formula I);
   the dashed line indicates a covalent chemical bond to a functional group, preferably an amine or hydroxyl moiety, of the solid support;
   X^{L-1} is selected from the group consisting of O, S, and a nitrogen atom (N) to which a residue selected from the group consisting of H, a phenyl moiety (Ph), and a C₁-C₆-alkyl group is bonded; and
   X^{L-2} is selected from the group consisting of O, S, a carbon atom (C) to which two residues independently selected from the group consisting of H and a C₁-C₆-alkyl group are bonded, and a nitrogen atom (N) to which a residue selected from the group consisting of H, a C₁-C₆-alkyl group, and a phenyl (Ph) moiety is bonded.
   In such embodiments, it will be understood that, if CA is a covalent chemical bond, CA is one of the dashed lines of Formulae L-I and L-II or the wavy line in Formulae L-III and L-IV.

In some embodiments, in the linker moiety of any one of Formulae L-III and L-IV:
Y¹ is selected from the group consisting of O and S;
Z¹ is selected from the group consisting of O-R^{z-1}, S-R^{z-1};
R^{z-1} is a protecting group removable under alkaline conditions, preferably the 2-cyanoethyl group (i.e. CH₂-CH₂-CN);
X^{L-1} is selected from the group consisting of O, and a nitrogen atom (N) to which a residue selected from the group consisting of a phenyl moiety (Ph) and a C₁-C₆-alkyl group is bonded; and
X^{L-2} is selected from the group consisting of O, S, a methylene group (CH₂), and a nitrogen atom (N) to which a residue selected from the group consisting of CH₃, and phenyl (Ph) is bonded.

In some embodiments, in the linker moiety of any one of Formulae L-III and L-IV:
Y¹ is selected from the group consisting of O and S;
Z¹ is O-R^{z-1};
R^{z-1} is the 2-cyanoethyl group (i.e. CH₂-CH₂-CN);
X^{L-1} is selected from the group consisting of O, and a nitrogen atom (N) to which a residue selected from the group consisting of a phenyl moiety (Ph) and CH₃ is bonded; and
X^{L-2} is O.

In some embodiments, in the linker moiety of any one of Formulae L-III and L-IV:
Y¹ is selected from the group consisting of O and S;
Z¹ is O-R^{z-1};
R^{z-1} is the 2-cyanoethyl group (i.e. CH₂-CH₂-CN);
X^{L-1} is a nitrogen atom (N) to which a phenyl moiety (Ph) is bonded; and
X^{L-2} is O.

In some embodiments, in said nucleoside or oligonucleotide, which is covalently linked to a solid support, e.g. the component C-0, according to any one of Formulae I, I-a, and I-b, if L is a linker moiety, L is a moiety according to any one of Formulae L-I, L-II, L-III, and L-IV and CA is a covalent chemical bond (i.e. not a capping moiety).

As used herein, the term "capping moiety" refers to any moiety, which is conjugated to a terminal nucleoside of the oligonucleotide strand, wherein the capping moiety is not a nucleoside or oligonucleotide moiety, and preferably comprises a carbohydrate moiety. Examples are the carbohydrate-comprising moieties disclosed in WO2009073809.

In some embodiments in said nucleoside or oligonucleotide, which is covalently linked to a solid support and comprises a backbone hydroxyl moiety protected by a protecting group PG-0, e.g. the component C-0, of any one of Formulae I, I-a, and I-b, the integer m is an integer in the range of 0-150, 0-100, 0-75, 0-50, 0-35, 0-30, 0-25, 0-20, 0-15, 0-10, 0-5, or 0-1. It will be understood that to state than an integer is in the range of e.g. 0-5 means that said integer may be 0, 1, 2, 3, 4, or 5. In some embodiments, in said nucleoside or oligonucleotide, which is covalently linked to a solid support and comprises a backbone hydroxyl moiety protected by a protecting group PG-0, e.g. the component C-0, of any one of Formulae I, I-a, and I-b, the integer m is 0. It will be understood that, if the integer m in any one of Formulae I, I-a, and I-b is 0, the protecting group PG-0 is bonded to the respective hydroxyl moiety of the then remaining nucleoside subunit carrying -CA-L-SM (e.g. nucleoside subunit x-0 in Formula I).

In some embodiments, in said nucleoside or oligonucleotide, which is covalently linked to a solid support and comprises a backbone hydroxyl moiety protected by a protecting group PG-0, e.g. the component C-0, e.g. of any one of Formulae I, I-a, and I-b, the protecting group PG-0 is selected from the group consisting of the triphenylmethyl group (i.e. the trityl group), the (p-methoxyphenyl)diphenylmethyl group (i.e. the MMT group), and the di(p-methoxyphenyl)phenylmethyl group (i.e. the DMT group). In some preferred embodiments in said nucleoside or oligonucleotide, which is covalently linked to a solid support and comprises a backbone hydroxyl moiety protected by a protecting group PG-0, e.g. the component C-0, e.g. of any one of Formulae I, I-a, and I-b, the protecting group PG-0 is the di(p-methoxyphenyl)phenylmethyl group (i.e. the DMT group).

In some embodiments, in Formulae I, I-a, and I-b, Y¹ is for each repetitive unit m O. In some embodiments, in Formulae I, I-a, and I-b, Y¹ is for each repetitive unit m S.

In some embodiments, in Formulae I, I-a, and I-b, R^{z-1} is a protecting group removable under alkaline conditions, wherein R^{z-1} may be the same or different at each occurrence. As used herein, a protecting group is "removable under alkaline conditions", if it can be cleaved by treatment with a base (wherein "a base" is not to be construed to mean any base, but specific bases adapted to the protecting group to be removed based on common knowledge of those skilled in the art and routine experimentation). The term "base" may in this context be understood as proton acceptor in the sense of the Brønsted-Lowry theory. Examples of such a base include ammonia, in particular an aqueous solution of ammonia (i.e. an aqueous ammonium hydroxide solution), methylamine, tert-butylamine, diethylamine, triethylamine, diisopropylethylamine, and the like. In some embodiments, in any one of Formulae I, I-a, and I-b, R^{z-1} is for each repetitive unit m independently a protecting group of the chemical structure CH₂-CH₂-EWG, where EWG is an electron withdrawing group, preferably a cyano group. The electron withdrawing group may for example be selected from the group consisting of a cyano group, a halogen atom such as a chlorine, fluorine, or bromine atom, an aldehyde group, a keto group, a carboxyester group, or a carboxamide group. In some preferred embodiments, in any one of Formulae I, I-a, and I-b, R^{z-1} is for each repetitive unit m a 2-cyanoethyl group (i.e. CH₂-CH₂-CN).

In some embodiments, in any one of Formulae I, I-a, and I-b, Z¹ is selected independently for each repetitive unit m from the group consisting of O-R^{z-1} and S-R^{z-1}. In some embodiments, in any one of Formulae I, I-a, and I-b, Z¹ is selected independently for each repetitive unit m from the group consisting of O-R^{z-1} and S-R^{z-1}, where R^{z-1} is for each repetitive unit m a 2-cyanoethyl group (i.e. CH₂-CH₂-CN). In such embodiments, Z¹ is selected independently for each repetitive unit m from the group consisting of O-CH₂-CH₂-CN and S-CH₂-CH₂-CN. In some embodiments, in any one of Formulae I, I-a, and I-b, Z¹ is for each repetitive unit m O-R^{z-1}, where R^{z-1} is for each repetitive unit m a 2-cyanoethyl group (i.e. CH₂-CH₂-CN). In such embodiments, Z¹ is for each repetitive unit m O-CH₂-CH₂-CN.

In some embodiments, in any one of Formulae I, I-a, and I-b, the terminal nucleoside subunit, whose hydroxyl moiety is bonded to the PG-0 protecting group, is a nucleoside subunit comprising a purine type nucleobase, preferably a nucleobase selected from the group consisting of adenine and guanine, in particular adenine.

The skilled person will understand that any kind of nucleobase B^{N} may be present in any one of Formulae I-a, and I-b. Reference is e.g. made to the explanations relating to the nucleobase given above. In some embodiments, in any one of Formulae I-a, and I-b, B^{N} is a nucleobase and at each occurrence independently selected from the group consisting of adenine, guanine, cytosine, 5-methylcytosine, thymine, and uracil. It will be understood by those skilled in the art, that any nucleobase B^{N} in any one of Formulae I-a, and I-b may optionally be protected, i.e. carry one or more protecting groups, without this being indicated specifically. Thus, when, for example, stating that B^{N} is adenine, guanine, cytosine, 5-methylcytosine, thymine, or uracil, this embraces the aforementioned nucleobases in protected form and in free form (i.e. with and without any protecting groups). The same rationale applies to nucleobases in general.

The skilled artisan is familiar with nucleobase protecting groups and knows how to select, introduce, and remove them. In particular, the exocyclic amino groups in nucleobases such as in adenine, guanine, cytosine, and 5-methylcytosine may be protected. Non-limiting examples of protecting groups for exocyclic amino groups of nucleobases comprise the acetyl group, the benzoyl group, the isobutyryl group, the pivaloyl group, the pivaloyloxymethyl group, the trifluoroacetyl group, the phenoxyacetyl group, the 4-isopropylphenoxyacetyl group, the 4-*tert-*butylphenoxyacetyl group, and the dimethylformamidinyl group. Carbonyl groups present in nucleobases such as in thymine, uracil, and guanine may also be protected, for example, by reaction with phenol, 2,5-dichlorophenol, 3-chlorophenol, 3,5-dichlorophenol, 2-formylphenol, 2-naphthol, 4-methoxyphenol, 4-chlorophenol, 2-nitrophenol, 4-nitrophenol, 4-acetylaminophenol, pentafluorophenol, 4-pivaloyloxybenzyl alcohol, 4-nitrophenethyl alcohol, 2-(methylsulfonyl)ethanol, 2-(phenylsulfonyl)ethanol, 2-cyanoethanol, 2-(trimethylsilyl)ethanol, dimethylcarbamoyl chloride, diethylcarbamoyl chloride, ethylphenylcarbamoyl chloride, 1-pyrrolidinecarbonyl chloride, 4-morpholinecarbonyl chloride, diphenylcarbamoyl chloride, and the like. Examples of preferred nucleobase protecting groups are compiled in the following Table T-1.

**Table T-1: Non-limiting overview of preferred nucleobase protecting groups.**

| Protecting group | typically used for |
|---|---|
| Bz = benzoyl; iBu = isobutyryl; | exocyclic amino group of adenine |
| PAC = phenoxyacetyl | |
| Ac = acetyl; Bz = benzoyl | exocyclic amino group of cytosine or 5-methylcytosine |
| iBu = isobutyryl; | exocyclic amino group of guanine |
| i-Pr-PAC = 4-isopropylphenoxyacetyl); dimethylformamidino | |

In some embodiments of the method of the invention, each nucleobase of said nucleoside or oligonucleotide, which is covalently linked to a solid support and comprises a backbone hydroxyl moiety protected by a protecting group PG-0, e.g. the component C-0, in particular each nucleobase B^{N} in any one of Formulae I, I-a, and I-b is independently selected from the group consisting of
- adenine, in which the exocyclic amino group is protected;
- guanine, in which the exocyclic amino group is protected;
- cytosine, in which the exocyclic amino group is protected;
- 5-methylcytosine, in which the exocyclic amino group is protected;
- thymine; and
- uracil.

In some embodiments of the method of the invention, each nucleobase of said nucleoside or oligonucleotide, which is covalently linked to a solid support and comprises a backbone hydroxyl moiety protected by a protecting group PG-0, e.g. the component C-0, in particular each nucleobase B^{N} in any one of Formulae I, I-a, and I-b is independently selected from the group consisting of
- adenine, in which the exocyclic amino group is protected by a benzoyl group, an isobutyryl group or a phenoxyacetyl group;
- guanine, in which the exocyclic amino group is protected by an isobutyryl group, a 4-isopropylphenoxyacetyl group or a dimethylformamidino group;
- cytosine, in which the exocyclic amino group is protected by an acetyl group or a benzoyl group;
- 5-methylcytosine, in which the exocyclic amino group is protected by an acetyl group or a benzoyl group;
- thymine; and
- uracil.

In some embodiments, in any one of Formulae I-a and I-b:
R^{VI} is selected independently at each occurrence from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in Formula I-a) or Formula I-b-tc (in Formula I-b).

In some embodiments, in any one of Formulae I-a and I-b:
R^{III} is selected independently at each occurrence from the group consisting of H, F, O-CH₃ (i.e. methoxy), and O-CH₂-CH₂-O-CH₃ (i.e. 2-methoxyethyl-1-oxy); and
R^{IV} is at each occurrence H.

In some embodiments, in Formula I:
PG-0 is a di(p-methoxyphenyl)phenylmethyl (DMT) protecting group;
m is an integer in the range of 0-150, 0-100, 0-75, 0-50, 0-35, 0-30, 0-25, 0-20, 0-15, 0-10, 0-5, or 0-1;
Y¹ is selected independently for each repetitive unit m from the group consisting of O and S;
Z¹ is selected independently for each repetitive unit m from the group consisting of O-R^{z-1} and S-R^{z-1};
R^{z-1} is for each repetitive unit m a 2-cyanoethyl group;
CA is a covalent chemical bond;
L is a linker moiety or a covalent chemical bond; and
SM is a solid support.

In some embodiments, in Formula I:
PG-0 is a di(p-methoxyphenyl)phenylmethyl (DMT) protecting group;
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is selected independently for each repetitive unit m from the group consisting of O-R^{z-1} and S-R^{z-1};
R^{z-1} is for each repetitive unit m a 2-cyanoethyl group;
CA is a covalent chemical bond;
L is a linker moiety according to any one of the aforementioned Formulae L-I, L-II, L-III, and L-IV or a covalent chemical bond; and
SM is a solid support.

In some embodiments, in Formula I:
PG-0 is a di(p-methoxyphenyl)phenylmethyl (DMT) protecting group;
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is for each repetitive unit m O-R^{z-1};
R^{z-1} is for each repetitive unit m a 2-cyanoethyl group;
CA is a covalent chemical bond;
L is a linker moiety according to any one of the aforementioned Formulae L-I, L-II, L-III, and L-IV; and
SM is a solid support.

In some embodiments, in any one of Formulae I-a and I-b:
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is selected independently for each repetitive unit m from the group consisting of O-R^{z-1} and S-R^{z-1};
R^{z-1} is for each repetitive unit m a 2-cyanoethyl group;
CA is a covalent chemical bond;
L is a linker moiety or a covalent chemical bond;
SM is a solid support;
B^{N} is a nucleobase, which may be the same or different at each occurrence;
R^{VI} is at each occurrence independently selected from the group consisting of H, F, O-(C₁-C₅-alkyl), O-(C₁-C₅-alkyl)-O-(C₁-C₅-alkyl), O-Si(Ci-C₅-alkyl)₃, and O-CH₂-O-Si(C₁-C₅-alkyl)₃; and
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in Formula I-a) or Formula I-b-tc (in Formula I-b).

In some embodiments, in any one of Formulae I-a and I-b:
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is selected independently for each repetitive unit m from the group consisting of O-R^{z-1} and S-R^{z-1};
R^{z-1} is for each repetitive unit m a 2-cyanoethyl group;
CA is a covalent chemical bond;
L is a linker moiety according to any one of the aforementioned Formulae L-I, L-II, L-III, and L-IV or a covalent chemical bond;
SM is a solid support;
B^{N} is a nucleobase, which may be the same or different at each occurrence;
R^{VI} is at each occurrence independently selected from the group consisting of H, F, O-(C₁-C₅-alkyl), O-(C₁-C₅-alkyl)-O-(C₁-C₅-alkyl), O-Si(Ci-C₅-alkyl)₃, and O-CH₂-O-Si(C₁-C₅-alkyl)₃; and
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in Formula I-a) or Formula I-b-tc (in Formula I-b).

In some embodiments, in any one of Formulae I-a and I-b:
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is for each repetitive unit m O-R^{z-1};
R^{z-1} is for each repetitive unit m a 2-cyanoethyl group;
CA is a covalent chemical bond;
L is a linker moiety or a covalent chemical bond;
SM is a solid support;
B^{N} is a nucleobase, which may be the same or different at each occurrence;
R^{VI} is selected independently at each occurrence from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in Formula I-a) or Formula I-b-tc (in Formula I-b).

In some embodiments, in any one of Formulae I-a and I-b:
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is for each repetitive unit m O-R^{z-1};
R^{z-1} is for each repetitive unit m a 2-cyanoethyl group;
CA is a covalent chemical bond;
L is a linker moiety according to any one of the aforementioned Formulae L-I, L-II, L-III, and L-IV or a covalent chemical bond;
SM is a solid support;
B^{N} is a nucleobase, which may be the same or different at each occurrence;
R^{VI} is selected independently at each occurrence from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy); and
R^{VII}, R^{VIII}, R^{IX}, and R^{X} are at each occurrence H.

In some embodiments, in any one of Formulae I-a and I-b:
m is an integer in the range of 0-50, 0-35, 0-30, 0-25, 0-20, 0-15, 0-10, 0-5, or 0-1;
Y¹ is selected independently for each repetitive unit m from the group consisting of O and S;
Z¹ is for each repetitive unit m O-R^{z-1};
R^{z-1} is for each repetitive unit m a 2-cyanoethyl group;
CA is a covalent chemical bond;
L is a linker moiety according to any one of the aforementioned Formulae L-I, L-II, L-III, and L-IV;
SM is a solid support;
B^{N} is a nucleobase, which may be the same or different at each occurrence;
R^{VI} is selected independently at each occurrence from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy); and
R^{VII}, R^{VIII}, R^{IX}, and R^{X} are at each occurrence H.

The term "providing a component C-0" in step (a) may be understood in the broadest sense. As such, "providing" may mean to make available or introduce the component C-0. For example, nucleoside loaded solid supports for many standard nucleosides may simply be acquired from commercial suppliers such as Merck and Cytiva, among many others. If the linker moiety is a succinate-type linker, e.g. of Formula L-1, or a hydroquinone-type linker, e.g. of Formula L-II, the first nucleoside subunit may typically already be bonded to the solid support via said linker, when obtaining it commercially. Alternatively, appropriately protected nucleosides may be loaded to a given solid support. For example, when using universal linkers, e.g. of Formula L-III or L-IV, the first nucleoside subunit is typically not loaded on the commercially obtained supports, so that step (a) may then comprise coupling the first nucleoside subunit (e.g. as phosphoramidite) to the universal linker support (from which a hydroxyl-protecting group has been removed beforehand, see for example US7202264). All of this is common practice in the field of oligonucleotide synthesis and well-known to those skilled in the art. The skilled person is aware of a plethora of possible immobilization strategies for this purpose and will routinely select one. Solid support-bound oligonucleotides may typically not be obtained commercially, but preferably be synthesized by any means. Again, the skilled artisan is well-aware of numerous ways of obtaining a component C-0 by means of oligonucleotide synthesis, for example, by means disclosed in US7202264B2, and R.T. Pon et al., Nucleic Acids Research 1997, 25(18), 3629-3635.

Step (a) of the method of the invention may involve conditioning the solid support by washing steps with various solvents. These steps may typically involve treating the solid support to which the component C-0 is covalently linked with the respective solvent, e.g. DMF, followed by draining said solvent.

Step (b) of the method of the invention is: incubating a nucleoside or oligonucleotide, which is covalently linked to a solid support and comprises a backbone hydroxyl moiety protected by a protecting group PG-0 comprising an optionally substituted triarylmethyl residue, with a deprotection mixture M-b, thereby cleaving the protecting group PG-0 from the nucleoside or oligonucleotide, wherein said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety.

If step (b) is performed after step (a) and as part of said first coupling cycle comprising steps (b) to (e), step (b) is: incubating the component C-0 of step (a) with a deprotection mixture M-b, thereby cleaving the protecting group PG-0 from the component C-0, so as to obtain a component C-0^{#} having a free backbone hydroxyl group.

Step (b') of the method of the invention is: incubating the (x-1)-th cycle oligonucleotide O-(x-1) obtained in the coupling cycle, i.e. the (x-1)-th coupling cycle, with a deprotection mixture M-b', thereby cleaving the protecting group PG-(x-1) from the (x-1)-th cycle oligonucleotide O-(x-1), so as to obtain a (x-1)-th cycle oligonucleotide (O-(x-1))^{#} having a free backbone hydroxyl group.

Said "nucleoside or oligonucleotide, which is covalently linked to a solid support and comprises a backbone hydroxyl moiety protected by a protecting group PG-0 comprising an optionally substituted triarylmethyl residue" has been defined. It may be the component C-0 which has also been defined. The term "cleaving a protecting group" has been defined. The protecting group PG-0 has been explained above. The protecting group PG-(x-1) also is a "protecting group comprising an optionally substituted triarylmethyl residue" as defined above. In some embodiments of the method of the invention, the protecting group PG-(x-1) is for each iteration of the coupling cycle selected from the group consisting of the triphenylmethyl group (i.e. the trityl group), the (*p*-methoxyphenyl)diphenylmethyl group (i.e. the MMT group), and the di(*p*-methoxyphenyl)phenylmethyl group (i.e. the DMT group). In some preferred embodiments of the method of the invention, the protecting group PG-(x-1) is for each iteration of the coupling cycle a di(*p-*methoxyphenyl)phenylmethyl group (i.e. the DMT group).

In some embodiments of the method of the invention, each protecting group comprising an optionally substituted triarylmethyl residue is a di(p-methoxyphenyl)phenylmethyl protecting group.

The (x-1)-th cycle oligonucleotide O-(x-1) has been explained to refer to the oligonucleotide obtained in the previous coupling cycle, i.e. the (x-1)-th coupling cycle. For example, in step (b') of the fifth iteration x = 5 of the coupling cycle, said (x-1)-th cycle oligonucleotide O-(x-1) is the fourth cycle oligonucleotide O-4. The serial number x is identical for an oligonucleotide and its protecting group PG. For example, the fourth cycle oligonucleotide O-4 comprises the protecting group PG-4 and the fifth cycle oligonucleotide comprises the protecting group PG-5.

The terms "backbone" and "free hydroxyl group" have been explained above. As used herein, a free backbone hydroxyl group is a free hydroxyl group located in the backbone of the respective nucleoside or oligonucleotide, for example a free 5'-OH group of a ribose or 2'-deoxyribose moiety. For the ease of understanding, the products obtained from a step (b) or (b'), all of which comprise per definition a free backbone hydroxyl group are denoted by a number sign (i.e. hashtag) in superscript ^{"#"}. Thus, the component obtained from submitting component C-0 to step (b) is referred to as component C-0^{#}. Along the same lines, the oligonucleotide obtained from submitting an (x-1)-th cycle oligonucleotide (O-(x-1)) obtained in the previous coupling cycle to step (b') is referred to as (x-1)-th cycle oligonucleotide (O-(x-1))^{#}.

In some embodiments of the method of the invention:
- the component C-0^{#} differs from the component C-0 only in that the protecting group PG-0 is absent, so that the hydroxyl moiety previously protected by the protecting group PG-0 is now present as a free hydroxyl group; and
- each (x-1)-th cycle oligonucleotide (O-(x-1))^{#} differs from the corresponding (x-1)-th cycle oligonucleotide O-(x-1) only in that the protecting group PG-(x-1) is absent, so that the hydroxyl moiety previously protected by the protecting group PG-(x-1) is now present as a free hydroxyl group.

The term "incubating" may in the context of step (b) refer to any process of combining said nucleoside or oligonucleotide, which is covalently linked to a solid support and comprises a backbone hydroxyl moiety protected by a protecting group PG-0 comprising an optionally substituted triarylmethyl residue, e.g. said component C-0, and said deprotection mixture M-b in a reaction vessel or reactor (e.g. a batch reactor or a column reactor). Typically, the reaction vessel or reactor may already contain said nucleoside or oligonucleotide, e.g. the component C-0, followed by addition of the deprotection mixture M-b.

The term "incubating" may in the context of step (b) refer to any process of combining said nucleoside or oligonucleotide, which is covalently linked to a solid support and comprises a backbone hydroxyl moiety protected by a protecting group PG-0 comprising an optionally substituted triarylmethyl residue, e.g. said component C-0, and said deprotection mixture M-b in a reaction vessel or reactor (e.g. a batch reactor or a column reactor). Typically, the reaction vessel or reactor may already contain said nucleoside or oligonucleotide, e.g. the component C-0, followed by addition of the deprotection mixture M-b. The term "incubating" may in the context of step (b') refer to any process of combining the (x-1)-th cycle oligonucleotide O-(x-1) obtained in the previous coupling cycle and a deprotection mixture M-b' in a reaction vessel or reactor (e.g. a batch reactor or a column reactor). Typically, the reaction vessel or reactor may already contain the (x-1)-th cycle oligonucleotide O-(x-1), followed by addition of the deprotection mixture M-b'.

Solid-phase oligonucleotide synthesis is commonly carried out using column reactors. As used herein, the term "column reactor" may be understood in the broadest sense as any reactor in which a column (e.g. made of stainless steel or glass or another material) is packed with a solid support, to which the growing oligonucleotide chains are tethered, wherein said column does preferably not comprise a mechanical stirrer or another agitator. Instead, any reaction solutions and solvents for washing are typically passed through the column, usually from top to bottom, but alternatively also alternatingly from top to bottom and from bottom to top to achieve fluidization of the packed support. The reaction solutions and solvents for washing may also be circulated through the column reactor (i.e. pass the column more than once). A column reactor may typically comprise a bottom frit and a top frit, with the solid support (to which the growing oligonucleotide chains are tethered) packed in between. The size and dimensions of the reaction column can be chosen according to the scale of synthesis intended. For example, column reactors with a minimal inner volume of 5, 10, 30, 40, 50, 60, 70, 75, 80, 90, 100, 110, 120, 140, 150, 160, 170, 180, 200, 220, 240, 260, 280, or 300 liters may be used. In certain embodiments, column reactors with a maximal inner volume of 1 to 500 liters, of 5 to 450 liters, of 10 to 400 liters, of 50 to 300 liters, or 100 to 300 liters may be used. The terms "fixed bed", "tight bed", and "packed bed" have been coined interchangeably for column reactors, in which the solid support carrying the growing oligonucleotide chains is not agitated, and the liquids are pumped unidirectionally through a settled bed of the solid support (also referred to as resin bed), which includes, e.g., unidirectional circulation of liquids through a loop comprising the column. This fixed bed approach is the current gold standard in automated solid-phase oligonucleotide synthesis. Fixed bed reactors have been known for decades (cf., e.g., US 6,623,703 B1 and US 6,825,339 B2) and are also commercially available, e.g., from Cytiva (cf., e.g., the OligoPilot^{™} oligonucleotide synthesizer and the OligoProcess^{™} oligonucleotide synthesizer). Fixed bed reactors are suitable for carrying out the method of the present invention. However, the method of the present invention is not limited to such reactors. Column reactors may also be operated as agitated bed reactors, which herein means that the suspension of the solid support inside the column is not densely packed and, at least once during the oligonucleotide synthesis, agitated by any means. Suitable means for agitation include sparging (in other words: bubbling) with an inert gas such as nitrogen and directing the liquid flow up and down inside the reactor (i.e., bidirectional flow) as, e.g., disclosed in EP 0130739 A2. Although rather uncommon in column reactors, stirring, e.g., mechanical stirring, constitutes an alternative means of agitation.

When using a column reactor, step (b) may comprise passing or circulating a deprotection mixture M-b through said column at a certain temperature, for a certain time, and with a certain flow rate. The present inventors surprisingly found that the flow rate of the deprotection mixture M-b, in particular if the deprotection mixture M-b is a liquid composition C, through the column reactor may optionally be rather low, e.g. below 300 cm/h, below 290 cm/h, below 280 cm/h, below 270 cm/h, 260 cm/h, below 250 cm/h, below 240 cm/h, below 230 cm/h, 220 cm/h, below 210 cm/h, below 200 cm/h, below 190 cm/h, below 180 cm/h, or below 160 cm/h. This increases the scalability of the process drastically. The same rationale applies to step (b') and the deprotection mixture M-b'.

In some embodiments of the method of the invention, the method is carried out in a column reactor and in step (b), the flow rate of said liquid composition C through the column reactor is below 300 cm/h. In some embodiments of the method of the invention, the method is carried out in a column reactor and in steps (b) and (b'), the flow rate of the liquid composition C through the column reactor is below 300 cm/h.

As an alternative, the inventors found that a batch reactor may be used with the methods of the present invention. As used herein, the term "batch reactor" may be understood in the broadest sense as any reaction vessel (e.g. any kind of tank) comprising an agitator (i.e. a mechanism or equipment allowing to agitate the contents of said vessel), so that the reactor allows for the suspension of a solid support in a solution or liquid composition. Any means of agitating the contents of the reaction vessel may be used. On large scale, for example, a mechanical stirrer may be employed. Alternatively, for example, in automated synthesizers for solid-phase oligonucleotide synthesis, agitation may be achieved by bubbling an inert gas (e.g. nitrogen) through the vessel containing the reaction mixture. A batch reactor for solid-phase oligonucleotide synthesis preferably comprises a membrane or filter allowing to drain any liquids from the reactor while retaining the solid support and conjugates of said solid support and the growing oligonucleotide chains inside the reactor. A type of batch reactor is a so-called stirred-bed reactor. As used herein, the term "stirred-bed reactor" refers to a batch reactor comprising a mechanical stirrer. Batch reactors for oligonucleotide synthesis are generally known to those skilled in the art. The term "stirred-bed reactor" has been coined for reactors, in which agitation of the solid support is at least partly achieved by stirring, typically mechanical stirring. Such reactors are known to the skilled artisan and are commercially available, e.g., from Büchi AG (Uster, Switzerland) (c.f., e.g., https://www.buchiglas.ch/fileadmin/buchiglas_international/download/dvs/Brochure s_and_Flyers/G_filter_reactors_flyer.pdf). It is a preferred option to carry out the method of the present invention in a stirred-bed reactor. As another example, CN 107881102 A discloses a stirred-bed reactor, which may be used for the method of the present invention. As another example, WO 00/66258 A2 discloses a stirred-bed reactor, which may be used for the method of the present invention. In this reactor, agitation of the suspension of the solid support is achieved by means of mechanical stirring and, optionally, by nitrogen bubbling. The term "sparged-bed reactor" has been coined for reactors, in which agitation of the solid support is at least partly achieved by sparging with an inert gas, typically nitrogen. Usually, the inert gas is bubbled through the suspension of the solid support from bottom to top, preferably through the bottom frit, which may have the additional benefit of preventing clogging of the bottom frit. Sparged-bed reactors have been used in solid-phase oligonucleotide synthesis for decades (cf., e.g., A.A. Padmapriya et al., Antisense Research and Development 1994, 4, 185-199, https://doi.org/10.1089/ard.1994.4.185; N.D. Sinha, "Large-Scale Oligonucleotide Synthesis Using the Solid-Phase Approach" in: S. Agrawal, "Protocols for Oligonucleotides and Analogs", Methods in Molecular Biology 1993, vol 20, Humana Press. https://doi.org/10.1385/0-89603-281-7:437) and are also suitable for the method of the present invention. Mechanical stirring and inert gas sparging may be used in combination. As also disclosed in Padmapriya et al., agitation may be achieved by simply shaking the reaction vessel. Such reactors are also suitable for the method of the present invention. Agitation in batch reactors may also be achieved by inverting the reaction vessel relative to a resting position as, e.g., disclosed in US 11,524,976 B2. Such reactors are also suitable for the method of the present invention. When applying the method of the present invention to large-scale synthesis (e.g., a scale of at least 0.1, 0.2, 0.3, 0.4, 0.5, 0.6, 0.7, 0.8, 0.9, 1.0, 2, 3, 4, 5, 6, 7, 8, 9, 10, 11, 12, 13, 14, 15, 20, 25, 30, or 35 mol of the target oligonucleotide O^{T}), stirred-bed reactors and sparged-bed reactors may be preferred, and stirred-bed reactors may be most preferred. The skilled artisan understands that reactors and devices for solid-phase peptide synthesis are usually also suitable for solid-phase oligonucleotide synthesis by the method of the present invention, after some obvious adaptions such as replacing the peptide synthesis reagents for oligonucleotide synthesis reagents and the peptide synthesis support for an oligonucleotide synthesis support. For example, automated peptide synthesizers such as Symphony^{®}, Symphony^{®} X or PurePep^{®} Sonata^{®} + by Gyros Protein Technologies may be used for the method of the present invention.

Batch reactors may typically be cylindrical with a top and a bottom enclosure. The top enclosure usually comprises one or more inlets for addition of the solid support, the wash solvent(s), and reagents. If a mechanical stirrer is employed, the stirring assembly is usually located above the reactor with a stirring shaft extending through an opening (usually a central port) in the top enclosure. The bottom enclosure usually comprises some sort of filtration means, e.g., a membrane or frit, capable of retaining the solid support and any oligonucleotide chains bound to it inside the reactor when draining the liquids from the reactor. Draining of the liquids may, optionally, be facilitated or accelerated by applying inert gas pressure (e.g., nitrogen pressure) to the reactor and/or by applying vacuum from the bottom of the reactor (i.e., beneath the filtration means). The bottom enclosure may also be designed to allow for inert gas sparging, i.e., bubbling of an inert gas such as nitrogen from the reactor bottom, through the suspension of the solid support. Batch reactors may be fabricated from any suitable material such as, e.g., stainless steel and glass, wherein stainless steel may be preferred on industrial scales. In batch reactors, reaction solutions and wash solvents may, for example, be added through inlets located at the top of the reactor. One or more further inlets and outlets may be used to add and remove gasses, in particular to create an inert gas atmosphere, e.g., a nitrogen atmosphere. Nitrogen pressure may be used to assist the draining of liquids. The wash solvents may be added from the reactor top to rinse down any material, which might be stuck to the reactor wall. The inlets may be designed so that the added liquids rinse the reactor wall. The inlets may, e.g., take the form of nozzles or spray cones, in particular, if it is desired to add the wash solvents or reagents without disturbing the resin bed. The arrangement of the inlets is not particularly limited. A separate inlet may be used for the wash solvents and some or each of the reagents. Using separate inlets may be beneficial for that it may reduce the risk of cross-contamination. Alternatively, all or some wash solvents and reagents may be added through the same inlet. In this case, it may be useful to flush the inlet with neat solvent(s) after reagent addition, to prevent cross-contamination. Reaction solutions and wash solvents may, for example, also be added through inlets located at the bottom of the reactor, preferably below the filter or membrane or frit. In such cases, the added liquids will typically enter the vessel through the filter or membrane or frit. In this fashion, the added liquids may cause agitation of the suspension of the solid support, and, potentially, prevent clogging of the filter or membrane or frit. Reaction solutions and wash solvents may also be added alternatingly from the top and from the bottom of the reactor. A reactor for solid-phase oligonucleotide synthesis can be designed so that it allows for controlling the temperature of its contents, e.g., by heating or by cooling. It is known that heating typically accelerates chemical reactions. Heating or cooling may, for example, be achieved by using jacketed (i.e., double-walled) reactors with a heating or cooling medium such as water circulating through the void space between the reactor walls. Other means of heating include microwave irradiation, infrared irradiation, and conductive heating. In such cases, the reactor material may need to be adapted accordingly, e.g., to be transparent to the respective irradiation. Alternatively, or additionally, the temperature of the reaction solution(s) and, optionally, wash solvent(s) may be adjusted before they enter the reaction vessel, e.g., via heating or cooling the storage tanks and/or the lines (i.e., tubing), which transport the reaction solutions or wash solvents to the reaction vessel. In particular, at an industrial scale, controlling the temperature of the storage tanks and/or the tubing may be preferred.

Also, the reactors (column reactors and batch reactors) disclosed in WO 2021/1100773 A1 may be used to carry out the method of the present invention. As another example, WO 2021/094518 A1 discloses reactors, which may be used for the method of the present invention.

Circulation loops are useful features of column reactors and batch reactors. Circulation loops are routinely used in combination with column reactors but can also advantageously be applied to batch reactors. For example, CN 107881102 A discloses batch reactors making use of a circulation loop. Such reactors are suitable for the method of the present invention. A circulation loop typically comprises a first port, through which the reaction medium or liquid can exit the reactor and enter the loop, and a second port, through which the reaction medium or liquid can again exit the loop and enter the reactor. Thus, reaction solutions and/or wash solvents may circulate through a loop, thereby repeatedly passing through a reaction vessel. Typically, a circulation loop may comprise one or more pumps or the like to force the reaction solutions and/or wash solvents through the loop. Advantages of circulation loops are known to those skilled in the art and include additional agitation as well as the option of including certain desirable features into the loop, e.g., instead of including them directly into the reaction vessel. For example, one or more measurement cells or detectors or probes of any kind may be located in the loop, to allow for real-time monitoring of reaction and/or wash steps, typically a coupling step and/or a deprotection step. As another example, a microwave irradiation center might be incorporated in the loop, so that the passing reaction medium or liquid can be subjected to microwave irradiation, which may speed up the reactions.

Online monitoring, also referred to as real-time monitoring, is known to those skilled in the art. Herein, these terms are used interchangeably and may be understood in the broadest sense to refer to any process of analyzing any reaction medium, liquid(s) or wash solvent(s) in the course of oligonucleotide synthesis, preferably to derive information on a reaction progress or wash progress. Two general strategies may be distinguished, both of which are suitable for the method of the present invention. In a fist strategy, the analysis takes place after removing a sample or even the entire reaction medium or liquid(s) or wash solvent(s) to be analyzed from the reactor, e.g., by draining through the filter or membrane or frit. In a second strategy, the reaction medium or liquid(s) or wash solvent(s) are analyzed inside the reactor and/or a circulation loop. The location of suitable detectors or measurement cells or probes may be adjusted to the desired strategy. In the first strategy, the detector or measurement cell or probe may typically be located outside the reaction vessel or column, usually beneath the filter or membrane or frit, preferably between the filter or membrane or frit and the waste or a storage tank. In the second strategy, the detector or measurement cell or probe may typically be located inside the reaction vessel or column and/or a circulation loop. If the utilized reactor comprises a circulation loop, it may be most convenient to place the detector or measurement cell or probe inside the loop, instead of directly inside the reaction vessel. However, it is also possible to place the detector or measurement cell or probe directly inside the reaction vessel, as, e.g., exemplified in T. Heilmann et al., Org. Process Res. Dev. 2023, 27, 65-77, https://doi.org/10.1021/acs.oprd.2c00203. Examples of suitable detectors comprise UV-detectors, UV-vis-detectors, infrared(IR)-detectors, conductivity detectors, NMR-detectors, as well as sensors or probes for measuring the density and/or or the refractive index, among others. UV(-vis)-spectroscopy (i.e., UV-detectors or UV-vis-detectors) may be particularly suitable for following the progress of a detritylation reaction such as, e.g., step (b) or (b') of the method of the present invention.

In solid-phase oligonucleotide synthesis, it is generally desirable to minimize or reduce the solvent consumption. The skilled artisan understands that reducing the volume of wash solvent(s) is a promising approach to reducing the overall solvent consumption of an oligonucleotide synthesis. In solid-phase oligonucleotide synthesis, usually, wash steps are carried out in between the reaction steps, e.g., in between step (a) and step (b) and/or step (b) and step (c) and/or step (c) and step (d) and/or step (d) and step (e) or (b') and/or step (e) and step (b') and/or step (b') and step (c') and/or step (c') and step (d') and/or step (d') and step (e') or (b') and/or step (e') and step (b') of the method of the present invention. A wash step may comprise one or more washings, during each of which the solid support carrying the growing oligonucleotide chains is treated with (i.e., incubated with) a wash solvent, followed by draining of the wash solvent. Additionally, or alternatively, a wash solvent may be passed through a settled bed of the solid support carrying the growing oligonucleotide chains (settled resin bed). The wash solvent may also be a mixture of solvents.

Different approaches are known for reducing the volume of wash solvent(s), wherein these approaches may also be used in combination. As a first approach, the volume of wash solvent per wash step and/or the number of wash steps per coupling cycle may be reduced as, e.g., disclosed in L. Xiao et al., Green Chem. 2023, 25, 4292-4301, https://doi.org/10.1039/D3GC00881A. This strategy can be used with the method of the present invention. The skilled artisan understands that this approach may not be limited to column reactors and may also be applied to batch reactors such as the stirred-bed reactors and the sparged-bed reactors disclosed herein and in the references cited herein. When carrying out the method of the present invention and wishing to reduce the overall solvent consumption, applying the strategy disclosed in L. Xiao et al. is advantageous, regardless of which reactor type is employed (e.g., column reactor or batch reactor). As a second approach, wash solvents may be (partly) recycled, i.e., reused. For example, B.I. Andrews et al., J. Org. Chem. 2021, 86, 49-61, https://dx.doi.org/10.1021/acs.joc.0c02291, teaches to collect the cleaner solvent from the end of a wash step and reuse it at the beginning of the next equivalent wash step. In this context, the term "equivalent wash step" refers to the corresponding wash step (e.g., the wash step after detritylation, e.g., in between steps (b) and (c) or steps (b') and (c')) of a subsequent coupling cycle. As apparent to those skilled in the art, the reactor setup may need to be adjusted to such solvent recycling, e.g., by introducing additional storage tanks to store the cleaner fractions of solvent, which are to be reused. Online monitoring may, for example, be used to judge on whether or not a fraction of a solvent should or should not be reused. The skilled artisan understands that wash solvents may not just be reused for the corresponding wash step in a subsequent coupling cycle but potentially also for another wash step in the same or in a subsequent coupling cycle. As one example, the cleaner fractions of the wash solvent used for the wash step after a coupling step (e.g., step (d) or (d') of the method of the present invention) may be reused in the wash step following the oxidation or sulfurization step (e.g., step (e) or (e') of the method of the present invention) in the same coupling cycle. When carrying out the method of the present invention and wishing to reduce the overall solvent consumption, applying a solvent recycling strategy is advantageous, regardless of which reactor type is employed (e.g., column reactor or batch reactor). As a third approach, wash steps may be conducted as displacement washes. As used herein, the term "displacement wash" refers to a process, in which a wash solvent is passed unidirectionally through a settled bed of the solid support without any additional agitation. Hence, displacement washing is distinct from dilution washing. As used herein, the term "dilution washing" refers to a process, in which the solid support to be washed is agitated in the wash solvent by any means, e.g., by stirring, nitrogen sparging or shaking, wherein, typically, several rounds of wash solvent addition, agitation, and draining of the wash solvent are performed - usually, each round with a fresh batch of the wash solvent. It will be understood that the solid support carries the growing oligonucleotide chains. The skilled artisan understands that displacement washing is routinely employed in fixed bed column reactors and typically requires less solvent(s) than dilution washing (cf., e.g., US 6,623,703 B1 and US 6,825,339 B2). In agitated bed column reactors and in batch reactors in general, in particular sparged-bed reactors and stirred-bed reactors, dilution washing is oftentimes employed, simply because the scale of the synthesis is typically rather small and solvent consumption therefore not too large. It is, however, clear that, when wishing to reduce the overall solvent consumption, in particular on an industrial scale, displacement washing can be applied in such reactors as well. Also, some wash steps may be performed as dilution washing and others may be performed as displacement washing. Also, within a single wash step, displacement washing and dilution washing may be combined. For example, a wash step may comprise a dilution wash portion, followed by a displacement wash portion or vice versa. For displacement washing in agitated bed column reactors or batch reactors in general, in particular stirred-bed reactors and sparged-bed reactors, the agitation of the solid support is typically stopped, and the support is allowed to settle to a so-called resin bed. Then, a wash solvent is passed unidirectionally through the non-disturbed, settled resin bed. Fresh solvent may be added in such a fashion that the settled resin bed is not disturbed, e.g., through spray cones or nozzles at the reactor top. Typically, it is desirable to implement a continuous flow of the wash solvent through the settled resin bed. This may be achieved by continuously draining solvent from the reactor. It is desirable that the resin bed does not run dry. Thus, new wash solvent is usually added - either continuously or discontinuously, in the latter case with a higher flow rate. The skilled artisan is aware of several strategies to adjust the solvent addition and solvent draining so that the settled resin bed does not run dry. For example, the height of the liquid above the settled resin bed may be monitored regularly or even continuously, e.g., manually by an operator, or, preferably, by a suitable detector or sensor or probe, e.g., a radar sensor, typically located inside the reaction vessel. The end point of such a displacement wash may be determined by online monitoring, e.g., using a suitable detector at the reactor outlet, wherein said reactor may monitor the concentration of one or more specific species in the drained solvent. Alternatively, such displacement washing may be stopped once a defined volume of solvent has been drained from the reactor. When carrying out the method of the present invention and wishing to reduce the overall solvent consumption, applying displacement washes is advantageous, regardless of which reactor type is employed (e.g., column reactor or batch reactor). As a fourth approach, the overall solvent consumption will obviously be reduced (or not increased), when not performing additional process steps such as the blocking step (f) or (f') of the method of the present invention, which are commonly also referred to as capping steps. WO 2017/223258 A1 teaches that a capping step, e.g., step (f) or (f') of the method of the present invention, may not be required in a coupling cycle comprising as step (e) or (e') a sulfurization step, in particular when using xanthane hydride (5-amino-3*H*-1,2,4-dithiazole-3-thione), phenylacetyl disulfide (PADS), 3*H-*1,2-benzodithiol-3-one 1,1-dioxide (Beaucage Reagent) or 3-(N,N-dimethylamino-methylidene)amino)-3*H*-1,2,4-dithiazole-5-thione (CAS RN: 1192027-04-5, DDTT) as the sulfurizing agent. This approach is compatible with the method of the present invention.

For example, batch reactors with a maximal inner volume of 1 to 1000 liters, of 5 to 650 liters, of 5 to 300 liters, of 10 to 600 liters, of 50 to 500 liters, or more than 1000 liters may be used for the method of the invention. As further examples, batch reactors with a minimal inner volume of 5, 10, 30, 40, 50, 60, 70, 75, 80, 90, 100, 150, 200, 250, 300, 450, 600, 650, 700, 750, or 800 liters may be used for the method of the invention.

In some embodiments of the method of the invention, at least step (b) is carried out in a batch reactor. In some embodiments of the method of the invention, at least steps (b) and (b') are carried out in a batch reactor. In some embodiments of the method of the invention, each iteration of the coupling cycle comprising steps (b) to (e) or steps (b) to (e') is carried out in a batch reactor. In some embodiments of the method of the invention, at least step (b) is carried out in a stirred-bed reactor. In some embodiments of the method of the invention, at least steps (b) and (b') are carried out in a stirred-bed reactor. In some embodiments of the method of the invention, each iteration of the coupling cycle comprising steps (b) to (e) or steps (b') to (e') is carried out in a stirred-bed reactor.

When using a batch reactor, step (b) may comprise adding a deprotection mixture M-b, which is a liquid composition C, into the reactor containing the component C-0 of step (a) followed by stirring or shaking or agitating by any means at a certain temperature for a certain time, followed by draining the deprotection mixture M-b, i.e. the liquid composition C, and any other liquids (and dissolved components) from the reactor through a filter or membrane or frit, thereby retaining the support-bound component C-0^{#} inside the reactor. When using a batch reactor, step (b') may comprise adding a deprotection mixture M-b', which may be a liquid composition C, into the reactor containing the (x-1)-th cycle oligonucleotide O-(x-1), followed by stirring or shaking or agitating by any means at a certain temperature for a certain time, followed by draining the deprotection mixture M-b', e.g. the liquid composition C, and any other liquids (and dissolved components) from the reactor through a filter or membrane or frit, thereby retaining the support-bound (x-1)-th cycle oligonucleotide O-(x-1)^{#} inside the reactor.

In some embodiments of the method of the invention, at least steps (b) and (b') are carried out in a batch reactor or at least steps (b) and (b') are carried out in a column reactor and the flow rate of the liquid composition C through the column reactor is below 300 cm/h.

As used herein, the terms "deprotection mixture M-b" and "deprotection mixture M-b'" refer to any mixture which may be used to effect cleavage of the PG-0 or PG-(x-1) protecting group, respectively. As known to the skilled artisan, such protecting groups comprising an optionally substituted triarylmethyl residue are typically removed by treatment with a protic acid or Lewis acid (i.e. an electron pair acceptor such as zinc bromide), preferably a protic acid. As used herein, the term "protic acid" may be understood in the broadest sense and may refer to any Brønsted acid, also referred to as Brønsted-Lowry acid. The terms "protic acid" and "Brønsted acid" (or "Brønsted-Lowry acid") are well-known to those skilled in the art. It is also well-known that the acid strength, simply speaking the tendency of a Brønsted-Lowry acid to donate a proton, may be expressed in terms of a pKa value, wherein a strong acid has a lower (i.e. smaller) pKa value than a weak acid. The term "pKa value" as such and the means of determining the pKa value of a protic acid form part of the common knowledge of the skilled artisan. Additionally, pKa values for commonly used Brønsted-Lowry acids (and many more) are available in tabulated form from the literature. As used herein, the pKa value is such determinable in water (i.e. aqueous solution) at 25 °C, unless indicated differently. Throughout this text, solutions used to cleave protecting groups comprising an optionally substituted triarylmethyl residue, in particular DMT protecting groups, are also referred to as detritylation cocktails.

It will be understood that the composition of the deprotection mixture M-b' may be the same or different for each iteration of step (b'), unless indicated differently in the context of specific embodiments. Furthermore, it will be understood that when stating that the deprotection mixture M-b or M-b' comprises "a protic acid", this may embrace a mixture of protic acids, unless indicated differently in the context of specific embodiments.

In some embodiments of the method of the invention, the deprotection mixture M-b and each deprotection mixture M-b' comprise a protic acid. In some embodiments of the method of the invention, the deprotection mixture M-b and each deprotection mixture M-b' comprises a protic acid having a pKa equal to or smaller than 4, equal to or smaller than 3.5, equal to or smaller than 3, equal to or smaller than 2.5, or equal to or smaller than 2. In some embodiments of the method of the invention, the deprotection mixture M-b and each deprotection mixture M-b' comprises a protic acid having a pKa in the range of 0-4, 0-3.5, 0-3, 0-2.5, or 0-2.

The protic acid of the deprotection mixture M-b or M-b' is not particularly limited in terms of its chemical structure. For example, the protic acid may be a carboxylic acid such as dichloroacetic acid (DCA), trichloroacetic acid (TCA), trifluoroacetic acid (TFA) or a sulfonic acid such as methanesulfonic acid or p-toluenesulfonic acid or a mineral acid such as sulfuric acid or hydrochloric acid or a mixture thereof. Carboxylic acids and sulfonic acids may be preferred, and carboxylic acids may be more preferred.

Carboxylic acids for use in the method of the present invention are known to those skilled in the art. Non-limiting examples of such carboxylic acids include monohalogenated acetic acids (e.g., 2-chloroacetic acid), dihalogenated acetic acids (e.g., 2,2-dichloroacetic acid), trihalogenated acetic acids (e.g., 2,2,2-trifluoroacetic acid, 2,2,2-trichloroacetic acid), 2-cyanoacetic acid, dicarboxylic acids (e.g., oxalic acid, malic acid, benzene-1,4-dicarboxylic acid), tricarboxylic acids (e.g., citric acid, benzene-1,3,5-tricarboxylic acid), tetracarboxylic acids (e.g., benzene-1,2,4,5-tetracarboxylic acid), and benzoic acid derivatives (e.g., 2-chlorobenzoic acid, 2,4-dichlorobenzoic acid, salicylic acid). The skilled artisan is also aware of sulfonic acids for use in the method of the present invention. Non-limiting examples of such sulfonic acids include alkyl sulfonic acids, e.g., of the formula (C₁-C₆-alkyl)-SO₃H (e.g., methanesulfonic acid, ethanesulfonic acid), in which one or more alkyl hydrogen residues may optionally be substituted (e.g., 3-hydroxypropane-1-sulfonic acid). Non-limiting examples of sulfonic acids for use in the method of the present invention further include arylsulfonic acids, wherein the aryl moiety preferably is a benzene moiety, and may optionally be substituted (e.g., benzenesulfonic acid, p-toluenesulfonic acid). Structurally more complex sulfonic acids such as, e.g., camphorsulfonic acid in any isomeric form may also be used. The skilled artisan is also aware of mineral acids for use in the method of the present invention. Non-limiting examples of such mineral acids include hydrogen halides (e.g., hydrogen chloride) and sulfuric acid. Explanations and examples pertaining to the aliphatic, aromatic or heteroaromatic amine, whose protonated form has a pKa in the range of 1-4, are provided in another section of this text. In particular, when using a mineral acid, a pre-formed salt of said mineral acid and an aliphatic, aromatic or heteroaromatic amine may be used in the method of the invention (e.g., 4-chloropyridinium hydrochloride).

According to the method of the invention, said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety. Furthermore, in at least one iteration of step (b'), said deprotection mixture M-b' is a liquid composition C comprising a solvent, a protic acid having a pKa equal to or smaller than 4, and at least one alcohol, in which one or more hydroxyl groups are covalently bonded directly to an optionally substituted phenyl moiety. It will be understood that the more than one liquid compositions C may be the same or different, i.e. comprise the same or different components in the same or different amounts or concentrations, as long as any such liquid composition C fulfils the general definition of a liquid composition C of the invention.

In some embodiments of the method of the invention, in step (b), the protecting group PG-0 is bonded to a hydroxyl moiety of a nucleoside moiety comprising a purine type nucleobase, preferably a nucleobase selected from the group consisting of adenine and guanine, in particular adenine. In some embodiments of the method of the invention, in at least one, preferably each, iteration of step (b'), in which the protecting group PG-(x-1) is bonded to a hydroxyl moiety of a nucleoside moiety comprising a purine type nucleobase, preferably a nucleobase selected from the group consisting of adenine and guanine, in particular adenine, said deprotection mixture M-b' is a liquid composition C comprising a solvent, a protic acid having a pKa equal to or smaller than 4, and at least one alcohol, in which one or more hydroxyl groups are covalently bonded directly to an optionally substituted phenyl moiety.

In some embodiments of the method of the invention:
- the backbone hydroxyl moiety protected by said protecting group PG-0 is part of a nucleoside moiety comprising a purine type nucleobase, preferably a nucleobase selected from the group consisting of adenine and guanine, in particular adenine; and
- in at least one iteration of the coupling cycle comprising steps (b') to (e'), in which said protecting group PG-(x-1) is part of a nucleoside moiety comprising a purine type nucleobase, preferably a nucleobase selected from the group consisting of adenine and guanine, in particular adenine, the deprotection mixture M-b' is a liquid composition C.

Step (b) and (b') of the method of the invention may typically be carried at a temperature between 15 °C and 25 °C, and may for convenience simply be carried out at out at ambient temperature. However, it is to be understood that any step (b) and (b') may be carried out at any suitable temperature, e.g. at a temperature of between -15 °C and 90 °C, for example at a temperature in the range of 0-90 °C, 10-70 °C, 10-60 °C, 10-50 °C, 10-40 °C, 15-30 °C, or 15-25 °C. The skilled artisan understands that increasing the reaction temperature will typically result in shorter reaction times. The means of increasing the reaction temperature are known to the skilled artisan and comprise, e.g., the use of a heating jacket (e.g. a double-walled reactor may be used) comprising a heating medium such as water, microwave irradiation, (in particular when using a flow reactor), infrared irradiation, and conductive heating.

The time for which steps (b) and (b') are carried is not particularly limited and may for example depend on the composition of the deprotection mixture M-b or M-b' and the reaction temperature. The skilled artisan will routinely determine a suitable reaction time, for example by means of monitoring the deprotection reaction of step (b) or (b') until full conversion (i.e. complete removal of the respective protecting group PG-0 or PG-(x-1)) is indicated. In particular, incubation with a liquid composition C of the invention is not particularly time sensitive and may be carried out for, e.g, at least 5 min, 10 min, 20 min, 30 min, or even 1 hour.

Each step (b) and (b') may consist of a single incubation with a deprotection mixture M-b or M-b' or may optionally comprise several repetitions of such incubation (i.e. several rounds of incubation), wherein for each round of incubation a fresh deprotection mixture M-b or or M-b', e.g. a liquid composition C, is typically used and drained prior to the next round of incubation (i.e. repetition). For example, an iteration of step (b) or (b') may comprise 1-5, 1-4, 1-3, or 2-3 rounds of incubation, each of which comprises incubation with a fresh deprotection mixture M-b or M-b'. In such cases, the deprotection mixtures M-b or M-b', e.g. the liquid compositions C, used for the rounds of incubation of the same iteration of step (b) or (b') preferably have (essentially) identical compositions. In particular, when stating that a step (b) or (b') is carried out using a certain and specified deprotection mixture M-b or M-b', e.g. a liquid composition C, said certain deprotection mixture M-b or M-b', e.g. said liquid composition C, has (essentially) the same composition for all rounds of incubation of the respective iteration of step (b) or (b'), unless indicated differently. This rationale may be exemplified by the following three examples:
As a first example, when stating that for step (b), the deprotection mixture M-b comprises a protic acid in a total molar amount in the range of 0.80-15.0 equivalents relative to the total molar amount of the protecting groups PG-0, this condition is fulfilled for each of the one or more deprotection mixtures M-b used for the one or more rounds of incubation of the respective step (b). This is to say that each of the one or more deprotection mixtures M-b used during the one or more rounds of incubation of said step (b) comprises a protic acid in a total molar amount in the range of 0.80-15.0 equivalents relative to the total molar amount of the protecting groups PG-0. As a second example, when stating that step (b) is carried out at a temperature in the range of 15-25 °C, this applies to all rounds of incubation which said step (b) may comprise. As a third example, when stating that step (b) is carried out for at least 20 min, this refers to the overall time for which step (b) is carried out, regardless of whether or not a single incubation (e.g. for 20 min) is performed or two rounds of incubation (e.g. for 10 min each) are performed.

Step (c) of the method of the invention is: providing a building block B-1 selected from the group consisting of a nucleoside and an oligonucleotide, wherein the building block B-1 comprises a backbone hydroxyl moiety protected by a protecting group PG-1 comprising an optionally substituted triarylmethyl residue, and a phosphorus moiety covalently bonded via its phosphorus atom to an oxygen atom of the backbone of the building block B-1.

Step (c') of the method of the invention is: providing a building block B-x selected from the group consisting of a nucleoside and an oligonucleotide, wherein the building block B-x comprises a backbone hydroxyl moiety protected by a protecting group PG-x comprising an optionally substituted triarylmethyl residue, and a phosphorus moiety covalently bonded via its phosphorus atom to an oxygen atom of the backbone of the building block B-x.

The terms "nucleoside", "oligonucleotide", "backbone hydroxyl moiety" have been explained. Any explanations and embodiments pertaining to a protecting group PG-(x-1) also apply to a protecting group PG-x. Thus, in some embodiments of the method of the invention, each protecting group PG-x is a DMT protecting group.

In some embodiments of the method of the invention, the building block B-1 and at least one, or each, building block B-x is selected from the group consisting of a nucleoside and an oligonucleotide comprising not more than 50, 40, 30, 25, 20, 15, 10, or 5 nucleoside subunits. In some embodiments of the method of the invention, the building block B-1 and at least one, or each, building block B-x is a nucleoside. In some embodiments of the method of the invention, the building block B-1 and at least one, or each, building block B-x is an oligonucleotide. In some embodiments of the method of the invention, the building block B-1 and at least one, or each, building block B-x is an oligonucleotide comprising not more than 50, 40, 30, 25, 20, 15, 10, or 5 nucleoside subunits.

In some preferred embodiments of the method of the invention, each building block B-1 and B-x comprises:
- exactly one backbone hydroxyl moiety protected by a protecting group PG-1 (for B-1) or PG-x (for B-x) comprising an optionally substituted triarylmethyl residue, and
- exactly one phosphorus moiety covalently bonded via its phosphorus atom to an oxygen atom of the backbone of the building block.

In some preferred embodiments of the method of the invention, each building block B-1 and B-x comprises:
- exactly one protecting group comprising an optionally substituted triarylmethyl residue, which is said protecting group PG-1 (for B-1) or PG-x (for B-x), and
- exactly one phosphorus moiety covalently bonded via its phosphorus atom to an oxygen atom of the backbone of the building block.

In some embodiments of the method of the invention, each of the building blocks B-1 and B-x is a compound of the following Formula II: 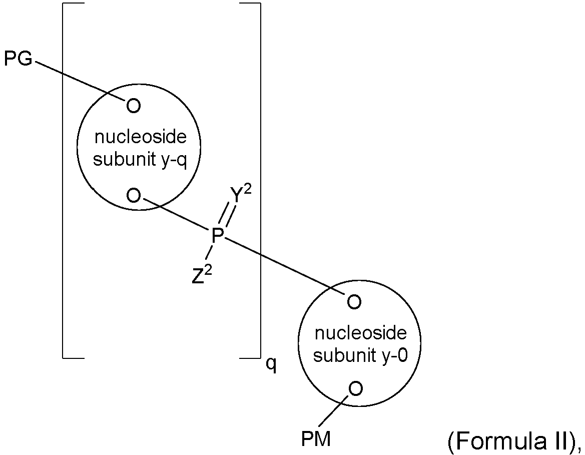 wherein in Formula II:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different (i.e. have the same or a different chemical structure);
PM is a phosphorus moiety;
PG is the protecting group PG-1 (for building block B-1) or PG-x (for a building block B-x) and comprises an optionally substituted triarylmethyl residue;
q is an integer equal to or larger than 0;
Y² is selected independently for each repetitive unit q from the group consisting of O and S;
Z² is selected independently for each repetitive unit q from the group consisting of O-R^{z-2}, S-R^{z-2}, and H; and
R^{z-2} is a protecting group, which may be the same or different for each repetitive unit q.

In some embodiments of the method of the invention, the building block B-1 or B-x of Formula II is a compound of the following Formula II-a: 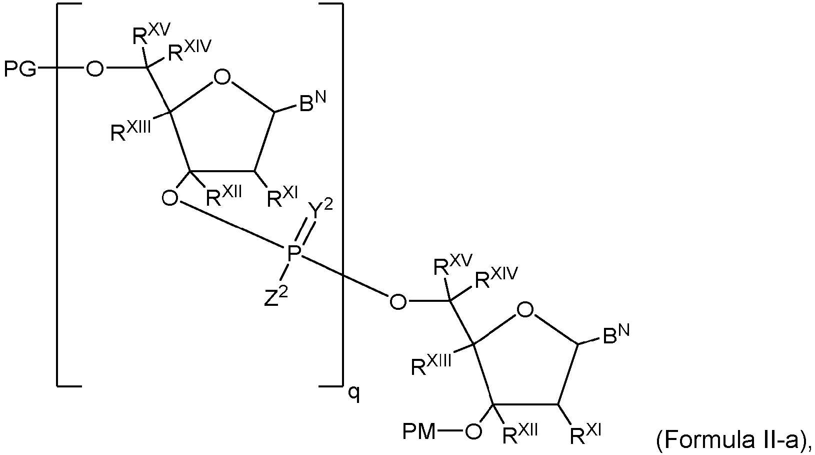 wherein in Formula II-a:
q, PG, Y², Z², R^{Z-2}, and PM are defined as for Formula II;
B^{N} is a nucleobase, which may be the same or different (i.e. have the same or a different chemical structure) at each occurrence;
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++,
where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the following Formula II-a-tc: 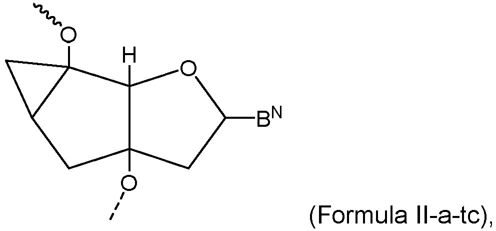
wherein in Formula II-a-tc:
   the oxygen atom from which the dashed line originates represents the oxygen atom bonded to the 3'-carbon atom, i.e. the carbon atom to which R^{XII} is bonded in Formula II-a;
   the dashed line indicates the covalent chemical bond interconnecting the respective oxygen atom of the nucleoside subunit of Formula II-a-tc and either PM or P(Y²)(Z²) in Formula II-a;
   the oxygen atom from which the wavy line originates represents the oxygen atom bonded to the 5'-carbon atom, i.e. the carbon atom to which C(R^{XIV})(R^{XV}) is bonded in Formula II-a; and
   the wavy line indicates the covalent chemical bond interconnecting the respective oxygen atom of the nucleoside subunit of Formula II-a-tc and either PG or P(Y²)(Z²) in Formula II-a.

In some embodiments of the method of the invention, the building block B-1 or B-x of Formula II, in particular of Formula II-a, is a compound of the following Formula II-b: 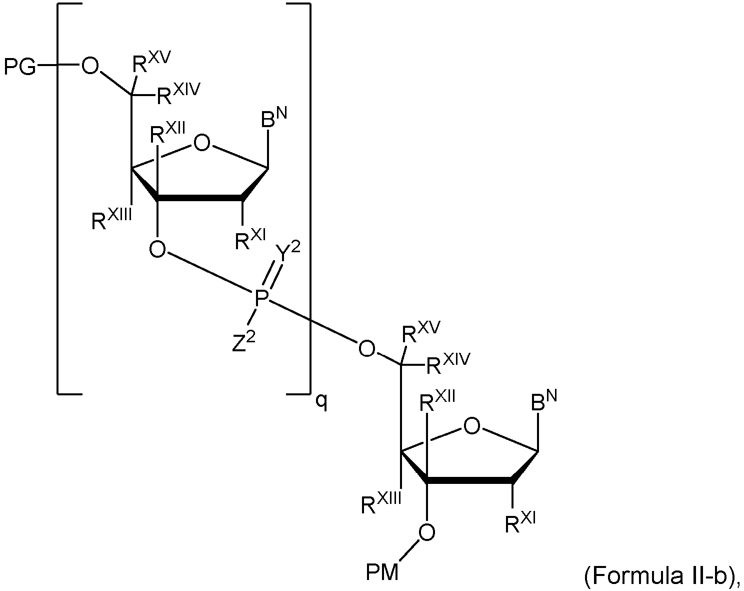 wherein in Formula II-b:
q, PG, Y², Z², R^{z-2}, PM, B^{N}, R^{XI}, and R^{XIII} are defined as for Formula II-a, and
for each nucleoside subunit independently, R^{XII}, R^{XIV}, and R^{XV} are either all H or they are bonded together so that the respective nucleoside subunit has a structure of the following Formula II-b-tc: 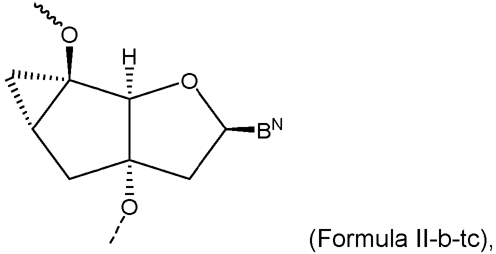
wherein in Formula II-b-tc:
   the oxygen atom from which the dashed line originates represents the oxygen atom bonded to the 3'-carbon atom, i.e. the carbon atom to which R^{XII} is bonded in Formula II-b;
   the dashed line indicates the covalent chemical bond interconnecting the respective oxygen atom of the nucleoside subunit of Formula II-b-tc and either PM or P(Y²)(Z²) in Formula II-b;
   the oxygen atom from which the wavy line originates represents the oxygen atom bonded to the 5'-carbon atom, i.e. the carbon atom to which C(R^{XIV})(R^{XV}) is bonded in Formula II-b; and
   the wavy line indicates the covalent chemical bond interconnecting the respective oxygen atom of the nucleoside subunit of Formula II-b-tc and either PG or P(Y²)(Z²) in Formula II-b.

As used herein, the term "phosphorus moiety" may be understood in the broadest sense and may refer to any atom group comprising at least one, preferably exactly one, phosphorus atom, wherein the term "atom group" may refer to two or more atoms. In such an atom group it is not required that each atom is covalently bonded to each further atom of said atom group, but that each atom is covalently bonded to at least one further atom of said atom group. From steps (d) and (d'), which will be laid out in more detail below, it is clear that said "phosphorus moiety covalently bonded via its phosphorus atom to an oxygen atom of the backbone of the building block" B-1 or B-x (e.g. the phosphorus moiety PM of any one of Formulae II, II-a, and II-b, is a phosphorus moiety capable of engaging in a condensation reaction with a free hydroxyl group. The skilled artisan is aware of phosphorus moieties which fulfill this condition and is able to select a suitable phosphorus moiety without undue experimentation. It is further known to those skilled in the art, that the phosphorus moiety which engages in such a condensation reaction will typically form an internucleosidic linkage group of the oligonucleotide formed via said condensation reaction. Said phosphorus moiety, e.g. the phosphorus moiety PM of any one of Formulae II, II-a, and II-b, may be a phosphorus (III) moiety, also referred to as P (III) moiety, i.e. a phosphorus moiety comprising a P (III) atom as defined herein. Non-limiting examples of such P (III) moieties are phosphoramidite moieties, as e.g. disclosed in X. Wei et al., Tetrahedron 2013, 69, 3615-3637, and H-phosphonate monoester moieties, as e.g. disclosed in J. Stawinski and R. Strömberg (2005), Di- and Oligonucleotide Synthesis Using H-Phosphonate Chemistry, in Methods in Molecular Biology, vol. 288: Oligonucleotide Synthesis: Methods and Applications, edited by P. Herdewijn, Humana Press Inc., Totowa NJ, https://doi.org/10.1385/1-59259-823-4:081. Alternatively, said phosphorus moiety, e.g. the phosphorus moiety PM of any one of Formulae II, II-a, and II-b, may be a phosphorus (V) moiety, also referred to as P (V) moiety, i.e. a phosphorus moiety comprising a P (V) atom as defined herein. Examples of such P (V) moieties comprise classical aryl phosphate diester moieties and the P (V) moieties disclosed by Baran et al. (Science 2018, 361, 1234-1238 and ACS Central Science 2021, 7, 1473-1485).

Preferred example of a building block B-1 or B-x, in which said phosphorus moiety, e.g. the phosphorus moiety PM of Formula II, is a phosphoramidite moiety, are compounds of Formula II-1.

In some embodiments of the method of the invention, each of the building blocks B-1 and B-x is a compound of the following Formula II-1: 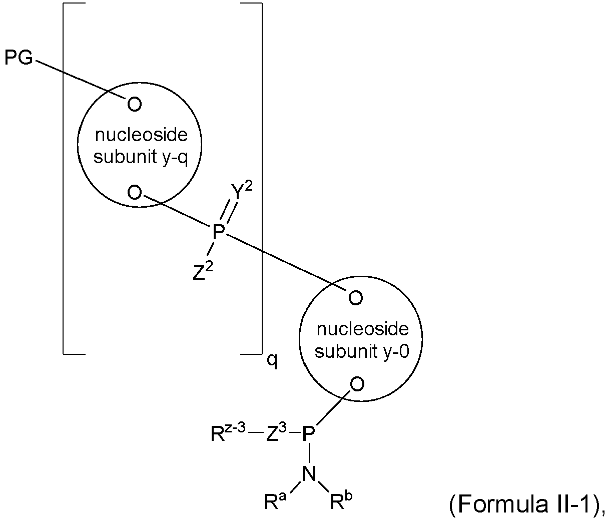 wherein in Formula II-1:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different (i.e. have the same or a different chemical structure);
PG is the protecting group PG-1 (for building block B-1) or PG (for a building block B-x) and comprises an optionally substituted triarylmethyl residue;
q is an integer equal to or larger than 0;
Y² is selected independently for each repetitive unit q from the group consisting of O and S;
Z² is selected independently for each repetitive unit q from the group consisting of O-R^{z-2} and S-R^{z-2};
R^{z-2} is a protecting group, which may be the same or different for each repetitive unit q;
Z³ is selected from the group consisting of O and S; and
R^{z-3} is a protecting group;
each of R^{a} and R^{b} is independently a C₁-C₆-alkyl group, wherein R^{a} and R^{b} may be the same or different and may also bond to each other to form a 5-membered or 6-membered aliphatic cyclic amine moiety together with the nitrogen atom to which R^{a} and R^{b} are bonded;
and wherein step (e) or step (e') is carried out in each coupling cycle.

In some embodiments of the method of the invention, the building block B-1 or B-x of Formula II-1 is a compound of the following Formula II-1-a: 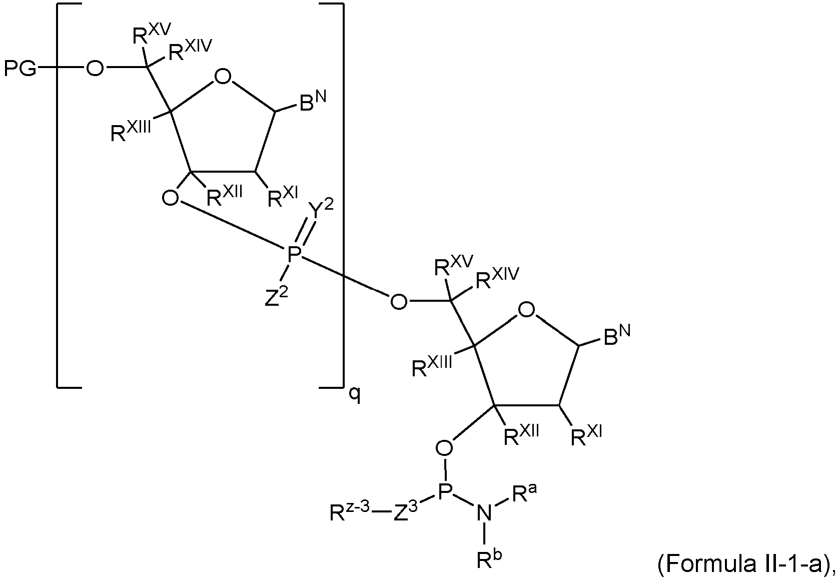 wherein in Formula II-1-a:
q, PG, Y², Z², R^{z-2}, Z³, R^{z-3}, R^{a}, and R^{b} are defined as for Formula II-1; and
B^{N}, R^{XI}, R^{XII}, R^{XIII}, R^{XIV}, and R^{XV} are defined as for Formula II-a, and
wherein step (e) or step (e') is carried out in each coupling cycle.

In some embodiments of the method of the invention, the building block B-1 or B-x of Formula II-1, in particular Formula II-1-a, is a compound of the following Formula II-1-b: 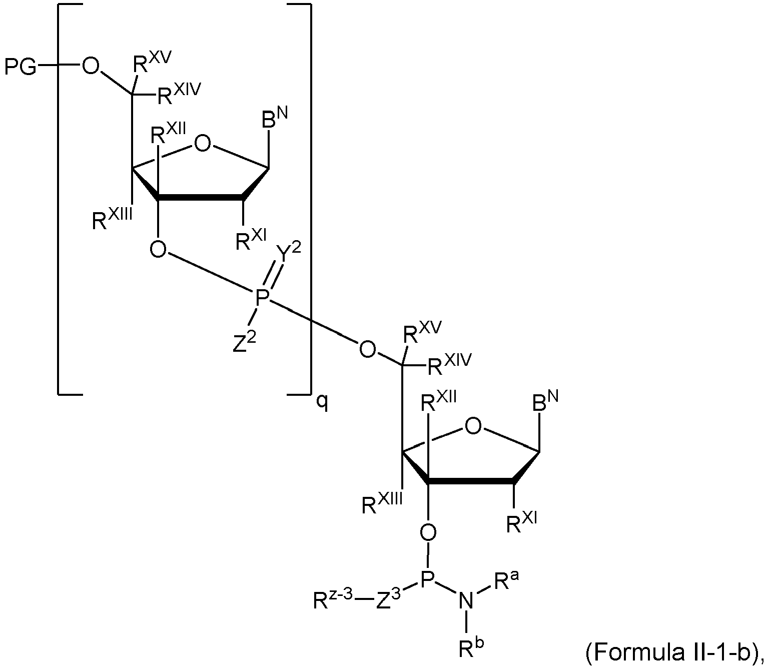 wherein in Formula II-1-b:
q, PG, Y², Z², R^{z-2}, Z³, R^{z-3}, R^{a}, and R^{b} are defined as for Formula II-1,
B^{N}, R^{XI}, R^{XII}, R^{XIII}, R^{XIV}, and R^{XV} are defined as for Formula II-b, and
wherein step (e) or step (e') is carried out in each coupling cycle.

Preferred example of a building block B-1 or B-x, in which said phosphorus moiety, e.g. the phosphorus moiety PM of any one of Formulae II, II-a, and II-b, is a H-phosphonate monoester moiety, are compounds of Formula II-2.

In some embodiments of the method of the invention, each of the building blocks B-1 and B-x is a compound of the following Formula II-2: 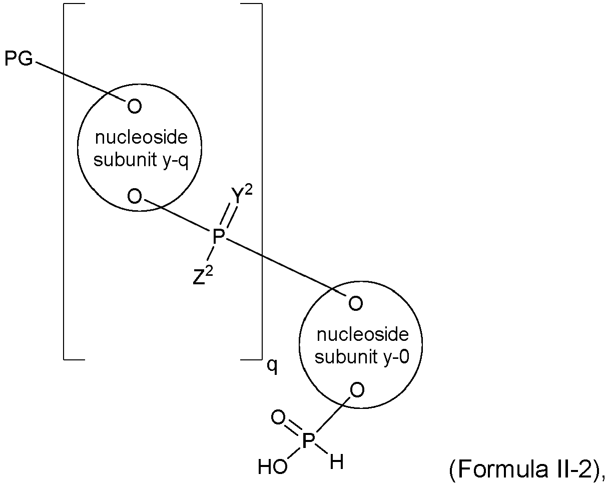 wherein in Formula II-2:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different (i.e. have the same or a different chemical structure);
PG is the protecting group PG-1 (for building block B-1) or PG (for a building block B-x) and comprises an optionally substituted triarylmethyl residue;
q is an integer equal to or larger than 0;
Y² is selected independently for each repetitive unit q from the group consisting of O and S;
Z² is selected independently for each repetitive unit q from the group consisting of H, O-R^{z-2}, and S-R^{z-2}; and
R^{z-2} is a protecting group, which may be the same or different for each repetitive unit q; and
wherein at least in the final coupling cycle, step (e) or step (e') is carried out.

In some embodiments of the method of the invention, the building block B-1 or B-x of Formula II-2 is a compound of the following Formula II-2-a: 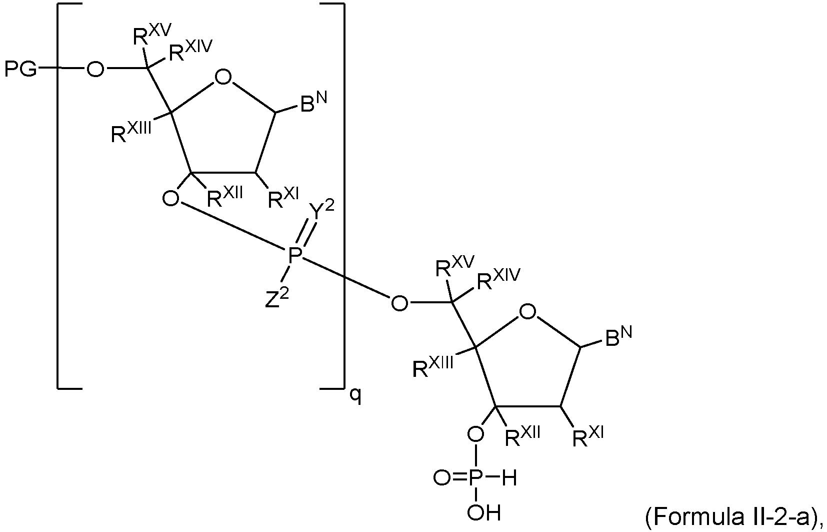 wherein in Formula II-2-a:
q, PG, Y², Z², and R^{z-2} are defined as for Formula II-2;
B^{N}, R^{XI}, R^{XII}, R^{XIII}, R^{XIV}, and R^{XV} are defined as for Formula II-a; and
wherein at least in the final coupling cycle, step (e) or step (e') is carried out.

In some embodiments of the method of the invention, the building block B-1 or B-x of Formula II-2, in particular Formula II-2-a, is a compound of the following Formula II-2-b: 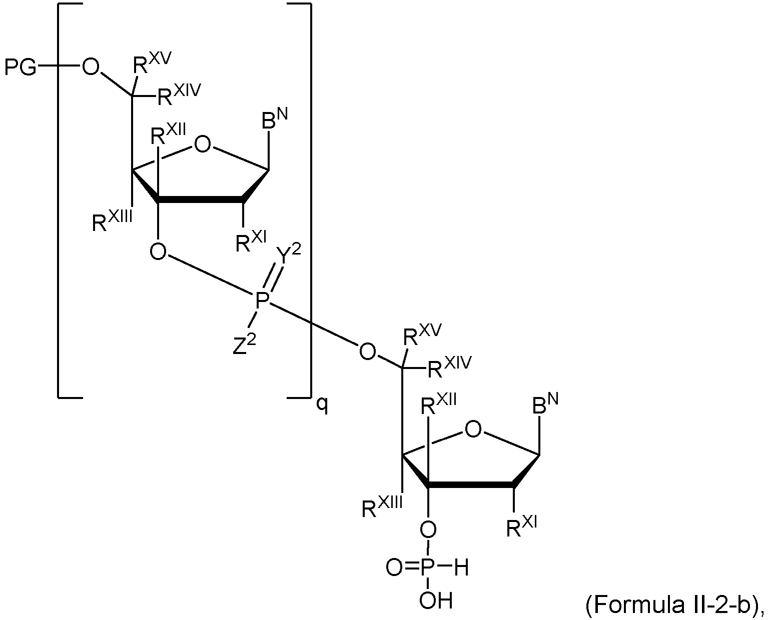 wherein in Formula II-2-b:
q, PG, Y², Z², and R^{z-2} are defined as for Formula II-2;
B^{N}, R^{XI}, R^{XII}, R^{XIII}, R^{XIV}, and R^{XV} are defined as for Formula II-b; and
wherein at least in the final coupling cycle, step (e) or step (e') is carried out.

In some embodiments, in the building block B-1 or B-x of any one of Formulae II, II-a, II-b, II-1, II-1-a, II-1-b, II-2, II-2-a, and II-2-b, the integer q is an integer in the range of 0-150, 0-100, 0-75, 0-50, 0-35, 0-30, 0-25, 0-20, 0-15, 0-10, 0-5, 0-3, 0-2, 0-1 or q is 0. It will be understood that to state than an integer is in the range of e.g. 0-5 means that said integer may be 0, 1, 2, 3, 4, or 5. In some embodiments, in the building block B-1 or B-x of any one of Formulae II, II-a, II-b, II-1, II-1-a, II-1-b, II-2, II-2-a, and II-2-b, the integer q is 0. It will be understood that, if the integer q in any one of Formulae II, II-a, II-b, II-1, II-1-a, II-1-b, II-2, II-2-a, and II-2-b is 0, the protecting group PG (i.e. PG-1 for B-1 and PG-x for B-x) is bonded to the respective hydroxyl moiety of the then remaining nucleoside subunit carrying the phosphorus moiety (e.g. nucleoside subunit y-0 in Formulae II, II-1 or II-2).

In some embodiments, in the building block B-1 or B-x, e.g. of any one of Formulae II, II-a, II-b, II-1, II-1-a, II-1-b, II-2, II-2-a, and II-2-b, the protecting group PG (i.e. PG-1 for B-1 and PG-x for B-x) is selected from the group consisting of the triphenylmethyl group (i.e. the trityl group), the (p-methoxyphenyl)diphenylmethyl group (i.e. the MMT group), and the di(p-methoxyphenyl)phenylmethyl group (i.e. the DMT group). In some preferred embodiments, in the building block B-1 or B-x, e.g. of any one of Formulae II, II-a, II-b, II-1, 11-1-a, II-1-b, II-2, II-2-a, and II-2-b, the protecting group PG (i.e. PG-1 for B-1 and PG-x for B-x) is the di(p-methoxyphenyl)phenylmethyl group (i.e. the DMT group).

In some embodiments, in the building block B-1 or B-x of any one of Formulae II, II-a, II-b, II-1, II-1-a, II-1-b, II-2, II-2-a, and II-2-b, Y² is for each repetitive unit q O. In some embodiments, in the building block B-1 or B-x of any one of Formulae II, II-a, II-b, II-1, II-1-a, II-1-b, II-2, II-2-a, and II-2-b, Y² is for each repetitive unit q S.

In some embodiments, in the building block B-1 or B-x of any one of Formulae II, II-a, II-b, II-1, II-1-a, II-1-b, II-2, II-2-a, and II-2-b, R^{z-2} is a protecting group removable under alkaline conditions, wherein R^{z-2} may be the same or different at each occurrence. In some embodiments, in the building block B-1 or B-x of any one of Formulae II, II-a, II-b, II-1, II-1-a, II-1-b, II-2, II-2-a, and II-2-b, R^{z-2} is for each repetitive unit q independently a protecting group of the chemical structure CH₂-CH₂-EWG, where EWG is an electron withdrawing group, preferably a cyano group. The electron withdrawing group may for example be selected from the group consisting of a cyano group, a halogen atom such as a chlorine, fluorine, or bromine atom, an aldehyde group, a keto group, a carboxyester group, or a carboxamide group. In some preferred embodiments, in the building block B-1 or B-x of any one of Formulae II, II-a, II-b, 11-1, 11-1-a, II-1-b, II-2, II-2-a, and II-2-b, R^{z-2} is for each repetitive unit q a 2-cyanoethyl group (i.e. CH₂-CH₂-CN).

In some embodiments, in the building block B-1 or B-x of any one of Formulae II, II-a, II-b, 11-1, II-1-a, and II-1-b, Z² is selected independently for each repetitive unit q from the group consisting of O-R^{z-2} and S-R^{z-2}. In some embodiments, in the the building block B-1 or B-x of any one of Formulae II, II-a, II-b, II-1, II-1-a, and II-1-b, Z² is selected independently for each repetitive unit q from the group consisting of O-R^{z-2} and S-R^{z-2}, where R^{z-2} is for each repetitive unit q a 2-cyanoethyl group (i.e. CH₂-CH₂-CN). In such embodiments, Z² is selected independently for each repetitive unit q from the group consisting of O-CH₂-CH₂-CN and S-CH₂-CH₂-CN. In some embodiments, in the building block B-1 or B-x of any one of Formulae II, II-a, II-b, II-1, II-1-a, and II-1-b, Z² is for each repetitive unit q O-R^{z-2}, where R^{z-2} is for each repetitive unit q a 2-cyanoethyl group (i.e. CH₂-CH₂-CN). In such embodiments, Z² is for each repetitive unit q O-CH₂-CH₂-CN.

In some embodiments, in the building block B-1 or B-x of any one of Formulae II, II-a, II-b, II-2, II-2-a, and II-2-b, Z² is for each repetitive unit q H. In some embodiments, in the building block B-1 or B-x of any one of Formulae II, II-a, II-b, II-2, II-2-a, and II-2-b, Y² is for each repetitive unit q O and Z² is for each repetitive unit q H.

In some embodiments, in the building block B-1 or B-x of any one of Formulae II, II-a, II-b, 11-1, II-1-a, II-1-b, II-2, II-2-a, and II-2-b, the terminal nucleoside subunit, whose hydroxyl moiety is bonded to the PG (i.e. PG-1 for B-1 and PG-x for B-x) protecting group, is a nucleoside subunit comprising a purine type nucleobase, preferably a nucleobase selected from the group consisting of adenine and guanine, in particular adenine.

The skilled person will understand that any kind of nucleobase B^{N} may be present in the building block B-1 or B-x of any one of Formulae II-a, II-b, II-1-a, II-1-b, II-2-a, and II-2-b. In some embodiments, in the building block B-1 or B-x of any one of Formulae II-a, II-b, II-1-a, II-1-b, II-2-a, and II-2-b, B^{N} is a nucleobase and at each occurrence independently selected from the group consisting of adenine, guanine, cytosine, 5-methylcytosine, thymine, and uracil. It will be understood by those skilled in the art, that any nucleobase B^{N} in any one of Formulae II-a, II-b, II-1-a, II-1-b, II-2-a, and II-2-b may optionally be protected, i.e. carry one or more protecting groups, without this being indicated specifically. Thus, when, for example, stating that B^{N} is adenine, guanine, 5-methylcytosine, cytosine, thymine, or uracil, this embraces the aforementioned nucleobases in protected form and in free form (i.e. with and without any protecting groups). The same rationale applies to nucleobases in general. Nucleobase protecting groups are known to those skilled in the art and have also been explained above, including, for example, the preferred nucleobase protecting groups compiled in Table T-1.

In some embodiments, each nucleobase of each building block B-1 or B-x, in particular each nucleobase B^{N} of each building block B-1 or B-x of any one of Formulae II, II-a, II-b, II-1, II-1-a, II-1-b, II-2, II-2-a, and II-2-b is independently selected from the group consisting of
- adenine, in which the exocyclic amino group is protected;
- guanine, in which the exocyclic amino group is protected;
- cytosine, in which the exocyclic amino group is protected;
- 5-methylcytosine, in which the exocyclic amino group is protected;
- thymine; and
- uracil.

In some embodiments, each nucleobase of each building block B-1 or B-x, in particular each nucleobase B^{N} of each building block B-1 or B-x of any one of Formulae II, II-a, II-b, II-1, II-1-a, II-1-b, II-2, II-2-a, and II-2-b is independently selected from the group consisting of
- adenine, in which the exocyclic amino group is protected by a benzoyl group, an isobutyryl group or a phenoxyacetyl group;
- guanine, in which the exocyclic amino group is protected by an isobutyryl group, a 4-isopropylphenoxyacetyl group or a dimethylformamidino group;
- cytosine, in which the exocyclic amino group is protected by an acetyl group or a benzoyl group;
- 5-methylcytosine, in which the exocyclic amino group is protected by an acetyl group or a benzoyl group;
- thymine; and
- uracil.

In some embodiments, in the building block B-1 or B-x of any one of Formulae II-a, II-b, II-1-a, II-1-b, II-2-a, and II-2-b:
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-x of any one of Formulae II-a, II-1-a, and II-2-a) or Formula II-b-tc (in a building block B-1 or B-x of any one of Formula II-b, II-1-b, and II-2-b).

In some embodiments, in the building block B-1 or B-x of any one of Formulae II-a, II-b, II-1-a, II-1-b, II-2-a, and II-2-b:
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XlV}, and R^{XV} are either all H or they are bonded together so that the respective nucleoside subunit has a structure of the aforementioned Formula II-a-tc (in a building block B-1 or B-x of any one of Formulae II-a, II-1-a, and II-2-a) or Formula II-b-tc (in a building block B-1 or B-x of any one of Formula II-b, II-1-b, and II-2-b).

In some embodiments, in the building block B-1 or B-x of any one of Formulae II-a, II-b, II-1-a, II-1-b, II-2-a, and II-2-b:
R^{XI} is selected independently at each occurrence from the group consisting of H, F, O-CH₃ (i.e. methoxy), and O-CH₂-CH₂-O-CH₃ (i.e. 2-methoxyethyl-1-oxy); and
each of R^{XII}, R^{XIII}, R^{XIV}, and R^{XV} is H at each occurrence.

In some embodiments, in the building block B-1 or B-x of any one of Formulae II-1, 11-1-a, and 11-1-b, Z³ is O. In some embodiments, in the building block B-1 or B-x of any one of Formulae II-1, II-1-a, and 11-1-b, Z³ is S.

In some embodiments, in the building block B-1 or B-x of any one of Formulae II-1, II-1-a, and II-1-b, R^{z-3} is a protecting group removable under alkaline conditions. In some embodiments, in the building block B-1 or B-x of any one of Formulae II-1, II-1-a, and II-1-b, R^{z-3} is a protecting group of the chemical structure CH₂-CH₂-EWG, where EWG is an electron withdrawing group, preferably a cyano group. The electron withdrawing group may for example be selected from the group consisting of a cyano group, a halogen atom such as a chlorine, fluorine, or bromine atom, an aldehyde group, a keto group, a carboxyester group, or a carboxamide group. In some preferred embodiments, in the building block B-1 or B-x of any one of Formulae II-1, II-1-a, and II-1-b, R^{z-3} is a 2-cyanoethyl group (i.e. CH₂-CH₂-CN). In some preferred embodiments, in the building block B-1 or B-x of any one of Formulae II-1, II-1-a, and II-1-b, Z³ is O and R^{z-3} is a 2-cyanoethyl group (i.e. CH₂-CH₂-CN).

In some embodiments, in the building block B-1 or B-x of any one of Formulae II-1, II-1-a, and II-1-b, each of R^{a} and R^{b} is independently a C₁-C₆-alkyl group, wherein R^{a} and R^{b} may be the same or different, and wherein R^{a} and R^{b} may not bond to each other. In some embodiments, in the building block B-1 or B-x of any one of Formulae II-1, II-1-a, and II-1-b, each of R^{a} and R^{b} is an isopropyl group (i.e. CH(CH₃)₂).

In some embodiments, in the building block B-1 or B-x of Formula II:
PM is a phosphorus moiety, preferably a phosphorus moiety selected from the group consisting of a phosphoramidite moiety and a H-phosphonate monoester moiety;
PG (i.e. PG-1 for B-1 and PG-x for B-x) is a di(p-methoxyphenyl)phenylmethyl (DMT) protecting group;
q is an integer in the range of 0-150, 0-100, 0-75, 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2}, S-R^{z-2}, and H; and
R^{z-2} is at each occurrence a 2-cyanoethyl group.

In some embodiments, in the building block B-1 or B-x of Formula II:
PM is a phosphoramidite moiety;
PG (i.e. PG-1 for B-1 and PG-x for B-x) is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} and S-R^{z-2}; and
R^{z-2} is at each occurrence a 2-cyanoethyl group.

In some embodiments, in the building block B-1 or B-x of Formula II:
PM is a phosphoramidite moiety;
PG (i.e. PG-1 for B-1 and PG-x for B-x) is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is for each repetitive unit q O-R^{z-2}; and
R^{z-2} is at each occurrence a 2-cyanoethyl group.

In some embodiments, in the building block B-1 or B-x of Formula II:
PM is a H-phosphonate monoester moiety;
PG (i.e. PG-1 for B-1 and PG-x for B-x) is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S, and preferably is O; and
Z² is for each repetitive unit q H.

In some embodiments, in the building block B-1 or B-x of any one of Formulae II-a and II-b:
PM is a phosphorus moiety, preferably a phosphorus moiety selected from the group consisting of a phosphoramidite moiety and a H-phosphonate monoester moiety;
PG (i.e. PG-1 for B-1 and PG-x for B-x) is a di(*p*-methoxyphenyl)phenylmethyl (DMT) protecting group;
q is an integer in the range of 0-150, 0-100, 0-75, 0-50, 0-35, 0-30, 0-25, 0-20, 0-15, 0-10, 0-5, 0-3, 0-2, 0-1 or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2}, S-R^{z-2}, and H;
R^{z-2} is at each occurrence a 2-cyanoethyl group;
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-x of Formula II-a) or Formula II-b-tc (in a building block B-1 or B-x of Formula II-b).

In some embodiments, in the building block B-1 or B-x of any one of Formulae II-a and II-b:
PM is a phosphorus moiety, preferably a phosphorus moiety selected from the group consisting of a phosphoramidite moiety and a H-phosphonate monoester moiety;
PG (i.e. PG-1 for B-1 and PG-x for B-x) is a di(p-methoxyphenyl)phenylmethyl (DMT) protecting group;
q is an integer in the range of 0-150, 0-100, 0-75, 0-50, 0-35, 0-30, 0-25, 0-20, 0-15, 0-10, 0-5, 0-3, 0-2, 0-1 or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2}, S-R^{z-2}, and H;
R^{z-2} is at each occurrence a 2-cyanoethyl group;
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-x of Formula II-a) or Formula II-b-tc (in a building block B-1 or B-x of Formula II-b).

In some embodiments, in the building block B-1 or B-x of any one of Formulae II-a and II-b:
PM is a phosphorus moiety, preferably a phosphorus moiety selected from the group consisting of a phosphoramidite moiety and a H-phosphonate monoester moiety;
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, 0-5, 0-3, 0-2, 0-1 or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2}, S-R^{z-2}, and H;
R^{z-2} is at each occurrence a 2-cyanoethyl group;
R^{XI} is selected independently at each occurrence from the group consisting of H, F, O-CH₃ (i.e. methoxy), and O-CH₂-CH₂-O-CH₃ (i.e. 2-methoxyethyl-1-oxy); and each of R^{XII}, R^{XIII}, R^{XIV}, and R^{XV} is H at each occurrence.

In some embodiments, in the building block B-1 or B-x of Formula II-1:
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S, and preferably is O;
R^{z-3} is a 2-cyanoethyl group; and
each of R^{a} and R^{b} is independently a C₁-C₆-alkyl group, preferably an isopropyl group.

In some embodiments, in the building block B-1 or B-x of Formula II-1:
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is for each repetitive unit q O-R^{z-2};
R^{z-2} is at each occurrence a 2-cyanoethyl group;
Z³ is O;
R^{z-3} is a 2-cyanoethyl group; and
each of R^{a} and R^{b} is an isopropyl group.

In some embodiments, in the building block B-1 or B-x of any one of Formula II-1-a and II-1-b:
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S, and preferably is O;
R^{z-3} is a 2-cyanoethyl group;
each of R^{a} and R^{b} is independently a C₁-C₆-alkyl group, preferably an isopropyl group;
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-x of Formula II-1-a) or Formula II-b-tc (in a building block B-1 or B-x of Formula II-1-b).

In some embodiments, in the building block B-1 or B-x of any one of Formula II-1-a and II-1-b:
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S and preferably is O;
R^{z-3} is a 2-cyanoethyl group;
each of R^{a} and R^{b} is independently a C₁-C₆-alkyl group, preferably an isopropyl group;
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-x of Formula II-1-a) or Formula II-b-tc (in a building block B-1 or B-x of Formula II-1-b).

In some embodiments, in the building block B-1 or B-x of any one of Formula II-1-a and II-1-b:
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S and preferably is O;
R^{z-3} is a 2-cyanoethyl group;
each of R^{a} and R^{b} is independently a C₁-C₆-alkyl group, preferably an isopropyl group;
R^{XI} is selected independently at each occurrence from the group consisting of H, F, O-CH₃ (i.e. methoxy), and O-CH₂-CH₂-O-CH₃ (i.e. 2-methoxyethyl-1-oxy); and each of R^{XII}, R^{XIII}, R^{XIV}, and R^{XV} is H at each occurrence.

In some embodiments, in the building block B-1 or B-x of Formula II-2:
PG (i.e. PG-1 for B-1 and PG-x for B-x) is a di(p-methoxyphenyl)phenylmethyl (DMT) protecting group;
q is an integer in the range of 0-50, 0-35, 0-30, 0-25, 0-20, 0-15, 0-10, or 0-5, or q is H;
Y² is selected independently for each repetitive unit q from the group consisting of O and S, and preferably is O; and
Z² is for each repetitive unit q H.

In some embodiments, in the building block B-1 or B-x of any one of Formulae II-2-a and II-2-b:
PG (i.e. PG-1 for B-1 and PG-x for B-x) is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
B^{N} is a nucleobase, which may be the same or different at each occurrence;
Y² is selected independently for each repetitive unit q from the group consisting of O and S, and preferably is O;
Z² is for each repetitive unit q H;
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-x of Formula II-2-a) or Formula II-b-tc (in a building block B-1 or B-x of Formula II-2-b).

In the method of the invention, the building block B-1 or B-x may be the same or different (i.e. have the same or a different chemical structure) for each iteration of the coupling cycle, unless indicated differently in the context of specific embodiments.

The term "providing a building block" B-1 or B-x in step (c) or (c') may be understood in the broadest sense. A building block B-1 or B-x for use in the method of the invention may, for example, be obtained commercially, in particular, if said phosphorus moiety is a phosphoramidite moiety or a H-phosphonate monoester moiety. Alternatively, a building block B-1 or B-x for use in the method of the invention may be obtained by means of chemical synthesis. The person skilled in the art is aware of methods of synthesizing such compounds, wherein the synthesis route will obviously depend on the chemical structure of said phosphorus moiety. Building blocks B-1 or B-x, e.g. of any one of Formulae II, II-a, and II-b, in which said phosphorus moiety, e.g. said phosphorus moiety PM, is a phosphoramidite moiety, in particular building block B-1 or B-x of any one of Formulae II-1, 11-1-a, and 11-1-b, may, for example, be synthesized as disclosed in, in K.V. Gothelf et al., Nature Communications 2021 and in X. Wei et al., Tetrahedron 2013, 69, 3615-3637. Building blocks B-1 or B-x, e.g. of any one of Formulae II, II-a, and II-b, in which said phosphorus moiety, e.g. said phosphorus moiety PM, is a H-phosphonate monoester moiety, in particular building block B-1 or B-x of any one of Formulae II-2, II-2-a, and II-2-b, may, for example, be synthesized as disclosed in J. Stawinski and R. Strömberg (2005), Di- and Oligonucleotide Synthesis Using H-Phosphonate Chemistry, in Methods in Molecular Biology, vol. 288: Oligonucleotide Synthesis: Methods and Applications, edited by P. Herdewijn, Humana Press Inc., Totowa NJ, https://doi.org/10.1385/1-59259-823-4:081. Building blocks B-1 or B-x, e.g. of any one of Formulae II, II-a and II-b, in which said phosphorus moiety, e.g. said phosphorus moiety PM, is a P (V) moiety suitable for chiral phosphorothioate synthesis may, for example, be synthesized as disclosed in P.S. Baran et al., Science 2018, 361, 1234-1238 (also see Supplementary Materials for this reference).

Step (d) of the method of the invention is: reacting the component C-0^{#} of step (b) with the building block B-1 of step (c) under conditions suitable to form a covalent bond between said free backbone hydroxyl group of the component C-0^{#} and the phosphorus atom of said phosphorus moiety of the building block B-1, thereby obtaining a first cycle oligonucleotide O-1.

Step (d') of the method of the invention is: reacting the (x-1)-th cycle oligonucleotide (O-(x-1))^{#} obtained in step (b') with the building block B-x of step (c') under conditions suitable to form a covalent bond between said free backbone hydroxyl group of the (x-1)-th cycle oligonucleotide (O-(x-1))^{#} and the phosphorus atom of said phosphorus moiety of the building block B-x, thereby obtaining a x-th cycle oligonucleotide O-x.

For example, in the fifth coupling cycle (x = 5), step (d') is: reacting the (5-1)-th cycle (i.e. fourth cycle) oligonucleotide (O-(5-1))^{#} (i.e. (O-4)^{#}) obtained in step (b') with the building block B-5 of step (c') under conditions suitable to form a covalent bond between said free backbone hydroxyl group of the fourth cycle oligonucleotide (O-(4))^{#} and the phosphorus atom of said phosphorus moiety of the building block B-x, thereby obtaining a 5-th cycle oligonucleotide O-5.

The component C-0^{#} as well as the (x-1)-th cycle oligonucleotide (O-(x-1))^{#} have been defined. The term "backbone hydroxyl group" refers to a hydroxyl group which is part of the backbone of the respective nucleoside or oligonucleotide and will be understood based on the above explanations of the terms "hydroxyl group" and "backbone". The building blocks B-1 and B-x have been defined.

The term "reacting" in step (d) may be understood in the broadest sense as any operation during which the component C-0^{#} and the building block B-1 are present in the same reaction vessel or reactor and engage in the bond forming reaction of step (d). The term "reacting" in step (d') may be understood in the broadest sense as any operation during which the (x-1)-th cycle oligonucleotide (O-(x-1))^{#} and the building block B-x are present in the same reaction vessel or reactor and engage in the bond forming reaction of step (d'). Typically, the component C-0^{#} or the (x-1)-th cycle oligonucleotide (O-(x-1))^{#} may already be contained in a reaction vessel or reactor, to which the building block B-1 or B-x is then added. Alternatively, the building block B-1 or B-x or a solution thereof may already be contained in a reaction vessel or reactor, to which the component C-0^{#} or the (x-1)-th cycle oligonucleotide (O-(x-1))^{#} is then added.

During step (d), a covalent (chemical) bond is formed between said free backbone hydroxyl group of the component C-0^{#} and the phosphorus atom of said phosphorus moiety of the building block B-1. During step (d'), a covalent (chemical) bond is formed between said free backbone hydroxyl group, of the (x-1)-th cycle oligonucleotide (O-(x-1))^{#} and the phosphorus atom of said phosphorus moiety of the building block B-x. The bond forming reaction of step (d) and (d') is herein also referred to as coupling or coupling reaction or condensation or condensation reaction, and steps (d) and (d') are also referred to as coupling steps or condensation steps.

The product obtained from the bond forming reaction of step (d) is the first cycle oligonucleotide O-1, which comprises the nucleoside sequence of the component C-0^{#} and of the building block B-1, wherein these two are now interconnected by an internucleosidic linkage group derived from the phosphorus moiety of the building block B-1. For example, the product obtained from the bond forming reaction of step (d') of the fifth coupling cycle (x = 5) is the fifth cycle oligonucleotide O-5 which comprises the nucleoside sequence of the fourth cycle oligonucleotide (O-4)^{#} and of the building block B-5, wherein these two are now interconnected by an internucleosidic linkage group derived from the phosphorus moiety of the building block B-5.

The "conditions suitable" for the bond forming reactions of steps (d) and (d') form part of the common knowledge of those skilled in the art. These conditions may, for example, depend on the chemical structure of said phosphorus moiety which is to engage in the bond forming reaction. Any (reaction) conditions suitable to achieve the desired bond forming reaction may be used in the method of the invention.

A bond forming reaction of step (d) or (d'), in which the phosphorus moiety engaging in said reaction is a phosphoramidite moiety, as e.g. present in the building block B-1 or B-x of any one of Formulae II-1, II-1-a, and II-1-b, is herein also referred to as phosphoramidite coupling (reaction). Such phosphoramidite couplings may preferably be performed in the presence of an activator. Any activator used in oligonucleotide synthesis by the so-called phosphoramidite method may be used in the method of the invention. The activator may, for example, be selected from the group consisting of:
- a tetrazole type activator such as 1*H*-tetrazole, 5-ethylthio-1*H*-tetrazole (ETT), 5-benzylthio-1*H*-tetrazole (BTT), 5-methylthio-1*H*-tetrazole (MTT), 1-methyl-5-mercaptotetrazole, 1-phenyl-5-mercaptotetrazole, and 5-(4-nitrophenyl)-1*H*-tetrazole,
- an imidazole type activator such as 4,5-dicyanoimidazole (DCI) and 2-bromo-4,5-dicyanoimidacole (2-Br-DCI),
- a 1-hydroxybenzotriazole type activator such as 1-hydroxybenzotriazole, 1-hydroxy-6-trifluorobenzotriazole, and 1-6-trifluoro-4-nitrobenzotriazole,
- a pyridinium salt type activator such as pyridinium hydrochloride, pyridinium *p*-toluenesulfonate, and pyridinium trifluoroacetate, and
- a saccharin type activator, i.e. salts obtained from reacting saccharin with an organic base such as pyridine, collidine, lutidine, picoline, *N*-methylimidazole, and triethylamine.

The activators disclosed in X. Wei et al., Tetrahedron 2013, 69, 3615-3637 may, for example, be used in phosphoramidite couplings. 1*H*-tetrazole, 5-ethylthio-1*H-*tetrazole (ETT), 5-benzylthio-1*H*-tetrazole (BTT), and 4,5-dicyanoimidacole (DCI) may be preferred. For example, X. Wei et al., Tetrahedron 2013, 69, 3615-3637 also discloses a range of suitable reaction conditions for these activators. N-methylimidazole (NMI) may be added alongside the activator, which may help to adjust the acidity of the solution. A phosphoramidite coupling of step (d) may preferably be performed in a solvent selected from the group consisting of acetonitrile, N,N-dimethylformamide (DMF), N,N-dimethylacetamide (DMA), N-methyl-2-pyrrolidone (NMP), N-methyl-4-piperidone, and mixtures thereof, optionally in combination with a non-polar solvent such as toluene, xylene or mesitylene. Acetonitrile, DMF, and a mixture thereof may be particularly preferred. The solvent (mixture) used for a phosphoramidite coupling is preferably substantially anhydrous, since water might react with phosphoramidites. The solvent for a phosphoramidite coupling may preferably comprise equal to or less than 1000 ppm or 750 ppm or 500 ppm or 250 ppm or 100 ppm or even 50 ppm of water, as determined by means of standard Karl Fischer titration at approximately 20 °C. Phosphoramidite couplings may, for example, be performed at a temperature in the range of 0-90 °C, 10-70 °C, 10-60 °C, 10-50 °C, 10-40 °C, 15-30 °C or 15-25 °C. For convenience, phosphoramidite couplings may simply be performed at room temperature. Increased temperatures may result in shorter reaction times. The reaction time may also depend on the chemical structure of the reactants and will routinely be selected by a skilled person, for example based on reaction monitoring using, e.g., thin-layer chromatography and/or high performance liquid chromatography (HPLC), optionally coupled to mass spectrometry.

A bond forming reaction of step (d) or (d'), in which the phosphorus moiety engaging in said reaction is a H-phosphonate monoester moiety, as e.g. present in the building block B-1 or B-x of any one of Formulae II-2, II-2-a, and II-2-b, is herein also referred to as H-phosphonate coupling (reaction). Such H-phosphonate couplings may typically be performed using a condensing agent. Any condensing agent used in oligonucleotide synthesis by the so-called H-phosphonate method may be used in the method of the invention. The condensing agent may, for example, be selected from the group consisting of pivaloyl chloride (PvCl), 1-adamantanecarbonyl chloride (AdCl), 2,2-dimethylbutyryl chloride, isobutyryl chloride, diphenyl chlorophosphate, 2,4,6-triisopropylbenzenesulfonyl chloride, bis(pentafluorophenyl) carbonate, 2-chloro-5,5-dimethyl-1,3,2-dioxaphosphorinane 2-oxide (also referred to as 5,5-dimethyl-2-oxo-2-chloro-1,3,2-dioxaphosphinane, DMOCP), and bis(2-oxo-3-oxazolidinyl)phosphinic chloride (OXP or BOP-CI). The building block B-1 or B-x comprising said H-phosphonate monoester moiety may be pre-activated with (i.e. incubated with) said condensing agent, and then be combined and reacted with the component C-0^{#} or the (x-1)-th cycle oligonucleotide (O-(x-1))^{#} to effect the condensation. The skilled person is familiar with suitable reaction conditions for such H-phosphonate couplings. For example, J. Stawinski and R. Strömberg (2005), Di- and Oligonucleotide Synthesis Using H-Phosphonate Chemistry, in Methods in Molecular Biology, vol. 288: Oligonucleotide Synthesis: Methods and Applications, edited by P. Herdewijn, Humana Press Inc., Totowa NJ, https://doi.org/10.1385/1-59259-823-4:081 discloses such reaction conditions. A H-phosphonate coupling of step (d) or (d') may preferably be performed in a solvent selected from the group consisting of acetonitrile, pyridine, and a mixture thereof. The solvent (mixture) used for a H-phosphonate coupling is preferably substantially anhydrous. The solvent for a phosphoramidite coupling may preferably comprise equal to or less than 3000 pm, 2000 ppm, 1500 ppm, 1000 ppm or 750 ppm or 500 ppm or 250 ppm or 100 ppm or even 50 ppm of water, as determined by means of standard Karl Fischer titration. H-phosphonate couplings may, for example, be performed at a temperature in the range of 0-90 °C, 10-70 °C, 10-60 °C, 10-50 °C, 10-40 °C, 15-30 °C, 15-25 °C. For convenience, H-phosphonate couplings may simply be performed at room temperature. Increased temperatures may result in shorter reaction times. The reaction time may also depend on the chemical structure of the reactants and will routinely be selected by a skilled person, for example based on reaction monitoring using, e.g., thin-layer chromatography and/or high performance liquid chromatography (HPLC), optionally coupled to mass spectrometry.

A bond forming reaction of step (d) or (d'), in which the phosphorus moiety engaging in said reaction is an arylphosphate diester moiety, is herein also referred to as phosphotriester coupling (reaction). Briefly, the arylphosphate diester moieties may typically be activated with an arylsulfonyl chloride activator, such as mesitylene-2-sulfonyl chloride (MsCl), usually in the presence of an auxiliary nucleophile such as 1-methylimidazole. Alternatively, pre-formed or in-situ generated 1-hydroxybenzotriazole-phosphotriesters may, for example, be used as phosphorus moiety instead of an aryl phosphate diester moiety in a phosphotriester coupling, again in the presence of an auxiliary nucleophile such as 1-methylimidazole.

A bond forming reaction of step (d) or (d'), in which the phosphorus moiety engaging in said reaction is a P (V) moiety allowing for chiral phosphorothioate synthesis may, for example, be performed as disclosed in Baran et al. (Science 2018, 361, 1234-1238 (see also the Supplementary Materials of said publication) and ACS Central Science 2021, 7, 1473-1485), or in a similar fashion.

Step (e) of the method of the invention is: optionally, incubating the first cycle oligonucleotide O-1 obtained in step (d) with an oxidizing or sulfurizing agent, thereby converting any P (III) atoms within said first cycle oligonucleotide O-1 to P (V) atoms.

Step (e') of the method of the invention is: optionally, incubating the x-th cycle oligonucleotide O-x obtained in step (d') with an oxidizing or sulfurizing agent, thereby converting any P (III) atoms within said x-th cycle oligonucleotide O-x to P (V) atoms.

It will be understood that converting any P (III) atoms to P (V) atoms does not change the name by which said first cycle oligonucleotide O-1 or said x-th cycle oligonucleotide O-x is referred to herein, since the terms first cycle oligonucleotide O-1 and x-th cycle oligonucleotide O-x embrace the respective oligonucleotide with any type of backbone structure, in particular with any type of internucleosidic linkage groups. Only the absence of the protecting group PG-0 or PG-(x-1) is indicated specifically by the aforementioned number sign (i.e. hashtag sign) "^{#}" put in superscript.

The terms "P (III) atom" and "P (V) atom" have been defined above. As used throughout this text, the term "oxidation state" "equals the charge of an atom after its homonuclear bonds have been divided equally and heteronuclear bonds assigned to the bond partners according to Allen electronegativity, except when the electronegative atom is bonded reversibly as a Lewis-acid ligand, in which case it does not obtain that bond's electrons", as described in the IUPAC Recommendations 2016 (P. Karen et al., Pure and Applied Chemistry 2016, 88(8), 831-839). The Allen electronegativities given in said reference are to be used and the P-H bond electron pair is assigned to H. As an example, the phosphorus atom of the H-phosphonate monoester moiety in any one of Formulae II-2, II-2-a, and II-2-b has the oxidation state III. As another example, the phosphorus atom of the phosphoramidite moiety in any one of Formulae II-1, II-1-a, and II-1-b has the oxidation state III. As a third example, the phosphorus atoms in the phosphodiester linkage groups of DNA and RNA have the oxidation state V.

The term "optionally" in steps (e) and (e') denotes that the respective step may or may not be carried out in a given iteration of the coupling cycle, unless indicated differently in the context of specific embodiments. As known to those skilled in the art, oligonucleotides comprising one or more P (III) atoms, in particular one or more P (III) linkage groups, are typically less stable than related oligonucleotides comprising only P (V) atoms, in particular only P (V) linkage groups (as e.g. present in DNA and RNA). In some embodiments of the method of the invention, the target oligonucleotide O^{T} comprises only P (V) atoms, in particular only P (V) linkage groups. If the first cycle oligonucleotide O-1 obtained in step (d) does not comprise any P (III) atoms, it may not be necessary to carry out step (e). If the x-th cycle oligonucleotide O-x obtained in step (d') does not comprise any P (III) atoms, it may not be necessary to carry out step (e') in the same iteration of the coupling cycle.

It is understood by those skilled in the art, that the oxidation state of the phosphorus atom within an internucleosidic linkage group formed in the bond forming reaction of a step (d) or a step (d') will typically depend on the chemical structure of said phosphorus moiety of said building block B-1 or B-x, which engaged in the respective bond forming reaction. Typically, the oxidation state of the phosphorus atom within such a phosphorus moiety will be preserved in the course of the bond forming reaction of step (d) or (d'). For example, if a phosphotriester coupling as defined herein is carried out in step (d) or (d') of a coupling cycle, the phosphorus atom of the resulting phosphotriester internucleosidic linkage group will typically be present as P (V) atom, i.e. the phosphotriester linkage group is a P (V) linkage group. The same principle applies to the P (V) chemistry utilized by Baran et al. (Science 2018, 361, 1234-1238 and ACS Central Science 2021, 7, 1473-1485).

On the other hand, if the phosphorus moiety of a building block B-1 or B-x engaging in a bond forming reaction of a step (d) or (d') comprises a P (III) atom, the resulting internucleosidic linkage group will typically also comprise a P (III) atom, i.e. be a P (III) linkage group. Depending on the stability of the so-obtained P (III) linkage group, step (e) or (e') may need to be carried out in the same coupling cycle x or may optionally be carried out in a later iteration of the coupling cycle, e.g. in the final, i.e. n-th coupling cycle. For example, the bond forming reaction of a step (d) or (d') may typically give a phosphite triester product (a phosphite triester internucleosidic linkage group, i.e. a P (III) linkage group), if said phosphorus moiety covalently bonded via its phosphorus atom to an oxygen atom of the backbone of the building block B-1 or B-x, e.g. the phosphorus moiety PM in any one of Formulae II, II-a, and II-b, is a phosphoramidite moiety such as, e.g., present in any one of Formulae II-1, 11-1-a, and 11-1-b, while a H-phosphonate diester product (a H-phosphonate diester internucleosidic linkage group, i.e. a P (III) linkage group) may typically be obtained, if said phosphorus moiety covalently bonded via its phosphorus atom to an oxygen atom of the backbone of the building block B-1 or B-x, e.g. the phosphorus moiety PM in any one of Formulae II, II-a, and II-b, is a H-phosphonate monoester moiety such as, e.g., present in any one of Formulae II-2, II-2-a, and II-2-b. H-phosphonate diester linkage groups may be more stable, e.g. under the conditions of steps (b) and (b'), than phosphite triester linkage groups, so that step (e) or (e') may not need to be performed in each iteration of the coupling cycle, in which said phosphorus moiety covalently bonded via its phosphorus atom to an oxygen atom of the backbone of the building block B-1 or B-x, e.g. the phosphorus moiety PM in any one of Formulae II, II-a, and II-b, is a H-phosphonate monoester moiety such as, e.g., present in any one of Formulae II-2, II-2-a, and 11-2-b. However, at least in the final coupling cycle, step (e) (in case the first coupling cycle is the final coupling cycle) or (e') is carried out, so that any P (III) atoms are converted to P (V) atoms.

In some embodiments of the method of the invention:
- the phosphorus moiety of the building block B-1 and each building block B-x is independently selected from the group consisting of a phosphoramidite moiety and a H-phosphonate monoester moiety;
- in each coupling cycle, in which said phosphorus moiety of the building block B-1 or the building block B-x is a phosphoramidite moiety, step (e) or (e') is carried out; and
- at least in the final coupling cycle, step (e) or (e') is carried out.

In some embodiments of the method of the invention:
- the phosphorus moiety of the building block B-1 and each building block B-x, e.g. the phosphorus moiety PM of any one of Formulae II, II-a, and II-b, is a phosphoramidite moiety; and
- in each coupling cycle, step (e) or (e') is carried out.

In some embodiments of the method of the invention:
- the phosphorus moiety of the building block B-1 and each building block B-x, e.g. the phosphorus moiety PM of any one of Formulae II, II-a, and II-b, is a H-phosphonate monoester moiety; and
- at least in the final coupling cycle, step (e) or (e') is carried out.

In some embodiments of the method of the invention:
- in each coupling cycle, the building block B-1 or B-x is independently selected from the group consisting of a compound of Formula II-1 and Formula II-2, preferably from the group consisting of Formula II-1-a and Formula II-2-a, in particular from the group consisting of Formula II-1-b and Formula II-2-b;
- in each coupling cycle, in which the building block B-1 or B-x is a compound of any one of Formulae II-1, II-1-a, and II-1-b, step (e) or (e') is carried out; and
- at least in the final coupling cycle, step (e) or (e') is carried out.

In some embodiments of the method of the invention:
- in each coupling cycle, the building block B-1 or B-x is a compound of Formula II-1, preferably of Formula II-1-a, in particular of Formula 11-1-b; and
- in each coupling cycle, step (e) or (e') is carried out.

In some embodiments of the method of the invention:
- in each coupling cycle, the building block B-1 or B-x is a compound of Formula II-2, preferably of Formula II-2-a, in particular of Formula II-2-b; and
- at least in the final coupling cycle, step (e) or (e') is carried out.

The "oxidizing agent" or the "sulfurizing agent" to be used in step (e) or (e') are not particularly limited in terms of their chemical structure as long as the respective agent is capable of converting any P (III) atoms within the respective oligonucleotide O-1 or O-x to P (V) atoms. An "oxidizing agent" and a "sulfurizing agent" may differ in the means of how P (III) atoms are converted to P (V) atoms. An "oxidizing agent" may introduce one or more covalent bonds between the phosphorus atom be oxidized and an oxygen atom. A "sulfurizing agent" may introduce one or more covalent bonds between the phosphorus atom to be sulfurized and a sulfur atom. As used herein, the term "oxidizing agent" preferably refers to any agent capable of converting a phosphite triester linkage group to a phosphate triester linkage group and a H-phosphonate diester linkage group to a phosphate diester (i.e. a phosphodiester) linkage group. As used herein, the term "sulfurizing agent" preferably refers to any agent capable of converting a phosphite triester linkage group to a thiophosphate triester linkage group and a H-phosphonate diester linkage group to a thiophosphate diester linkage group (i.e. a phosphorothioate) linkage group.

Iodine may be a preferred oxidizing agent. For example, an aqueous solution of iodine may be used, preferably in combination with a base such as pyridine. Optionally, a co-solvent such as tetrahydrofuran (THF) or acetonitrile may be added. As one example, a solution of iodine (e.g. 50 mM, i.e. 50 mmol/L) in a mixture of water and pyridine (e.g. 1:9 v/v) may be used. Alternatively, peroxides such as tert-butyl hydroperoxide, cumene hydroperoxides, bis-trimethylsilyl peroxide, 2-butanone peroxide, and hydrogen peroxide, or peroxy acids such as m-chloroperbenzoic acid (mCPBA) may, for example, be used as oxidizing agents. As another alternative, (1S)-(+)-(10-camphorsulfonyl)-oxaziridine (CSO) may, for example, be used as oxidizing agent, e.g. as a 0.5 M (i.e. 0.5 mol/L) solution in, e.g., acetonitrile. The oxidizing agent may, for example, be applied in form of a 0.005-5.0 M solution, preferably a 0.01-1.0 M solution in a suitable solvent, for example, selected from the group consisting of pyridine, acetonitrile, water, tetrahydrofuran, and mixtures thereof.

Xanthane hydride (5-amino-3*H*-1,2,4-dithiazole-3-thione) may be a preferred sulfurizing agent. For example, a solution of xanthane hydride in pyridine may be used, optionally in combination with a co-solvent such as acetonitrile. As one example, a solution of xanthane hydride (e.g. 0.2 M) in pyridine may be used. As another example, a solution of xanthane hydride (e.g. 0.1 M) in a mixture of pyridine and acetonitrile (e.g. 1:1, v/v) may be used. Alternatively, 1,4-dithiothreitol (DTT), phenylacetyl disulfide (PADS), 3*H*-1,2-benzodithiol-3-one 1,1-dioxide (Beaucage Reagent), 3*H*-1,2-benzodithiol-3-one, 5-ethoxy-3*H*-1,2,4-dithiazol-3-one (EDITH), or 3-(N,N-dimethylamino-methylidene)amino)-3*H*-1,2,4-dithiazole-5-thione (CAS RN: 1192027-04-5, DDTT) may be used as sulfurizing agents. The sulfurizing agent may, for example, be applied in form of a 0.005-5.0 M solution, preferably a 0.01-1.0 M solution in a suitable solvent, for example, selected from the group consisting of pyridine, acetonitrile, water, tetrahydrofuran, and mixtures thereof.

Steps (e) and (e') may preferably be performed at a temperature in the range of 0-90°C, 10-70 °C, 10-60 °C, 10-50 °C, 10-40 °C, 15-30 °C, or 15-25 °C. For convenience, step (e) and (e') may simply be performed at room temperature. Increased temperatures may result in shorter reaction times. The reaction time may also depend on the chemical structure of the reactants and will routinely be selected by a skilled person, for example, based on reaction monitoring using, e.g., thin-layer chromatography and/or high performance liquid chromatography (HPLC), optionally coupled to mass spectrometry.

The term "incubating" may in the context of step (e) refer to any process of combining the first cycle oligonucleotide O-1 obtained in step (d) and an oxidizing agent or sulfurizing agent as defined herein in a reaction vessel or reactor (e.g. a batch reactor or a column reactor). The term "incubating" may in the context of step (e') refer to any process of combining the x-th cycle oligonucleotide O-x obtained in step (d') of the x-th coupling cycle and an oxidizing agent or sulfurizing agent as defined herein in a reaction vessel or reactor (e.g. a batch reactor or a column reactor). Typically, the reaction vessel or reactor may already contain the respective oligonucleotide, followed by addition of said oxidizing or sulfurizing agent or a solution thereof.

In some embodiments of the method of the invention:
- the first coupling cycle further comprises a step (f) of reacting free hydroxyl groups with a blocking agent, wherein step (f) is carried out after step (d) or after step (e); and/or
- at least one, or each, iteration of the (n-1) iterations of the coupling cycle comprising steps (b') to (e') further comprises a step (f') of reacting free hydroxyl groups with a blocking agent, wherein step (f') is carried out after step (d') or after step (e').

In some embodiments of the invention, the first coupling cycle further comprises a step (f) of reacting free hydroxyl groups with a blocking agent, wherein step (f) is carried out after step (d) or after step (e). In some embodiments of the invention, at least one, or each, iteration of the (n-1) iterations of the coupling cycle comprising steps (b') to (e') further comprises a step (f') of reacting free hydroxyl groups with a blocking agent, wherein step (f') is carried out after step (d') or after step (e').

The term "free hydroxyl groups" in steps (f) and (f') will be understood from what has been laid out above. A free hydroxyl group formed in the course of step (b) or (b') is supposed to engage in the condensation reaction of step (d) or (d'), which consumes said free hydroxyl group in the sense of incorporating it into a newly-formed internucleosidic linkage group. However, a (typically quite small, e.g. < 1 %, < 0.5 % or < 0.1 %) fraction of the free hydroxyl groups may not engage in the condensation reaction of step (d) or (d'). Such (unreacted) free hydroxyl groups would be available to participate in the condensation reaction of step (d) or (d') of the following coupling cycle. This however may not be desirable, since it would give an oligonucleotide product lacking one nucleoside subunit. Such oligonucleotide products may be difficult to remove from the target oligonucleotide O^{T} later on, since they may differ from O^{T} only in the absence of a single nucleoside subunit. Steps (f) and (f') may serve to prevent the formation of such difficult to remove side products by blocking any (unreacted) free hydroxyl groups before entering a new iteration of the coupling cycle.

As used herein, the terms "blocking agent" and "capping agent" are used interchangeably to denote any chemical reagent capable of acylating, preferably acetylating, a free hydroxyl group. Capping agents for oligonucleotide synthesis form part of the common knowledge of those skilled in the art. Any blocking (i.e. capping) agents known from oligonucleotide synthesis may be used in the method of the invention. Preferred examples of such blocking agents comprise anhydrides of carboxylic acids, in particular acetic anhydride. For example, acetylation may be achieved by treating the growing oligonucleotide chains with neat acetic anhydride or a solution thereof, for example, in acetonitrile. An organic base such as N-methylimidazole, pyridine, lutidine (e.g. 2,6-lutidine), collidine or a mixture thereof may be used as blocking agent. As one example, a 1:1 mixture (v/v) of a capping mixture A (Cap A: 20% acetic anhydride in acetonitrile, v/v) and a capping mixture B (Cap B: *N*-methylimidazole, 2,6-lutidine, acetonitrile, 20:30:50 v/v/v) may be used as blocking agent.

The term "reacting" in steps (f) and (f') may be understood in the broadest sense as any operation during which the solid-support bound growing oligonucleotide chains are brought in contact with said blocking agent, so that the blocking/capping (i.e. acylation, preferably acetylation) of the (unreacted) free hydroxyl groups may occur.

It will be understood that, since the component C-0 is covalently linked to a solid support, the first cycle oligonucleotide O-1 is also covalently linked to said solid support, unless cleaved from it. It will also be understood that, since the building block B-1 used to prepare the first cycle oligonucleotide O-1 comprises the protecting group PG-1, the first cycle oligonucleotide O-1 will also comprise said protecting group PG-1, unless cleaving the protecting group PG-1. Such cleavage may be performed in step (b') of the second coupling cycle. If no such second coupling cycle is performed, the protecting group PG-1 and one or more further protecting groups, including the solid support, may still be cleaved from the first cycle oligonucleotide O-1.

In some embodiments of the method of the invention:
- the method further comprises a step (g) of incubating the first cycle oligonucleotide O-1 with a deprotection mixture M-g, thereby cleaving the protecting group PG-1 from the first cycle oligonucleotide O-1, so as to obtain a first cycle oligonucleotide (O-1)^{#} having a free backbone hydroxyl group; and/or
- the method further comprises a step (h) of cleaving the first cycle oligonucleotide O-1 or (O-1)^{#} from the solid support;
wherein if both steps (g) and (h) are performed, they may be performed in any order. If step (e) is performed, step (g) and/or step (h) are preferably performed after step (e). If both steps (g) and (h) are performed, step (h) is preferably performed after step (g). It will be understood that if step (g) and/or step (h) is performed, no second coupling cycle is performed.

It will be understood that, since the component C-0 is covalently linked to a solid support, the first cycle oligonucleotide O-1, and, if prepared, any (x-1)-th cycle oligonucleotide O-(x-1), (O-(x-1))^{#}, and any x-th cycle oligonucleotide O-x up to the n-th cycle oligonucleotide O-n is also covalently linked to said solid support, unless cleaving the respective oligonucleotide from said support. It will also be understood that, since the building block B-n used to prepare the n-th cycle oligonucleotide O-n in the final iteration n of the coupling cycle comprises the protecting group PG-n, the n-th cycle oligonucleotide O-n will also comprise said protecting group PG-n, unless cleaving the protecting group PG-n.

In some embodiments of the method of the invention:
- the method further comprises a step (g') of incubating the n-th cycle oligonucleotide O-n with a deprotection mixture M-g', thereby cleaving the protecting group PG-n from the n-th cycle oligonucleotide O-n, so as to obtain a n-th cycle oligonucleotide (O-n)^{#} having a free backbone hydroxyl group; and/or
- the method further comprises a step (h') of cleaving the n-th cycle oligonucleotide O-n or (O-n)^{#} from the solid support;
and wherein, if both steps (g') and (h') are performed, they may be performed in any order. If step (e') is performed in the final (i.e. n-th) coupling cycle, step (g') and/or step (h') are preferably performed after step (e'). If both steps (g') and (h') are performed, step (h') is preferably performed after step (g').

It will be understood that any explanations and embodiments pertaining to the deprotection mixture M-b may also apply to the deprotection mixture M-g. It will also be understood that any explanations and embodiments pertaining to the deprotection mixture M-b' may also apply to the deprotection mixture M-g'.

In the context of steps (h) and (h'), cleaving an oligonucleotide from the solid support may be understood in the broadest sense as any operation, typically a chemical reaction, leading to the cleavage of the covalent linkage between the respective oligonucleotide and said solid support. If the respective oligonucleotide is covalently linked to a solid support via a direct covalent bond, cleaving an oligonucleotide from the solid support in step (h) and (h') refers to the cleavage of said direct covalent bond. If the respective oligonucleotide is covalently linked to a solid support via a linker moiety, cleaving an oligonucleotide from the solid support in steps (h) and (h') refers to the cleavage of the covalent bond between said linker moiety and the respective oligonucleotide. In both cases, the respective oligonucleotide will no longer be covalently linked to the solid support. If a "capping moiety" has been introduced between the terminal nucleoside moiety of component C-0 and the linker moiety or the solid support, as e.g. shown in Formulae I, I-a, and I-b (if CA is not a covalent chemical bond), said capping moiety will not be cleaved from the respective oligonucleotide in the course of step (h) or step (h'), in contrast to a linker moiety. Therefore, as used herein, the expression "capping moiety" may refer to any moiety, which is conjugated to a terminal nucleoside moiety of the oligonucleotide strand. If present in the component C-0, the capping moiety is also comprised in the final oligonucleotide product, i.e. in the target oligonucleotide O^{T}. An example for such a strategy is the insertion of a 3'-GalNAc conjugate as described in, e.g., WO2009073809.

A person skilled in the art knows how to cleave an oligonucleotide from a solid support. Typically, such cleavage may be achieved by treating the support-bound oligonucleotide with a base such as an organic amine or alkali hydroxides, wherein concentrated aqueous ammonia (i.e. aqueous ammonium hydroxide solution) is most common and herein preferred. This base (e.g. ammonia) treatment may, for example, be performed at room temperature or under heating, for example to 40-60 °C, e.g. in an autoclave or sealed vessel. Under such alkaline conditions, the typical nucleobase protecting groups will be cleaved as well. As non-limiting, but common examples, any isobutyryl groups from the exocyclic amino group of guanine, any benzoyl groups from the exocyclic amino group of adenine, and any benzoyl or acetyl groups from the exocyclic amino group of cytosine or 5-methylcytosine will typically be cleaved (i.e. removed) under such alkaline conditions.

It is understood by those skilled in the art that the protecting groups R^{z-1} of a component C-0 of any one of Formulae I, I-a, and 1-b, as well as the protecting groups R^{z-2} of each building block B-1 and B-x of any one of Formulae II, II-a, II-b, II-1, II-1-a, II-1-b, II-2, II-2-a, and II-2-b, and the protecting groups R^{z-3} of each building block B-1 and B-x of any one of Formulae II-1, II-1-a, and II-1-b will typically still be comprised in the n-th cycle oligonucleotide O-n which is to be cleaved from the solid support. Any such protecting groups R^{z-1}, R^{z-2}, and R^{z-3} will typically be selected so that they may be cleaved (i.e. removed) under alkaline conditions, i.e. during the cleavage step (h'). R^{z-1}, R^{z-2}, and R^{z-3} being the 2-cyanoethyl protecting group is a prime example for this strategy. Any 2-cyanoethyl protecting groups may be removed in the course of the base treatment to effect cleavage from the solid support.

As known to those skilled in the art, the cleavage step (h) and step (h') may comprise subsequent treatments with different types of bases. For example, first, a solution of an organic amine such as diethylamine (DEA) or triethylamine (TEA), e.g. in a suitable solvent such as acetonitrile, may be used to remove the 2-cyanoethyl protecting groups, preferably at room temperature, followed by treatment with concentrated aqueous ammonia, preferably at a temperature in the range of 40-60 °C, to effect cleavage from the solid support and removal of base labile permanent protecting groups such as the nucleobase protecting groups.

In some embodiments of the method of the invention, said method further comprises a step (i) of modifying the first cycle oligonucleotide O-1 or (O-1)^{#}. In some embodiments of the method of the invention, said method further comprises a step (i') of modifying the n-th cycle oligonucleotide O-n or (O-n)^{#}.

In the context of steps (i) and (i') of the method of the invention, the term "modifying" may be understood in the broadest sense to embrace "chemically modifying" and/or "biotechnologically modifying" the respective oligonucleotides. In the context of steps (i) and (i') of the method of the invention, the term "chemically modifying" refers to subjecting the oligonucleotide to be chemically modified to one or more chemical reactions. Such a chemical reaction may, for example, be conjugation with a carbohydrate moiety, the introduction or removal of one or more protecting groups, and intramolecular bond formation to achieve cyclization. In the context of steps (i) and (i') of the method of the invention, the term "biotechnologically modifying" refers to subjecting the oligonucleotide to be biotechnologically modified to one or more enzymatic reactions. As used herein, the term "enzymatic reaction" refers to any reaction which is enabled by and/or catalyzed by one or more enzymes. For example, the one or more enzymatic reactions of step (i) and (i') may be used to ligate two or more nucleosides or oligonucleotides.

In some embodiments of the method of the invention, said method further comprises a step (k) of isolating the target oligonucleotide O^{T}. The means of isolating oligonucleotides upon oligonucleotide synthesis form part of the common knowledge of those skilled in the art. Typically, such a step of isolating an oligonucleotide comprises one or more purification steps and one or more steps aiming at obtaining the oligonucleotide in solid form. For oligonucleotide purification, chromatographic methods may typically be used, in particular ion exchange (especially anion exchange) chromatography and reversed phase (RP) HPLC, e.g. in form of hydrophobic interaction HPLC. These techniques are known to those skilled in the art. Additionally, a step of isolating an oligonucleotide may comprise ultrafiltration and/or desalting steps. For example, a solution obtained after cleaving oligonucleotides from the solid support may be submitted to ultrafiltration and/or desalting, ion exchange chromatography, and another round of ultrafiltration and/or desalting. Alternatively, the support-cleaved oligonucleotides may be submitted to reversed phase (RP) HPLC, e.g. in form of hydrophobic interaction HPLC. The latter method may preferably be performed, if the oligonucleotides still carry the 5'-terminal hydroxyl protecting group, e.g. the DMT group. Said 5'-protecting group may even be removed on the RP-HPLC column by passing an acidic solution through the column. If the 5'-terminal protecting group has been removed prior to purification, e.g. prior to cleaving the oligonucleotide from the support, ion exchange chromatography may be preferred. Purification is typically followed by one or more steps aiming at obtaining the oligonucleotide in solid form. Lyophilization or spray drying may, for example, be used. In some cases, it may be desirable to obtain the oligonucleotide in form of a salt with certain counter ions. In such cases salt exchange may be performed, typically prior to lyophilization or spray drying.

In some embodiments of the method of the invention, the synthesis is carried out at a scale of at least 100 mmol of the target oligonucleotide O^{T}. This means that in such embodiments, the maximum theoretical amount of the target oligonucleotide O^{T} to be obtained from the synthesis by the method of the invention is at least 100 mmol. The maximum theoretical amount of the target oligonucleotide O^{T} equals the total molar amount of the component C-0 provided in step (a), under the assumption that all process steps are 100 % efficient (i.e. proceed with quantitative yield of the desired product). For example, the scale of the synthesis would be 100 mmol, if 100 mmol of component C-0 have been utilized (e.g. provided in step (a)). It will be understood that the scale refers to a single synthesis process (i.e. to a single batch) and not to the sum of several batches handled in parallel or subsequently. This does of course not exclude such parallel or subsequent handling of batches by the method of the invention. Since the component C-0 is covalently linked to a solid support, the molar mass of the component C-0 may not be accessible. In this case, the molar amount of the component C-0 is typically assumed to be identical to the molar amount of functional groups (typically hydroxyl moieties) at the solid support used to synthesize the oligonucleotide. Herein, the term "the synthesis" refers to the synthesis of the target oligonucleotide O^{T} by the method of the invention, unless indicated differently. In some embodiments of the method of the invention, the synthesis is carried out at a scale of at least 0.1, 0.2, 0.3, 0.4, 0.5, 0.6, 0.7, 0.8, 0.9, 1.0, 2, 3, 4, 5, 6, 7, 8, 9, 10, 11, 12, 13, 14, 15, 20, 25, 30, or 35 mol.

In one aspect, the present invention provides the use of a liquid composition C comprising a solvent, a protic acid having a pKa equal to or smaller than 4, and at least one alcohol, in which alcohol, one or more hydroxyl groups are covalently bonded directly to an optionally substituted phenyl moiety, for suppressing nucleobase cleavage, in particular depurination, while effecting cleavage of a protecting group comprising an optionally substituted triarylmethyl residue, in particular a di(p-methoxyphenyl)phenylmethyl protecting group, from a hydroxyl moiety during the chemical synthesis of an oligonucleotide. In one aspect, the present invention provides the use of a liquid composition C comprising a solvent, a protic acid having a pKa equal to or smaller than 4, and at least one alcohol, in which alcohol, one or more hydroxyl groups are covalently bonded directly to an optionally substituted phenyl moiety, for suppressing nucleobase cleavage, in particular depurination, while effecting cleavage of a protecting group comprising an optionally substituted triarylmethyl residue, in particular a di(p-methoxyphenyl)phenylmethyl protecting group, from a hydroxyl moiety during the solid-phase synthesis of an oligonucleotide. In one aspect, the present invention provides the use of a liquid composition C comprising a solvent, a protic acid having a pKa equal to or smaller than 4, and at least one alcohol, in which alcohol, one or more hydroxyl groups are covalently bonded directly to an optionally substituted phenyl moiety, for suppressing nucleobase cleavage, in particular depurination, while effecting cleavage of a protecting group comprising an optionally substituted triarylmethyl residue, in particular a di(*p*-methoxyphenyl)phenylmethyl protecting group, from a hydroxyl moiety during the solid-phase synthesis of an oligonucleotide, wherein said hydroxyl moiety is part of a nucleoside moiety comprising a purine type nucleobase, preferably a nucleobase selected from the group consisting of adenine and guanine, in particular adenine.

As used herein, the term "suppressing nucleobase cleavage" may be understood in the broadest sense as reducing the degree of (undesired) nucleobase cleavage. Nucleobase cleavage may be prevented completely or partly upon cleavage of the triarylmethyl type protecting group, e.g. the DMT protecting group. Along the same lines, the term "suppressing depurination" may be understood in the broadest sense as reducing the degree of (undesired) depurination. Depurination may be prevented completely or partly upon cleavage of the triarylmethyl type protecting group, e.g. the DMT protecting group.

The degree of nucleobase cleavage / depurination may be used to compare different detritylation protocols with regards to undesired nucleobase cleavage / depurination. Briefly, in a chromatogram (preferred detection wavelength: 260 nm) obtained from HPLC-MS analysis of a synthesized oligonucleotide (cleaved from the support, if support-assisted synthesis was used), the areas of (i.e. underneath) peaks of the nucleobase cleavage-derived / depurination-derived side products may be summed up to obtain the summed up peak area of all identified nucleobase cleavage-derived / depurination-derived side products. The degree of nucleobase cleavage / depurination in percent (%) may then be determined by dividing the summed up peak area of nucleobase cleavage-derived / depurination-derived side products by the area of (i.e. underneath) the peak of the desired product of the respective oligonucleotide synthesis, followed by multiplication with 100 % to arrive at a value in percent (%). Obviously, a low degree of nucleobase cleavage / depurination is desirable. It will be understood that the terms "area of a peak" and "area underneath a peak" are herein used interchangeably. It will be understood that different detritylation protocols may preferably be compared for the synthesis of the same target oligonucleotide (leading to the same nucleobase cleavage-derived / depurination-derived side products).

In some embodiments, suppressing nucleobase cleavage is reducing the degree of nucleobase cleavage by at least 5 %, 10 %, 25 %, 50 %, 75 %, or at least 90 % in comparison to a comparable detritylation protocol differing only in that a 1.218 M (i.e. 1.218 mol/L) solution of DCA in toluene is used instead of the liquid composition C of the invention. Along the same lines, in some embodiments, suppressing depurination is reducing the degree of depurination by at least 5 %, 10 %, 25 %, 50 %, 75 %, or at least 90 % in comparison to a comparable detritylation protocol differing only in that a 1.218 M (i.e. 1.218 mol/L) solution of DCA in toluene is used instead of the liquid composition C of the invention. In this context, the term "comparable detritylation protocol" may refer to a protocol (i.e. procedure or method) of removing a protecting group comprising an optionally substituted triarylmethyl residue, preferably a DMT protecting group, under similar conditions, e.g. same substrate, scale, temperature, time, and volume of the detritylation cocktail, differing only with respect to the composition of the detritylation cocktail, e.g. 1.218 M DCA in toluene vs a liquid composition C of the present invention. The meaning of the term "reducing" in the context of the degree of nucleobase cleavage or the degree of depurination will be understood by a person skilled in the art, and may be exemplified as follows: If dividing the degree of depurination of a first synthesis A using the liquid composition C of the invention for detritylation by the degree of depurination of a second synthesis B using 1.218 M DCA in toluene for detritylation gives a quotient of 0.95, the degree of depurination is said to be reduced by 5 %.

The terms "effecting cleavage of a protecting group" and "cleaving a protecting group" may be understood in the broadest sense and refer to any process of removing a protecting group from an atom or functional group, e.g. a hydroxyl moiety, so that the latter is again available in free form, e.g. as a hydroxyl group.

In one aspect, the present invention provides a composition comprising
- an oligonucleotide which is covalently linked to a solid support and comprises a hydroxyl moiety protected by a protecting group comprising an optionally substituted triarylmethyl residue, in particular a di(p-methoxyphenyl)phenylmethyl (DMT) protecting group, and
- a liquid composition C comprising a solvent, a protic acid having a pKa equal to or smaller than 4, and at least one alcohol, in which one or more hydroxyl groups are covalently bonded directly to an optionally substituted phenyl moiety.

In a preferred embodiment, the composition and/or one or more components are preferably defined as in the context of the method herein.

In a preferred embodiment, the protecting group comprising an optionally substituted triarylmethyl residue is defined as in the context of the method herein, the protic acid is defined as in the context of the method herein, the at least one alcohol and/or the concentration thereof are defined as in the context of the method herein, and/or said solvent is defined as in in the context of the method herein.

In a preferred embodiment, the protecting group comprising an optionally substituted triarylmethyl residue, the protic acid, the at least one alcohol and/or the concentration thereof, and the solvent are each defined as in in the context of the method herein.

In one aspect, the present invention provides a composition comprising
- an oligonucleotide which is covalently linked to a solid support and comprises a hydroxyl moiety protected by a protecting group comprising an optionally substituted triarylmethyl residue, in particular a di(p-methoxyphenyl)phenylmethyl (DMT) protecting group, wherein said hydroxyl moiety is part of a nucleoside moiety comprising a purine type nucleobase, preferably a nucleobase selected from the group consisting of adenine and guanine, in particular adenine, and
- a liquid composition C comprising a solvent, a protic acid having a pKa equal to or smaller than 4, and at least one alcohol, in which one or more hydroxyl groups are covalently bonded directly to an optionally substituted phenyl moiety.

In some embodiments of the method, the use, and the composition of the invention, each protecting group comprising an optionally substituted triarylmethyl residue is at each occurrence a di(*p*-methoxyphenyl)phenylmethyl (DMT) protecting group. In such embodiments of the method of the invention, the protecting group PG-0, the protecting group PG-1 and each protecting group PG-x and each protecting group PG-(x-1) is a DMT protecting group.

In some embodiments of the method, the use, and the composition of the invention, each of said at least one alcohol, in which one or more hydroxyl groups are covalently bonded directly to an optionally substituted phenyl moiety is independently an alcohol of the following Formula D: 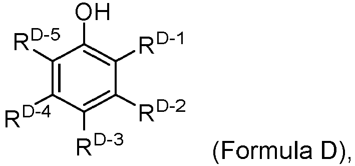 wherein in Formula D:
R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN. As used herein, in line with common practice, a carbon-bound oxygen atom may be written in brackets to denote that it is a carbonyl oxygen atom, which does not bear any further substituents. For example: O(C₁-C₆-alkyl) denotes an alkoxy group, in which the C₁-C₆-alkyl residue is bonded to the oxygen atom, while C(O)(C₁-C₆-alkyl) denotes an alkanoyl group, in which the C₁-C₆-alkyl residue is bonded to a carbonyl carbon atom.

In some embodiments, in an alcohol of the aforementioned Formula D, R^{D-1}, R^{D-2}, R^{D-3}, R^{D-4}, and R^{D-5} are independently of each other selected from the group consisting of H, CH₃, OCH₃, and OH, with the proviso that at least four of the residues R^{D-1} , R^{D-2}, R^{D-3}, R^{D-4}, and R^{D-5} are H. In some embodiments, in an alcohol of the aforementioned Formula D, R^{D-1}, R^{D-2}, R^{D-3}, R^{D-4}, and R^{D-5} are independently of each other selected from the group consisting of H, CH₃, and OCH₃, with the proviso that at least four of the residues R^{D-1}, R^{D-2}, R^{D-3}, R^{D-4}, and R^{D-5} are H.

In some embodiments of the method, the use, and the composition of the invention, each of said at least one alcohol, in which one or more hydroxyl groups are covalently bonded directly to an optionally substituted phenyl moiety is independently selected from the group consisting of *m*-cresol, 4-methoxyphenol, phenol, and resorcinol. In some embodiments of the method, the use, and the composition of the invention, each of said at least one alcohol, in which one or more hydroxyl groups are covalently bonded directly to an optionally substituted phenyl moiety is independently selected from the group consisting of *m*-cresol, 4-methoxyphenol, and phenol. In some embodiments of the method, the use, and the composition of the invention, each of said at least one alcohol, in which one or more hydroxyl groups are covalently bonded directly to an optionally substituted phenyl moiety is independently selected from the group consisting of *m*-cresol and 4-methoxyphenol. In some embodiments of the method, the use, and the composition of the invention, said at least one alcohol, in which one or more hydroxyl groups are covalently bonded directly to an optionally substituted phenyl moiety is m-cresol.

In some embodiments of the method, the use, and the composition of the invention, the liquid composition C comprises said at least one alcohol, in which one or more hydroxyl groups are covalently bonded directly to an optionally substituted phenyl moiety, in a molar concentration of 0.45-5.6 mol/L, 0.9-5.0 mol/L, 1.0-4.5 mol/L, 1.0-4.0 mol/L, 1.5-4.0 mol/L, 1.5-3.5 mol/L, 2.0-3.5 mol/L, 2.0-3.0 mol/L, or 2.5-3.0 mol/L. Herein, reference is occasionally made to molar concentrations (e.g. in mol/L (i.e. M) or in mmol/L (i.e. mM)). The molar concentration of a component of a solution or liquid composition, e.g. a liquid composition C, is herein determined by dividing the total molar amount of the respective component as added to said solution or liquid composition by the total volume of said solution or liquid composition. The total volume of a solution or liquid composition is herein determined volumetrically directly from said solution or composition after all components have been added. The term "total molar amount of the respective component" is used to highlight that, if the respective component should be added in several portions to said solution or composition, the molar amounts of these distinct portions are to be summed up to arrive at said "total molar amount of the respective component". Likewise, if the respective component is in fact a mixture of two or more components, the "total molar amount of the respective component" denotes the summed up molar amounts of these two or more components. For example, said at least one alcohol, in which one or more hydroxyl groups are covalently bonded directly to an optionally substituted phenyl moiety, may be a mixture of, e.g., 4-methoxyphenol and *m*-cresol, wherein, in this case, the total molar amount of said alcohol would be the sum of the molar amounts of 4-methoxyphenol and *m*-cresol. Unless indicated differently, volumes are herein determined at 22 °C.

In some embodiments of the method, the use, and the composition of the invention, the liquid composition C comprises said at least one alcohol, in which one or more hydroxyl groups are covalently bonded directly to an optionally substituted phenyl moiety, in a molar concentration of 0.45-5.60 mol/L and/or
each of said at least one alcohol, in which one or more hydroxyl groups are covalently bonded directly to an optionally substituted phenyl moiety is independently an alcohol of the following Formula D: 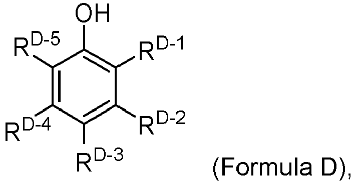
wherein in Formula D:
   R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN.

In some embodiments of the method, the use, and the composition of the invention, the liquid composition C comprises a solvent, a protic acid having pKa equal to or smaller than 4, and at least one alcohol, in which one or more hydroxyl groups are covalently bonded directly to an optionally substituted phenyl moiety, wherein
- said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof;
- each of said at least one alcohol, in which one or more hydroxyl groups are covalently bonded directly to an optionally substituted phenyl moiety, is independently an alcohol of the following Formula D: 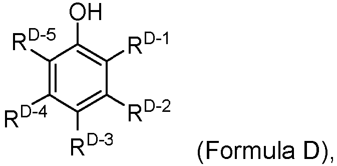 wherein in Formula D:
   R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN; and
- the sum of molar concentrations of alcohols of Formula D in the liquid composition C is within the range of 0.45-5.6 mol/L, 0.9-5.0 mol/L, 1.0-4.5 mol/L, 1.0-4.0 mol/L, 1.5-4.0 mol/L, 1.5-3.5 mol/L, 2.0-3.5 mol/L, 2.0-3.0 mol/L, or 2.5-3.0 mol/L.

In some embodiments of the method, the use, and the composition of the invention, the liquid composition C does not contain any of trifluoroethanol (i.e., 2,2,2-trifluoroethanol, TFE), hexafluoroisopropanol, (i.e., 1,1,1,3,3,3-hexafluoro-2-propanol, HFIP), pentafluoropropanol, 1,1,1,3,3,3-hexafluoro-2-methyl-2-propanol, and nonafluoro tertiary butyl alcohol. In some embodiments of the method, the use, and the composition of the invention, the liquid composition C does not contain any polyfluorinated alcohol. In this context, the term "polyfluorinated alcohol" refers to an alcohol having in its chemical structure two or more fluorine atoms covalently bonded to the same carbon atom. TFE and HFIP are examples of such polyfluorinated alcohols. In some embodiments of the method, the use, and the composition of the invention, the liquid composition C does not contain any fluorinated alcohol. In this context, the term "fluorinated alcohol" refers to an alcohol having in its chemical structure one or more fluorine atoms covalently bonded to a carbon atom. TFE and HFIP are thus also examples of fluorinated alcohols. However, 2-fluoroethanol is a fluorinated alcohol as defined herein but is not a polyfluorinated alcohol as defined herein.

In some embodiments of the method, the use, and the composition of the invention, the liquid composition C comprises a solvent, a protic acid having pKa equal to or smaller than 4, and at least one alcohol, in which one or more hydroxyl groups are covalently bonded directly to an optionally substituted phenyl moiety, wherein
- said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof;
- each of said at least one alcohol, in which one or more hydroxyl groups are covalently bonded directly to an optionally substituted phenyl moiety, is independently an alcohol of the following Formula D: 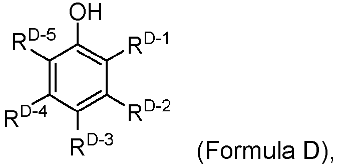 wherein in Formula D:
   R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN; and
- the liquid composition C does not contain any of trifluoroethanol (i.e., 2,2,2-trifluoroethanol, TFE), hexafluoroisopropanol, (i.e., 1,1,1,3,3,3-hexafluoro-2-propanol, HFIP), pentafluoropropanol, 1,1,1,3,3,3-hexafluoro-2-methyl-2-propanol, and nonafluoro tertiary butyl alcohol, and does preferably not contain any polyfluorinated alcohol.

In some embodiments of the method, the use, and the composition of the invention, the liquid composition C comprises a solvent, a protic acid having pKa equal to or smaller than 4, and at least one alcohol, in which one or more hydroxyl groups are covalently bonded directly to an optionally substituted phenyl moiety, wherein
- said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof;
- each of said at least one alcohol, in which one or more hydroxyl groups are covalently bonded directly to an optionally substituted phenyl moiety, is independently an alcohol of the following Formula D: 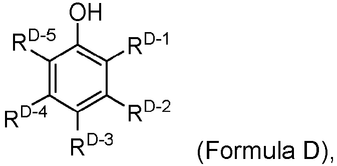 wherein in Formula D:
   R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN;
- the sum of molar concentrations of alcohols of Formula D in the liquid composition C is within the range of 0.45-5.6 mol/L, 0.9-5.0 mol/L, 1.0-4.5 mol/L, 1.0-4.0 mol/L, 1.5-4.0 mol/L, 1.5-3.5 mol/L, 2.0-3.5 mol/L, 2.0-3.0 mol/L, or 2.5-3.0 mol/L; and
- the liquid composition C does not contain any of trifluoroethanol (i.e., 2,2,2-trifluoroethanol, TFE), hexafluoroisopropanol, (i.e., 1,1,1,3,3,3-hexafluoro-2-propanol, HFIP), pentafluoropropanol, 1,1,1,3,3,3-hexafluoro-2-methyl-2-propanol, and nonafluoro tertiary butyl alcohol, and does preferably not contain any polyfluorinated alcohol.

In some embodiments of the method, the use, and the composition of the invention, the total molar amount of said at least one alcohol, in which one or more hydroxyl groups are covalently bonded directly to an optionally substituted phenyl moiety, comprised in the liquid composition C is in the range of 2.0-150.0, 2.0-120.0, 2.0-100.0, 2.0-95.0, 2.5-95.0, 3.0-95.0, 3.0-90.0, 3.0-85.0, 3.0-80.0, 3.0-75.0, 3.0-70.0, or 3.0-65.0 equivalents relative to the total molar amount of nucleobases. The term "total molar amount of nucleobases" may refer to the overall molar amount of all nucleobases present in the respective mixture, e.g. the liquid composition C. It is understood that the nucleobases will essentially all, or at least mostly, be part of nucleoside subunits of the oligonucleotides, from which the protecting group comprising an optionally substituted triarylmethyl residue, preferably the DMT group, is to be cleaved. For example, if the liquid composition C is used to cleave DMT protecting groups from 1.0 mol of an oligonucleotide, whose molecules each comprise 5 nucleobases, the total molar amount of nucleobases is 5.0 mol.

In some embodiments of the method, the use, and the composition of the invention, said protic acid comprised in the liquid composition C has a pKa in the range of -10 to 4, -7 to 4, -6 to 4, -5 to 4, -4 to 4, -3 to 4, -2 to 4, -1 to 4, 0 to 4, 0 to 3, or 0 to 2. The term "protic acid" and the conditions for determining the pKa value have been laid out above.

In some embodiments of the method, the use, and the composition of the invention, said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof. In some embodiments of the method, the use, and the composition of the invention, said protic acid comprised in the liquid composition C is selected from the group consisting of a carboxylic acid, a sulfonic acid, a mineral acid, a protonated aliphatic or heteroaromatic amine, whose protonated form has a pKa in the range of 1-4, and mixtures thereof. In some embodiments of the method, the use, and the composition of the invention, said protic acid comprised in the liquid composition C is selected from the group consisting of a carboxylic acid, a sulfonic acid, a mineral acid, a protonated heteroaromatic amine, whose protonated form has a pKa in the range of 1-4, and mixtures thereof.

Examples of carboxylic acids of the liquid composition C of the invention are halogenated acetic acids such as trifluoroacetic acid (TFA), dichloroacetic acid (DCA), and trichloroacetic acid (TCA), wherein TFA and DCA may be preferred. Examples of sulfonic acids of the liquid composition C of the invention are methanesulfonic acid and *p*-toluenesuffonic acid. Examples of mineral acids of the liquid composition C of the invention are hydrochloric acid and sulfuric acid.

Examples of aliphatic amines of the liquid composition C of the invention are triethylamine (TEA) and diisopropylethylamine (DIPEA). Examples of aromatic amines of the liquid composition C of the invention are diphenylamine and aniline derivatives with electron withdrawing substituents. As used herein, the term "electron withdrawing substituent(s)" may refer to substituents selected from the group consisting of a halogen atom such as a chlorine, fluorine, or bromine, a cyano group, an aldehyde group, a keto group, a carboxyester group, or a carboxamide group, unless indicated differently in the context of specific embodiments. Examples of heteroaromatic amines of the liquid composition C of the invention are pyrimidine, pyridine, pyrazine, thiazole, pyridazine, pyrazole or triazole, all of which may optionally be substituted with electron donating or electron withdrawing substituents. For example, the heteroaromatic amine may be a pyrimidine, a pyridine, a thiazole, a pyridazine, a pyrazole, or a 1,2,4-triazole, substituted with one or more electron withdrawing substituent(s). As another example, the heteroaromatic amine may be a pyrimidine or a pyrazine substituted with one or more electron donating substituent(s). The electron donating substituent(s) may for example be a methoxy group. Preferably, the heteroaromatic amine may be a pyridine, which is substituted with one or more electron withdrawing substituents selected from the group consisting of a halogen atom, a cyano group, an aldehyde group, a keto group, a carboxyester group, and a carboxamide group. Preferably, the heteroaromatic amine may be a pyridine, in which exactly one hydrogen residue is substituted by an electron withdrawing substituent selected from the group consisting of a cyano group and a halogen atom (F, Cl, Br, I). In particular, the heteroaromatic amine may be selected from the group consisting of 4-cyanopyridine, 3-cyanopyridine, 4-chloropyridine, 3-chloropyridine, and a mixture thereof, among which 4-cyanopyridine may be most preferred. For example, the pKa values of diversely substituted pyridinium ions may be taken from or determined according to the procedure disclosed in: A. Fischer et al., Journal of the Chemical Society 1964, 3591-3596.

It will be understood by those skilled in the art that a protonated form of an aliphatic, aromatic or heteroaromatic amine is typically obtained by combining said amine with a protic acid, e.g. a carboxylic acid or sulfonic acid or mineral acid, which is capable of protonating the amine. It will be understood that said combination of an acid and an amine may be a preformed salt of the acid and the amine. Alternatively, the acid and the heteroaromatic amine may be added as such (i.e. not as preformed salt). Thus, the amine may be combined with a protic acid, wherein the protic acid has a smaller pKa value than the protonated form of the amine. The combination of said amine and said protic acid may occur beforehand, so as to obtain a salt of a protic acid and an amine. Alternatively, the amine and a protic acid may be combined in the liquid composition C.

In some embodiments of the method, the use, and the composition of the invention, said protic acid comprised in the liquid composition C is selected from the group consisting of a (hetero)alkylsulfonic acid, an (hetero)arylsulfonic acid, a hydrogen halide, sulfuric acid, a protonated heteroaromatic amine, whose protonated form has a pKa in the range of 1-4, and mixtures thereof. In some embodiments of the method, the use, and the composition of the invention, said protic acid comprised in the liquid composition C is selected from the group consisting of a dihalogenated acetic acid, a trihalogenated actic acid, an alkylsulfonic acid, an arylsulfonic acid, a hydrogen halide, sulfuric acid, a protonated heteroaromatic amine, whose protonated form has a pKa in the range of 1-4, and mixtures thereof. In some embodiments of the method, the use, and the composition of the invention, said protic acid comprised in the liquid composition C is selected from the group consisting of a dihalogenated acetic acid, a trihalogenated actic acid, an alkylsulfonic acid, an arylsulfonic acid, a hydrogen halide, a protonated heteroaromatic amine selected from the group consisting of 4-cyanopyridine, 3-cyanopyridine, 4-chloropyridine, and 3-chloropyridine, and mixtures thereof. In some embodiments of the method, the use, and the composition of the invention, said protic acid comprised in the liquid composition C is selected from the group consisting of trifluoroacetic acid, dichloroacetic acid, trichloroacetic acid, hydrochloric acid, methanesulfonic acid, benzenesulfonic acid, *p*-toluenesulfonic acid, a combination of any of these acids with a heteroaromatic amine selected from the group consisting of 4-cyanopyridine, 3-cyanopyridine, 4-chloropyridine, and 3-chloropyridine, and mixtures thereof. In some embodiments of the method, the use, and the composition of the invention, said protic acid comprised in the liquid composition C is selected from the group consisting of trifluoroacetic acid, dichloroacetic acid, trichloroacetic acid, hydrochloric acid, methanesulfonic acid, *p-*toluenesulfonic acid, a combination of any of these acids with a heteroaromatic amine selected from the group consisting of 4-cyanopyridine, 3-cyanopyridine, 4-chloropyridine, and 3-chloropyridine, and mixtures thereof. In some embodiments of the method, the use, and the composition of the invention, said protic acid comprised in the liquid composition C is selected from the group consisting of trifluoroacetic acid, dichloroacetic acid, hydrochloric acid, methanesulfonic acid, a combination of any of these acids with a heteroaromatic amine selected from the group consisting of 4-cyanopyridine, 3-cyanopyridine, 4-chloropyridine, and 3-chloropyridine, and mixtures thereof. In some embodiments of the method, the use, and the composition of the invention, said protic acid comprised in the liquid composition C is selected from the group consisting of trifluoroacetic acid, dichloroacetic acid, 4-chloropyridinium hydrochloride, and 4-cyanopyridiniumtrifluoroacetate. In some embodiments of the method, the use, and the composition of the invention, said protic acid comprised in the liquid composition C is selected from the group consisting of dichloroacetic acid and 4-cyanopyridinium trifluoroacetate. In some embodiments of the method, the use, and the composition of the invention, said protic acid comprised in the liquid composition C is dichloroacetic acid. In some embodiments of the method, the use, and the composition of the invention, said protic acid comprised in the liquid composition C is 4-cyanopyridinium trifluoroacetate.

In some embodiments of the method, the use, and the composition of the invention, the liquid composition C comprises said protic acid in a concentration of 0.01-2.0 mol/L, 0.05-1.5 mol/L, 0.05-1.25 mol/L, 0.05-1.0 mol/L, 0.05-0.9 mol/L, 0.05-0.8 mol/L, 0.05-0.7 mol/L, 0.05-0.6 mol/L, 0.05-0.5 mol/L, 0.05-0.4 mol/L, 0.05-0.35 mol/L, 0.06-0.35 mol/L, 0.07-0.35 mol/L, 0.08-0.35 mol/L, 0.09-0.35 mol/L, 0.09-0.3 mol/L, or 0.09-0.25 mol/L. The means of determining the molar concentration of a component of a solution or liquid composition, e.g. a liquid composition C, have been laid out above and apply to said protic acid comprised in the liquid composition C. As laid out above, said protic acid may also be a protonated aliphatic, aromatic or heteroaromatic amine, whose protonated form has a pKa in the range of 1-4. As also laid out above, such a protonated form of an amine may typically be obtained by combining said amine with a protic acid, e.g. a carboxylic acid or sulfonic acid or mineral acid, which is capable of protonating the amine. This combination may either occur by adding the amine and the acid into the respective solution or by preforming a salt of said acid and said amine and adding the preformed salt to the solution. If a preformed salt is used, the molar concentration of the protic acid is to be determined based on the total molar amount of said preformed salt, which was used to prepare the liquid composition, e.g. the liquid composition C, provided that the acid and the amine are present in a 1:1 stoichiometry in said salt, as e.g. in 4-chloropyridinium hydrochloride. Otherwise, the stoichiometry of the acid and the amine in said salt will routinely be taken into account. If, however, a protic acid and a non-protonated amine are added separately and protonation of the amine may occur after addition, the total molar amount of the protic acid added alongside the amine in the first place is to be used for calculating the concentration of the protic acid in the liquid composition, e.g. the liquid composition C. As a first example: If 4-cyanopyridine and trifluoroacetic acid (TFA) are added separately during the preparation of a liquid composition C, the total molar amount of TFA is used for calculating the molar concentration of said protic acid comprised in the liquid composition C. As a second example: If a preformed salt, e.g. 4-chloropyridinium hydrochloride, is added during the preparation of a liquid composition C, the total molar amount of said salt is used for calculating the molar concentration of said protic acid comprised in said liquid composition C.

In some embodiments of the method, the use, and the composition of the invention, the total molar amount of said protic acid comprised in the liquid composition C is in the range of 0.80-15.0, 1.0-13.0, or 2.0-12.50 equivalents relative to the total molar amount of protecting groups comprising an optionally substituted triarylmethyl residue, preferably the di(*p*-methoxyphenyl)phenylmethyl (DMT) groups. The term "total molar amount" when referring to a protecting group such as the DMT group refers to the total molar amount of the compound(s) carrying the respective protecting group multiplied with the amount of said protecting group per molecule of these compound(s). For example, if a composition comprises 1.0 mol of a compound having exactly one DMT group per molecule (and no other compound in said composition comprises any DMT groups), the total molar amount of DMT groups in said composition equals the total molar amount of said compound and would in this example also be 1.0 mol.

In some embodiments of the method, the use, and the composition of the invention, said solvent comprised in the liquid composition C is an aprotic solvent. As used herein, an "aprotic solvent" is a solvent that is not a hydrogen bond donor. Hence, an aprotic solvent may be a solvent without any O-H or N-H bonds. Thus, an alcohol is not an aprotic solvent as defined herein. In some embodiments of the method, the use, and the composition of the invention, said solvent comprised in the liquid composition C is a non-halogenated aprotic solvent. As used herein, a "non-halogenated solvent" is a solvent which does not comprise any halogen atoms (in particular F, Cl, Br, I) in its chemical structure. In some embodiments of the method, the use, and the composition of the invention, said liquid composition C comprises essentially no halogenated solvents. Examples of such halogenated solvents are dichloromethane (DCM), chloroform, and 1,2- or 1,1-dichloromethane. As used herein, the term "essentially no halogenated solvent" preferably means that any halogenated solvents (i.e. any solvents comprising in their chemical structure at least one halogen atom) together account for equal to or less than 3.0 %, 2.0 %, 1.0 %, 0.1 %, 0.01 %, or 0.001 % of the overall volume of said liquid composition C. If, for example, one or more halogenated solvents should be added during the preparation of a liquid composition C, the summed up volumes of these one or more halogenated solvents as added may be divided by the volumetrically determined total volume of the liquid composition C after all solvents and components have been added, followed by multiplication with 100 % to arrive at a value in % which represents the volume-% for which said one or more halogenated solvents account. The volume of the added solvent(s) and the volume of the liquid composition may be determined at 22 °C. Preferably, no halogenated solvent is added during the preparation of a liquid composition C and any potentially comprised traces of halogenated solvents in the liquid composition C may only be impurities of the solvent(s) or component(s) as obtained (commercially).

In some embodiments of the method, the use, and the composition of the invention, said solvent comprised in the liquid composition C is selected from the group consisting of a halogenated hydrocarbon solvent, a (hetero)aromatic solvent, an alkyl (hetero)aromatic solvent, a (hetero)aromatic ether, an alkyl (hetero)aryl ether, and mixtures thereof. Non-limiting examples of a halogenated hydrocarbon solvent comprise dichloromethane (DCM), dichloroethane, and chloroform. A non-limiting example of a (hetero)aromatic solvent is benzene. Non-limiting examples of an alkyl (hetero)aromatic solvent are toluene, *o*-xylene, *m*-xylene, *p*-xylene, and mesitylene. A non-limiting example of a (hetero)aromatic ether is diphenyl ether. A non-limiting example of an alkyl (hetero)aryl ether is anisole.

In some embodiments of the method, the use, and the composition of the invention, said solvent comprised in the liquid composition C is selected from the group consisting of
- benzene, in which one or more hydrogen residues may optionally be substituted by a C₁-C₃-alkyl group or a O(C₁-C₃-alkyl) group (e.g. benzene, toluene, *o*-xylene, *m*-xylene, *p*-xylene, mesitylene, or anisole);
- a halogenated C₁-C₃-alkyl solvent (e.g. dichloromethane, 1,1- or 1,2-dichloroethane, or chloroform);
- an O(C₁-C₆-alkyl)₂ ether with in total not less than 4 carbon atoms (e.g. diethyl ether or cycplopentyl methyl ether);
- an aliphatic cyclic ether with 4-6 ring carbon atoms, preferably 4 or 5 ring carbon atoms, in which one or more hydrogen residues may optionally be substituted by a C₁-C₃-alkyl group (e.g. tetrahydrofuran, tetrahydropyran or 1,4-dioxane);
- a C₅-C₉-alkyl solvent (e.g. pentane, hexanes, cyclohexane, heptane, octane or nonane);
- a C₁-C₃-alkyl solvent in which exactly one hydrogen residue is substituted for a nitrile group (CN) (e.g. acetonitrile or propionitrile);
- an ester solvent of the formula (C₁-C₆-alkyl)-O-C(O)-(C₁-C₆-alkyl) (e.g. ethyl acetate); and
- mixtures thereof.

In some embodiments of the method, the use, and the composition of the invention, said solvent comprised in the liquid composition C is selected from the group consisting of
- benzene, in which one or more hydrogen residues may optionally be substituted by a C₁-C₃-alkyl group or a O(C₁-C₃-alkyl) group (e.g. benzene, toluene, o-xylene, m-xylene, p-xylene, mesitylene, or anisole);
- an O(C₁-C₆-alkyl)₂ ether with in total not less than 4 carbon atoms (e.g. diethyl ether or cycplopentyl methyl ether);
- an aliphatic cyclic ether with 4-6 ring carbon atoms, preferably 4 or 5 ring carbon atoms, in which one or more hydrogen residues may optionally be substituted by a C₁-C₃-alkyl group (e.g. tetrahydrofuran, tetrahydropyran or 1,4-dioxane);
- a C₅-C₉-alkyl solvent (e.g. pentane, hexanes, cyclohexane, heptane, octane or nonane);
- a C₁-C₃-alkyl solvent in which exactly one hydrogen residue is substituted for a nitrile group (CN) (e.g. acetonitrile or propionitrile);
- an ester solvent of the formula (C₁-C₆-alkyl)-O-C(O)-(C₁-C₆-alkyl) (e.g. ethyl acetate); and
- mixtures thereof.

In some embodiments of the method, the use, and the composition of the invention, said solvent comprised in the liquid composition C is selected from the group consisting of
- benzene, in which one or more hydrogen residues may optionally be substituted by a C₁-C₃-alkyl group or a O(C₁-C₃-alkyl) group (e.g. benzene, toluene, *o*-xylene, *m*-xylene, *p*-xylene, mesitylene, or anisole);
- an O(C₁-C₆-alkyl)₂ ether with in total not less than 4 carbon atoms (e.g. diethyl ether or cycplopentyl methyl ether);
- an aliphatic cyclic ether with 4-6 ring carbon atoms, preferably 4 or 5 ring carbon atoms, in which one or more hydrogen residues may optionally be substituted by a C₁-C₃-alkyl group (e.g. tetrahydrofuran, tetrahydropyran or 1,4-dioxane);
- a C₅-C₉-alkyl solvent (e.g. pentane, hexanes, cyclohexane, heptane, octane or nonane); and
- mixtures thereof.

In some embodiments of the method, the use, and the composition of the invention, said solvent comprised in the liquid composition C is selected from the group consisting of
- benzene, in which one or more hydrogen residues may optionally be substituted by a C₁-C₃-alkyl group or a O(C₁-C₃-alkyl) group (e.g. benzene, toluene, *o*-xylene, *m*-xylene, *p*-xylene, mesitylene, or anisole);
- a halogenated C₁-C₃-alkyl solvent (e.g. dichloromethane, 1,1- or 1,2-dichloroethane, or chloroform); and
- mixtures thereof.

In some embodiments of the method, the use, and the composition of the invention, said solvent comprised in the liquid composition C is benzene, in which one or more hydrogen residues may optionally be substituted by a C₁-C₃-alkyl group or a O(C₁-C₃-alkyl) group (e.g. benzene, toluene, *o*-xylene, *m*-xylene, *p*-xylene, mesitylene, or anisole) or a mixture of such solvents.

In some embodiments of the method, the use, and the composition of the invention, said solvent comprised in the liquid composition C is selected from the group consisting of toluene, anisole, *o*-xylene, *m*-xylene, *p*-xylene, mesitylene, dichloromethane, and mixtures thereof. In some embodiments of the method, the use, and the composition of the invention, said solvent comprised in the liquid composition C is selected from the group consisting of toluene, anisole, *o*-xylene, *m*-xylene, *p*-xylene, mesitylene, and mixtures thereof. In some embodiments of the method, the use, and the composition of the invention, said solvent comprised in the liquid composition C is selected from the group consisting of toluene, anisole, and mixtures thereof. In some embodiments of the method, the use, and the composition of the invention, said solvent comprised in the liquid composition C is toluene. In some embodiments of the method, the use, and the composition of the invention, said solvent comprised in the liquid composition C is anisole. In some embodiments of the method, the use, and the composition of the invention, said solvent comprised in the liquid composition C is selected from the group consisting of toluene, anisole, dichloromethane, and mixtures thereof.

In some embodiments of the method, the use, and the composition of the invention, the total volume of said solvent comprised in the liquid composition C accounts for 50-95 %, 50-90 %, 50-85 %, 50-80 %, 50-75 %, 50-70 %, or 60-70 % of the total volume of the liquid composition C. In order to determine the percentage of the total volume for which said solvent accounts, the total volume of said solvent added during the preparation of the liquid composition C is divided by the total volume of the liquid composition C, followed by multiplication with 100 % to arrive at a value in percent (%). The total volume of the liquid composition is herein determined volumetrically directly from said composition after all components have been added. The volume of the added solvent(s) and the volume of the liquid composition are to be determined at 22 °C.

In some embodiments of the method, the use, and the composition of the invention, the total volume of the liquid composition C is in the range of 5-100 mL, 10-90 mL, 10-80 mL, 20-75 mL, 10-70 mL, 10-60 mL, 20-60 mL, 30-60 mL or 40-60 mL per 1 millimole (mmol) of the protecting group comprising an optionally substituted triarylmethyl residue, preferably the DMT group, to be cleaved (i.e. removed). The skilled practitioner will routinely adapt the total volume of the liquid composition C, so that the volume of the slurry containing the solid support is minimal while assuring efficient mass transport of reagents to the growing oligonucleotide chains and suitable macroscopic properties of the slurry.

In some embodiments of the method, the use, and the composition of the invention:
- the total molar amount of said protic acid comprised in the liquid composition C is in the range of 0.80-15.0 equivalents relative to the total molar amount of the protecting groups comprising an optionally substituted triarylmethyl residue; and
- the total molar amount of said alcohol, in which one or more hydroxyl groups are covalently bonded directly to an optionally substituted phenyl moiety, comprised in the liquid composition C is in the range of the 2.0-210.0 equivalents relative to the total molar amount of nucleobases.

In some embodiments of the method, the use, and the composition of the invention, the liquid composition C further comprises a carbocation scavenger. As used herein, the term "carbocation scavenger" relates to a nucleophilic compound, which may be used to bind a carbocation or to consume a carbocation by formal donation of a hydride anion, thereby preventing undesired side reactions of the carbocation. Typical examples of such carbocations are carbocations formed during the cleavage of protecting groups comprising an optionally substituted triarylmethyl residue. For example, the cleavage of a di(*p*-methoxyphenyl)phenylmethyl (DMT) protecting group may result in a DMT cation (i.e. a di(*p*-methoxyphenyl)phenylmethyl cation). Examples of carbocation scavengers are aliphatic alcohols (e.g. methanol and ethanol), water, silanes (e.g. triisopropylsilane (TIS) and triethylsilane (TES)), N-heterocycles (e.g. pyrrole, 3-methylpyrrole, 2,4-dimethylpyrrole indole, 2-methylindole, thiols and thiophenols (e.g. 1,2-ethanedithiol (EDT), 1,4-dithioerythrol (DTE), 1,4-dithiothreitol (DTT), 3,6-dioxa-1,8-octanedithiol (DODT), 1,4-benzenedimethanthiol (BDMT), 1,4-butanedithiol, 2-mercaptoethanol, cysteine, thiophenol, *p*-thiocresol, and thiomalic acid), and polyalkylbenzenes (e.g. 1,3,5-trimethylbenzene and pentamethylbenzene).

In some embodiments of the method, the use, and the composition of the invention, the contact time between the liquid composition C and the oligonucleotide from which said protecting group comprising an optionally substituted triarylmethyl residue is to be cleaved is at least 5 min, at least 6 min, at least 7 min, at least 8 min, at least 9 min, at least 10 min, at least 15 min, at least 20 min, at least 30 min, or even more than 1 hour. Such comparably long-time intervals may allow the use of standard pumps having moderate pumping rates for comparably large-size reactors (e.g., column or batch reactors). Such long contacting times would result in undesired cleavage of nucleobases, in particular depurination, when used for detritylation cocktails commonly used in the art such as, e.g., detritylation cocktails containing larger contents of acids such as dichloroacetic acid (DCA) without an alcohol as defined in the present invention. It is a particular advantage of the present invention that the methods of oligonucleotide synthesis can be scaled up without a need to increase the maximum flow rate of the liquid handling system. Furthermore, compared to the standard deprotection protocol using 1.218 M dichloroacetic acid in toluene, the methods of the present invention may allow to increase batch size by a factor of at least 5 fold, preferably as least 10, 20, 30, or 40 fold, while using the same liquid handling system for supplying liquid to and draining liquid from the reaction vessel. For the sake of clarity, it is noted that the reaction vessel may be changed to accommodate the larger amount of reagents.

In some embodiments of the method, the use, and the composition of the invention:
- each of said at least one alcohol, in which one or more hydroxyl groups are covalently bonded directly to an optionally substituted phenyl moiety, comprised in the liquid composition C is an alcohol of the above-mentioned Formula D;
- said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof;
- said solvent comprised in the liquid composition C is an aprotic solvent, preferably a non-halogenated aprotic solvent; and
- the liquid composition C comprises said at least one alcohol, in which one or more hydroxyl groups are covalently bonded directly to an optionally substituted phenyl moiety, in a molar concentration of 0.45-5.6 mol/L.

In some embodiments of the method, the use, and the composition of the invention:
- each of said at least one alcohol, in which one or more hydroxyl groups are covalently bonded directly to an optionally substituted phenyl moiety, comprised in the liquid composition C is an alcohol of the above-mentioned Formula D;
- said protic acid comprised in the liquid composition C is selected from the group consisting of a carboxylic acid, a sulfonic acid, a mineral acid, a protonated heteroaromatic amine, whose protonated form has a pKa in the range of 1-4, and mixtures thereof;
- said solvent comprised in the liquid composition C is an aprotic solvent, preferably a non-halogenated aprotic solvent; and
- the liquid composition C comprises said at least one alcohol, in which one or more hydroxyl groups are covalently bonded directly to an optionally substituted phenyl moiety, in a molar concentration of 0.45-5.6 mol/L.

In some embodiments of method, the use, and the composition of the invention:
- each of said at least one alcohol, in which one or more hydroxyl groups are covalently bonded directly to an optionally substituted phenyl moiety, comprised in the liquid composition C is an alcohol of the above-mentioned Formula D, wherein R^{D-1}, R^{D-2}, R^{D-3}, R^{D-4}, and R^{D-5} are independently of each other selected from the group consisting of H, CH₃, and OCH₃, with the proviso that at least four of the residues R^{D-1}, R^{D-2}, R^{D-3}, R^{D-4}, and R^{D-5} are H;
- said protic acid comprised in the liquid composition C is selected from the group consisting of trifluoroacetic acid, dichloroacetic acid, trichloroacetic acid, hydrochloric acid, methanesulfonic acid, benzenesulfonic acid, p-toluenesulfonic acid, a combination of any of these acids with a heteroaromatic amine selected from the group consisting of 4-cyanopyridine, 3-cyanopyridine, 4-chloropyridine, and 3-chloropyridine, and mixtures thereof;
- said solvent comprised in the composition C is selected from the group consisting of benzene, in which one or more hydrogen residues may optionally be substituted by a C₁-C₃-alkyl group or a O(C₁-C₃-alkyl) group, and dichloromethane; and
- the liquid composition C comprises said at least one alcohol, in which one or more hydroxyl groups are covalently bonded directly to an optionally substituted phenyl moiety, in a molar concentration of 0.45-5.6 mol/L.

In some embodiments of method, the use, and the composition of the invention:
- each of said at least one alcohol, in which one or more hydroxyl groups are covalently bonded directly to an optionally substituted phenyl moiety, comprised in the liquid composition C is an alcohol of the above-mentioned Formula D, wherein R^{D-1}, R^{D-2}, R^{D-3}, R^{D-4}, and R^{D-5} are independently of each other selected from the group consisting of H, CH₃, and OCH₃, with the proviso that at least four of the residues R^{D-1}, R^{D-2}, R^{D-3}, R^{D-4}, and R^{D-5} are H;
- said protic acid comprised in the liquid composition C is selected from the group consisting of trifluoroacetic acid, dichloroacetic acid, trichloroacetic acid, hydrochloric acid, methanesulfonic acid, *p*-toluenesulfonic acid, a combination of any of these acids with a heteroaromatic amine selected from the group consisting of 4-cyanopyridine, 3-cyanopyridine, 4-chloropyridine, and 3-chloropyridine, and mixtures thereof;
- said solvent comprised in the composition C is benzene, in which one or more hydrogen residues may optionally be substituted by a C₁-C₃-alkyl group or a O(C₁-C₃-alkyl); and
- the liquid composition C comprises said at least one alcohol, in which one or more hydroxyl groups are covalently bonded directly to an optionally substituted phenyl moiety, in a molar concentration of 0.45-5.6 mol/L.

In some embodiments of the method, the use, and the composition of the invention:
- each of said at least one alcohol, in which one or more hydroxyl groups are covalently bonded directly to an optionally substituted phenyl moiety, comprised in the liquid composition C is selected from the group consisting of *m*-cresol, 4-methoxyphenol, and phenol;
- said protic acid comprised in the liquid composition C is selected from the group consisting of trifluoroacetic acid, dichloroacetic acid, trichloroacetic acid, hydrochloric acid, methanesulfonic acid, *p*-toluenesulfonic acid, a combination of any of these acids with a heteroaromatic amine selected from the group consisting of 4-cyanopyridine, 3-cyanopyridine, 4-chloropyridine, and 3-chloropyridine, and mixtures thereof;
- said solvent comprised in the composition C is selected from the group consisting of toluene, anisole, *o*-xylene, *m*-xylene, *p*-xylene, mesitylene, and mixtures thereof; and
- the liquid composition C comprises said at least one alcohol, in which one or more hydroxyl groups are covalently bonded directly to an optionally substituted phenyl moiety, in a molar concentration of 0.45-5.6 mol/L.

In some embodiments of the method, the use, and the composition of the invention:
- each of said at least one alcohol, in which one or more hydroxyl groups are covalently bonded directly to an optionally substituted phenyl moiety, comprised in the liquid composition C is selected from the group consisting of *m*-cresol, 4-methoxyphenol, and phenol;
- said protic acid comprised in the liquid composition C is selected from the group consisting of trifluoroacetic acid, dichloroacetic acid, hydrochloric acid, methanesulfonic acid, a combination of any of these acids with a heteroaromatic amine selected from the group consisting of 4-cyanopyridine, 3-cyanopyridine, 4-chloropyridine, and 3-chloropyridine, and mixtures thereof;
- said solvent comprised in the composition C is selected from the group consisting of toluene, anisole, *o*-xylene, *m*-xylene, *p*-xylene, mesitylene, and mixtures thereof; and
- the liquid composition C comprises said at least one alcohol, in which one or more hydroxyl groups are covalently bonded directly to an optionally substituted phenyl moiety, in a molar concentration of 0.45-5.6 mol/L;
- the liquid composition C comprises said protic acid in a molar concentration of 0.01-2.0 mol/L.

The present invention also refers to a use of a liquid composition C (deprotection mixture M-b) comprising a solvent, a protic acid having a pKa equal to or smaller than 4, and at least one alcohol, in which one or more hydroxylgroups are covalently bonded directly to an optionally substituted phenyl moiety, for cleaving one or more protecting groups PG-0 form a nucleoside or oligonucleotide.

Preferably, the liquid composition C (deprotection mixture M-b) and/or one or more of its components is described as laid out in the context of the method or the composition.

### Description of the Figure

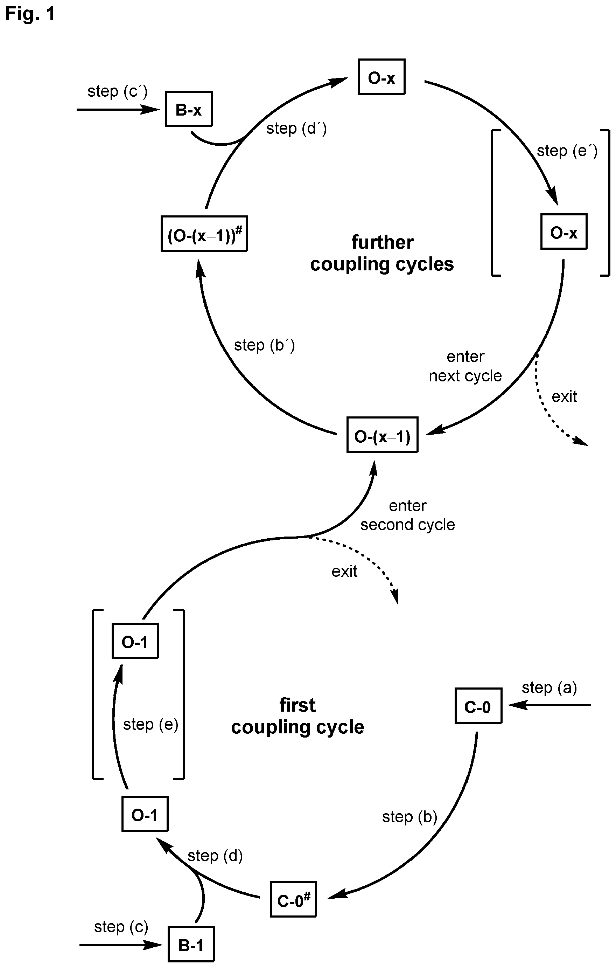

**Figure 1** illustrates the one or more coupling cycles which may be performed as part of the method of the invention.

The method of the invention may comprise a first coupling cycle comprising steps (b) to (e), wherein step (e) is optional which is indicated in Figure 1 by embracing step (e) in brackets. Prior to the first coupling cycle, a component C-0 is provided [step (a)]. In the first coupling cycle, the protecting group PG-0 is removed from said component C-0 [step (b)], and the resulting component C-0^{#} is reacted with a provided first building block B-1 [steps (c), (d)] to yield a first cycle oligonucleotide O-1. Optionally, any P (III) atoms within O-1 may be converted to P (V) atoms by incubation with an oxidizing or sulfurizing agent [step (e)].

The method of the invention may further comprise one or more coupling cycles comprising steps (b') to (e'), wherein step (e') is optional which is indicated in Figure 1 by embracing step (e') in brackets.

The first cycle oligonucleotide O-1 will then be used as an educt O-(x-1), of the second coupling cycle (x=2). The protecting group PG-(x-1), i.e. PG-1, is removed [step (b')], and the resulting oligonucleotide (O-(x-1))^{#}, i.e. (O-1)^{#}, is reacted with a provided building block B-x, i.e. B-2 [steps (c'), (d')] to yield a x-th cycle oligonucleotide O-x, i.e. a second cycle oligonucleotide O-2. Optionally, any P (III) atoms within O-x, i.e. O-2, may be converted to P (V) atoms by use of an oxidizing or sulfurizing agent [step (e')].

Up to (n-1) further coupling cycles comprising steps (b') to (e') may be performed, in each of which the oligonucleotide O-x of the previous coupling cycle is used as educt O-(x-1).

The fact that the first cycle oligonucleotide O-1 need not be subjected to the second coupling cycle and the fact that each x-th cycle oligonucleotide O-x need not be subjected to another coupling cycle are indicated in Figure 1 by the dashed "exit" arrows.

### Examples

### General methods and information

### 1. Determination of weight gain and yield

The experimental weight gain was determined by subtracting the mass of the solid support from the mass of the dried support-bound oligonucleotides after completion of all coupling cycles, the final dimethoxytrityl removal step (i.e. after the additional step (g) or (g') was carried out), and the diethylamine treatment to remove the 2-cyanoethyl protecting groups from the internucleosidic linkage groups. To determine the mass of the solid support, the mass of the dimethoxytrityl groups (i.e. the di(p-methoxyphenyl)phenylmethyl groups or DMT groups) was subtracted from the mass of the starting resin (which consisted of the solid support, optionally carrying the starting nucleoside, with dimethoxytrityl groups) according to the scale of the synthesis and the molecular weight of the dimethoxytrityl group. The theoretical maximum weight gain was determined by multiplying the molecular weight of the assembled oligonucleotide strand after removal of any 2-cyanoethyl protecting groups from the internucleosidic linkage groups (without taking the molecular weight of the solid support into account) with the molar amount of the DMT-protected hydroxyl moieties originally available at the staring resin (assuming that all of these hydroxyl moieties now carry the desired oligonucleotide strand). Unless indicated differently, yields are given in percent (%) and were obtained by dividing the experimental weight gain by the theoretical maximum weight gain, followed by multiplication with 100 % to arrive at a value in percent.

### 2. Determination of purity

Unless indicated differently, oligonucleotide purity was determined by analytical reversed phase HPLC using a C18 stationary phase, and elution was effected by a gradient (eluent A: HFIP/TEA/H₂O; eluent B: methanol). The detection wavelength was 260 nm. Unless specified otherwise, the reported oligonucleotide purities in percent (%) were obtained by dividing the area of (i.e. underneath) the peak of the target oligonucleotide by the sum of areas of (i.e. underneath) all significant peaks (including the peak of the target oligonucleotide), followed by multiplication with 100 % to arrive at a value in percent (%). Unless indicated differently, the UV trace was integrated applying a minimal relative area threshold of 0.05 %. Unless indicated differently, the determination of purity was performed using support-cleaved oligonucleotides. Such support-cleaved oligonucleotides were obtained as stated in section "4. Synthesis of oligonucleotides", unless indicated differently.

### 3. Determination of the degree of depurination

Unless indicated differently, the degree of depurination was determined from the UV-trace of the respective HPLC-MS measurements via the following steps (i)-(iii):
(i) The area of (i.e. underneath) the peak(s) of the one or more identified depurination-derived side products was determined. If more than one such peak was identified, the peak areas were summed up to obtain the summed up peak area of all identified depurination-derived side products.
(ii) The area of the peak of the target oligonucleotide was determined.
(iii) The peak area or summed up peak area determined in step (i) was divided by the peak area determined in step (ii), followed by multiplication with 100 % to arrive at a value in percent (%).

Unless indicated differently, the determination of the degree of depurination was performed using the support-cleaved oligonucleotides. Such support-cleaved oligonucleotides were obtained as stated in section "4. Synthesis of oligonucleotides", unless indicated differently.

### 4. Synthesis of oligonucleotides

### Reactors

Unless indicated differently, all syntheses were carried out in form of automated oligonucleotide synthesis. In such automated syntheses, all reactions were performed in 25 mL sparged-bed reactors (herein also referred to as R-1), in which agitation was achieved by bubbling N₂ gas through the reactor.

Alternatively, if indicated specifically, oligonucleotide syntheses were performed manually, in a 1 L stirred-bed reactor equipped with a mechanical stirrer (herein also referred to as R-2).

All reactor types were equipped with filters, frits, or membranes of appropriate pore sizes for retaining the resin in the reactor and draining solvents and reagents.

### Starting resins (solid supports)

Two sorts of commercially available DMT-protected polystyrene starting resins were used, unless indicated differently:
- resins to which the 5'-O-DMT-protected starting nucleoside moieties (i.e. the 3'-terminal nucleoside subunits of the oligonucleotide strands to be synthesized) were linked via a succinate-type linker (ester bond with the 3'-hydroxyl moiety of a nucleoside moiety and amide bond with an amine moiety at the resin); or
- resins carrying DMT-protected UnyLinker^{™} moieties (i.e. not already carrying the 3'-terminal nucleoside subunits). In these cases, the 3'-terminal nucleoside subunits were added via a coupling cycle as summarized in Table 1 below.

### Preparation of specific solutions

### General Protocol A (GP-A): Preparation of phosphoramidite solutions

The 5'-O-DMT protected nucleoside phosphoramidite derivatives with optional 2'-modifications (e.g. 2'-deoxy, 2'-fluoro or 2'-O-methyl, i.e. 2'-methoxy) of the benzoyl protected adenosine (A^{Bz}), benzoyl protected cytidine (C^{Bz}) [or acetyl protected cytidine (C^{Ac}), if indicated specifically], isobutyryl protected guanosine (G^{iBu}), thymidine (T), and uridine (U), were typically dissolved at a concentration of 0.2 M in dry acetonitrile or in a mixture of dry acetonitrile and N,N-dimethylformamide. Molecular sieves (3 Å) were added and the headspace was flushed with nitrogen. The solution was then dried for a minimum of 12 h.

### General Protocol B (GP-B): Preparation of a liquid composition C

The liquid compositions C were typically prepared by dissolving the protic acid in a mixture of the indicated solvent, the at least one alcohol, in which one or more hydroxyl groups are covalently bonded directly to an optionally substituted phenyl moiety, and, where applicable, an additional carbocation scavenger.

### Standard coupling cycle

Unless indicated differently, all syntheses were performed using 3'-phosphoramidite nucleosides according to the desired sequence from 3' to 5' direction.

The syntheses were typically executed at a scale of 0.1 mmol based on the resin weight and substitution (approx. 0.25-0.35 mmol/g).

Prior to the first coupling cycle, the desired amount (according to the desired scale of the synthesis) of the starting resin was transferred to the reactor and washed with acetonitrile (ACN, 5-10 mL/g) for 2 min. For each of the nucleoside phosphoramidites to be added, one coupling cycle according to the following Table 1 was carried out, unless indicated differently.

**Table 1: Typical coupling cycle for the synthesis of oligonucleotides.**

| **step** | **repetitions^{a)}** | **volume per mass resin^{b)}** | **time per repetition^{c)}** |
|---|---|---|---|
| detritylation | 1-8 | 6.28-25.1 mL/g | 5-20 min |
| wash | 1-7 | 21.1-221 mL/g | 1 min |
| | 1 | 8.6-13.1 mL/g | 2-3 min |
| coupling | 1 | 3.14 mL/g | 15 min |
| wash | 1 | 21.1-31.6 mL/g | 1 min |
| oxidation^{d)} | 1 | 12.6 mL/g | 5 min |
| wash^{d)} | 2 | 21.1-94.9 mL/g | 1 min |
| or | | | |
| sulfurization^{e)} | 1 | 12.6 mL/g | 5 min |
| wash^{e)} | 2 | 21.1 - 94.9 mL/g | 1 min |
| capping | 1 | 3.1-7.9 mL/g | 2 min |
| wash | 3-6 | 21.1-94.9 mL/g | 1 min |

| | | | |
|---|---|---|---|
| a) Each repetition equals one treatment of the support-bound growing oligonucleotide chains with the respective solution; a value of "1" indicates a single treatment; for each treatment a new batch of the respective solution was used; prior to each repetition, the solution of the previous repetition was drained. b) The mass of the starting resin was used as point of reference for all coupling cycles. The given volume refers to the summed up volume of all repetitions. c) Contact time between the growing oligonucleotide chains and the respective solution during each repetition. d) and e) Each coupling cycle comprised either the oxidation and wash step denoted with d) or the sulfurization and wash step denoted with e), but not both. | | | |

The detritylation step was performed using a liquid composition C prepared according to GP-B or a comparative detritylation cocktail, e.g. a detritylation cocktail from the prior art. Prior to detritylation, the resin was typically washed with the solvent of the respective detritylation cocktail, e.g. DCM or toluene.

The coupling step was typically performed using a phosphoramidite solution prepared according to GP-A, to which 5-(ethylthio)-1H-tetrazole activator (ETT, 0.5 M) had been added.

The oxidation step was typically performed using a solution of iodine (50 mM) in a mixture of water and pyridine (1:9, v/v).

The sulfurization step was typically performed using a solution of xanthane hydride (0.2 M) in pyridine or using a solution of xanthane hydride (0.1 M) in a mixture of acetonitrile and pyridine (2:3, v/v).

The capping step was typically performed using a mixture (1:1, v/v) of Cap A (20 vol-% acetic anhydride in acetonitrile) and Cap B (*N*-methylimidazole, 2,6-lutidine, acetonitrile, 20:30:50, v/v/v).

The wash steps were typically performed using acetonitrile. Only prior to detritylation, DCM (e.g. when using DCM as solvent of the detritylation cocktail) or toluene (e.g. when using toluene or anisole as solvent for the detritylation cocktail) were used for the wash steps.

Unless indicated differently, each repetition of each step of a typical coupling cycle comprised contacting the solid support carrying the growing oligonucleotide chains with the respective solution (e.g. a detritylation cocktail for detritylation steps or neat solvent for wash steps) for the indicated time under stirring (stirred-bed reactor) or shaking (syringe reactor) or agitating by nitrogen bubbling (sparged-bed reactor), followed by draining of the respective solution through a filter or frit or membrane of appropriate pore size, so as to retain the support-bound growing oligonucleotides inside the reactor.

In the following Examples, the number of repetitions of each detritylation step as well as the contact time with the detritylation cocktail per repetition is indicated specifically in tabular form. For example, two repetitions with 10 min contact time each would be referred to as 2 × 10 min or 10 + 10 min.

Unless indicated differently, syntheses were performed at 22°C.

The coupling cycles were repeated according to the sequence using the appropriate nucleoside phosphoramidites, unless indicated differently. After completion of all coupling cycles, the final 5'-O-DMT detritylation was performed using the same procedure that was used in the previous cycles including the wash step. Subsequently the cyanoethyl-protected phosphate triester or thiophosphate triester linkage groups were deprotected by treatment with diethylamine (DEA) in acetonitrile, unless indicated differently. Alternatively, the cyanoethyl protecting groups could be removed during resin cleavage by treatment with aqueous ammonium hydroxide solution. The resin was then dried under reduced pressure for a minimum of 16 h.

The resin carrying the fully-assembled oligonucleotides was weighed, and a trial cleavage and deprotection of the sample was performed. For this purpose, typically, 10-50 mg dried resin was weighed into a suitable reaction vessel (e.g. a 2 mL PP-tube). Aqueous ammonium hydroxide solution (i.e. aqueous ammonia solution, 25-28 wt-%, 1-2 mL) was added. The tube was closed, sealed, shaken, and heated to 45-55°C for 2-20 h, depending on length and sequence of the oligonucleotide. The supernatant was typically removed using a syringe and transferred to a second tube (2-5 mL). The ammonia was typically removed using a vacuum centrifuge or with a slight stream of nitrogen gas. The solution (containing the support-cleaved oligonucleotides) was then diluted with H₂O to the desired concentration, followed by analysis via reversed phase HPLC or HPLC-MS.

### 5. Target oligonucleotides

Example 1:
   5'-mUmUmUmUfA-3'
Example 2:
   5'-mAsfCsmAfAfAfAmGFCmMAfAmAmAmCfAmGfGmUfCmUmAmGsmAsmA-3'

The following notation was used to denote the nucleoside subunits:
A = adenosine,
dA = 2'-deoxyadenosine,
G = guanosine,
dG = 2'-deoxyguanosine,
C = cytidine,
dC = 2'-deoxycytidine,
T = thymidine (2'-deoxy and not ribothymidine),
U = uridine,
"f" indicates that the respective nucleoside moiety (denoted to the right of the letter "f") bears a 2'-fluoro (i.e. 2'-F) substituent (e.g. fA for adenosine with 2'-F modification),
"m" indicates that the respective nucleoside moiety (denoted to the right of the letter "m") bears a 2'-methoxy (i.e. 2'-OMe) substituent (e.g. mU for uridine with 2'-OMe modification).

Unless indicated differently, all internucleosidic linkage groups are phosphodiester linkage groups. The letter "s" to the right of a nucleoside symbol indicates that the internucleosidic linkage group in 3'-position of the respective nucleoside-subunit is a phosphorothioate linkage group instead of a phosphodiester linkage group (e.g. fAsmC for a phosphorothioate linkage group interconnecting the 3'-hydroxyl moiety of 2'fluoroadenosine and the 5'-hydroxyl moiety of 2'-methoxycytidine).

Herein, unless indicated differently, all oligonucleotides are denoted in 5' to 3' direction (i.e. the nucleoside subunit denoted to the very left is the 5'-terminal nucleoside subunit).

### Example 1: Synthesis of 5'-mUmUmUmUfA-3' using different detritylation conditions

The target oligonucleotide 5'-mUmUmUmUfA-3' was synthesized according to the general methods laid out above with an oxidation step in each coupling cycle, and with varying detritylation conditions. The syntheses and the results are compiled in the following Table E-1.

**Table E-1: Synthesis of 5'-mUmUmUmUfA-3' using different detritylation conditions**

| **synthesis^{a)}** | **detritylation cocktail^{b)}** | **detritylation: number of repetitions × contact time per repetition** | **purity** | **yield** | **degree of depurination** |
|---|---|---|---|---|---|
| 1.1^{e)} | DCA (1.218 M) in toluene | 2 × 10 min | 88 % | 96 % | 0.38 % |
| 1.2^{e)} | DCA (0.243 M) in toluene | 2 × 10 min | 89 % | 43 % | 0.11 % |
| 1.3^{d)} | DCA (0.243 M) in toluene + *m*-cresol (2.867 M) | 2 × 10 min | 93 % | 75 % | 0.10 % |
| 1.4^{e)} | DCA (0.243 M) in toluene + ethanol (2.867 M) | 2 × 10 min | n.d.^{c)} | n.d.^{c)} | n.d.^{c)} |
| 1.5^{d)} | TFA (0.092 M) and 4-CYP (0.096 M) in anisole + *m*-cresol (2.867 M) | 2 × 10 min | 92 % | 84 % | 0.09 % |
| 1.6^{e)} | TFA (0.092 M) and 4-CYP (0.096 M) in anisole | 2 × 10 min | 69% | 36% | 0.10% |
| 1.7^{d)} | TFA (0.092 M) and 4-CYP (0.096 M) in anisole + 4-methoxyphenol (2.867 M) | 2 × 10 min | 92 % | 87 % | 0.12 % |
| 1.8^{d)} | TFA (0.092 M) and 4-CYP (0.096 M) in toluene + *m*-cresol (2.867 M) | 2 × 10 min | 93 % | 76 % | 0.13 % |
| 1.9^{d)} | TFA ( 0.092 M) and 4-CYP (0.096 M) in toluene + phenol (2.867 M) | 2 × 10 min | 92 % | 85 % | 0.20 % |
| 1.10^{d)} | MSA (0.062 M) in toluene + *m*-cresol (2.867 M) | 2 × 10 min | 92 % | 83 % | 0.12 % |
| 1.11^{d)} | 4-CIPy-HCl (0.267 M) in toluene + *m*-cresol (2.867 M) | 2 × 10 min | 86 % | 84 % | 0.10 % |
| 1.12^{d)} | DCA (0.243 M) in dichloromethane + *m*-cresol (2.867 M) | 2 × 10 min | 93% | 74% | 0.10% |

| | | | | | |
|---|---|---|---|---|---|
| Abbreviations: DCA = dichloroacetic acid, MSA = methanesulfonic acid, 4-CIPy-HCl = 4-chloropyridinium hydrochloride (preformed salt), TFA = trifluoroacetic acid, 4-CYP = 4-cyanopyridine. a) Starting resin: polystyrene resin carrying DMT-protected UnyLinker^{™} moieties; Scale: 0.10 mmol (i.e. 0.10 mmol of 3'-nucleoside subunits at resin); Reactor: automated synthesis in a sparged-bed reactor (R-1). b) Detritylation volume: 5.0 mL / 0.10 mmol starting material (i.e. 0.10 mmol of DMT-protected OH-groups on the starting resin) per detritylation repetition. c) n.d. = not determined, since no product formation was observed at all. d) According to the invention. e) Comparative. | | | | | |

Due to the stability of abasic sites in 2'-F / 2'-OMe oligonucleotides during ammonia treatment, the only noteworthy depurination-derived side product was a compound differing from the target oligonucleotide 5'-mUmUmUmUfA-3' only in that an abasic site was located at the 3'-terminal fA-nucleoside subunit (i.e. the adenine nucleobase was substituted for a hydroxyl group). Thus, the degree of depurination was determined by dividing the area of (i.e. underneath) the peak of said single depurination-derived side product by the area of (i.e. underneath) the peak of the target oligonucleotide, followed by multiplication with 100 %, to arrive at a value in percent (%).

The syntheses summarized in Table E-1 differ only with regards to the detritylation cocktail that was utilized. Synthesis 1.1 of Table E-1 utilized a 1.218 M solution of DCA in toluene (i.e. 10 vol-% DCA in toluene) which is a standard detritylation cocktail for industrial synthesis. When reducing the concentration of DCA to 0.243 M (i.e. 2 vol-%, synthesis 1.2), a decrease of the degree of depurination from 0.38 % to 0.11 % was observed, which was, however, accompanied by a reduction of the yield from 96 % to 43 % (cf. syntheses 1.1. and 1.2). The degree of depurination could be further reduced to 0.10 % by addition of *m*-cresol (2.867 M, synthesis 1.3), which also led to an increase of the yield from 43 % to 75 % (cf. syntheses 1.2 and 1.3). Replacing *m*-cresol for the same concentration of ethanol (synthesis 1.4) completely prevented product formation. Thus, in terms of reducing the degree of depurination while achieving an acceptable yield, synthesis 1.3 according to the invention outperformed the comparative syntheses 1.1, 1.2, and 1.4.

The detritylation cocktail of synthesis 1.5 differs from the detritylation cocktail of synthesis 1.3 in that a combination of TFA and 4-cyanopyridine was used as protic acid instead of DCA and in that the solvent was anisole instead of toluene. The degree of depurination (0.09 %) was again lower than for the industrial standard protocol of synthesis 1.1 (0.38 %) (cf. entries 1.5 and 1.1). In the absence of *m*-cresol (synthesis 1.6) an unacceptably low yield of 36 % instead of 84 % was obtained (cf. syntheses 1.5 and 1.6).

The detritylation cocktail of synthesis 1.7 differs from the detritylation cocktail of synthesis 1.5 in that 4-methoxyphenol was used as the alcohol instead of *m*-cresol. The detritylation cocktail of synthesis 1.8 differs from the detritylation cocktail of synthesis 1.5 in that toluene was used as solvent instead of anisole. The detritylation cocktail of synthesis 1.9 differs from the detritylation cocktail of synthesis 1.8 in that phenol was used as the alcohol instead of *m*-cresol. In syntheses 1.7, 1.8, and 1.9, the degree of depurination was lower than for the industrial standard protocol (cf. syntheses 1.1, 1,7, 1.8, and 1.9) showing that the method of the invention allows for variation with regards to the alcohol and the solvent.

Besides DCA (an exemplary carboxylic acid, synthesis 1.3) and the combination of TFA (an exemplary carboxylic acid) and 4-cyanopyridine (syntheses 1.5, 1.7, 1.8, and 1.9), methanesulfonic acid (MSA, an exemplary sulfonic acid) and a preformed salt of hydrochloric acid (an exemplary mineral acid) and 4-chloropyridine were used as protic acids of the detritylation cocktail (syntheses 1.10 and 1.11). In both cases, the degree of depurination was reduced as compared to the industrial standard protocol (cf. syntheses 1.1, 1.10, and 1.11). These results show that the method of the invention allows for variation with regards to the protic acid.

**Example 2:** Synthesis of 5'-mAsfCsmAfAfAfAmGFCmAfAmAmAmCfAmGfGmUfCmUmAmGsmAsmA-3' 5'-mAsfCsmAfAfAfAmGfCmAfAmAmAmCfAmGfGmUfCmUmAmGsmAsmA-3' (target oligonucleotide) was synthesized according to the general methods laid out above. When the nucleoside moiety to be added should be interconnected to the growing oligonucleotide via a phosphodiester linkage group, an oxidation step was performed. When the nucleoside moiety to be added should be interconnected to the growing oligonucleotide via a phosphorothioate linkage group, a sulfurization step was performed.

Starting resin: polystyrene resin with 5'-O-DMT protected 3'-terminal nucleoside subunit (i.e. 5'-O-DMT-A) bonded via succinate-type linker;
Scale: 0.10 mmol (i.e. 0.10 mmol of 3'-nucleoside subunits at resin);
Reactor: automated synthesis in a sparged-bed reactor (R-1);
Detritylation cocktail: TFA (0.7 vol-%, 0.092 M) and 4-CYP (0.9 wt-%, 0.096 M) in toluene + *m*-cresol (30 vol-%, 2.87 M)
Detritylation volume: 5.0 mL / 0.10 mmol starting material (i.e. 0.10 mmol of DMT-protected OH-groups on the starting resin) per detritylation repetition.

The target oligonucleotide was obtained in a yield of 87 % and with a purity of 76 %, indicating that the method of the invention is suited for the synthesis of longer oligonucleotides.

## Claims

1. A method for the solid-phase synthesis of a target oligonucleotide O^{T} comprising a step (b) of incubating a nucleoside or oligonucleotide, which is covalently linked to a solid support and comprises a backbone hydroxyl moiety protected by a di(*p*-methoxyphenyl)phenylmethyl protecting group PG-0 with a deprotection mixture M-b, thereby cleaving the protecting group PG-0 from the nucleoside or oligonucleotide, wherein said deprotection mixture M-b is a liquid composition C comprising a solvent, a protic acid having a pKa equal to or smaller than 4, and at least one alcohol, in which one or more hydroxyl groups are covalently bonded directly to an optionally substituted phenyl moiety, wherein
- said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof; and
- each of said at least one alcohol, in which one or more hydroxyl groups are covalently bonded directly to an optionally substituted phenyl moiety, is independently an alcohol of the following Formula D: 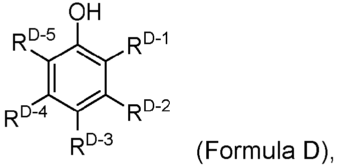 wherein in Formula D:
R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN.

2. The method according to claim 1, wherein the target oligonucleotide O^{T} comprises a first cycle oligonucleotide O-1, and the method comprises the following step (a) and a first coupling cycle comprising the following steps (b) to (e):
(a) providing a component C-0 selected from the group consisting of a nucleoside and an oligonucleotide, wherein the component C-0 is covalently linked to a solid support and comprises a backbone hydroxyl moiety protected by a di(p-methoxyphenyl)phenylmethyl protecting group PG-0;
(b) incubating the component C-0 of step (a) with a deprotection mixture M-b, thereby cleaving the protecting group PG-0 from the component C-0, so as to obtain a component C-0^{#} having a free backbone hydroxyl group;
(c) providing a building block B-1 selected from the group consisting of a nucleoside and an oligonucleotide, wherein the building block B-1 comprises a backbone hydroxyl moiety protected by a di(p-methoxyphenyl)phenylmethyl protecting group PG-1 and a phosphorus moiety covalently bonded via its phosphorus atom to an oxygen atom of the backbone of the building block B-1;
(d) reacting the component C-0^{#} of step (b) with the building block B-1 of step (c) under conditions suitable to form a covalent bond between said free backbone hydroxyl group of the component C-0^{#} and the phosphorus atom of said phosphorus moiety of the building block B-1, thereby obtaining a first cycle oligonucleotide O-1;
(e) optionally, incubating the first cycle oligonucleotide O-1 obtained in step (d) with an oxidizing or sulfurizing agent, thereby converting any P (III) atoms within said first cycle oligonucleotide O-1 to P (V) atoms;
wherein in step (b), said deprotection mixture M-b is a liquid composition C as defined in claim 1.

3. The method according to claim 2 wherein the target oligonucleotide O^{T} comprises a n-th cycle oligonucleotide O-n, and the method further comprises performing (n-1) iterations of a coupling cycle comprising the following steps (b') to (e'), wherein n is an integer in the range of 2 to 99, which denotes the total number of coupling cycles performed to obtain the n-th cycle oligonucleotide O-n, and each individual coupling cycle comprising the following steps (b') to (e') is identified by a serial number x, which runs in steps of 1 from 2 to n:
(b') incubating the (x-1)-th cycle oligonucleotide O-(x-1) obtained in the previous coupling cycle with a deprotection mixture M-b', thereby cleaving the di(p-methoxyphenyl)phenylmethyl protecting group PG-(x-1) from the (x-1)-th cycle oligonucleotide O-(x-1), so as to obtain a (x-1)-th cycle oligonucleotide (O-(x-1))^{#} having a free backbone hydroxyl group;
(c') providing a building block B-x selected from the group consisting of a nucleoside and an oligonucleotide, wherein the building block B-x comprises a backbone hydroxyl moiety protected by a di(p-methoxyphenyl)phenylmethyl protecting group PG-x and a phosphorus moiety covalently bonded via its phosphorus atom to an oxygen atom of the backbone of the building block B-x;
(d') reacting the (x-1)-th cycle oligonucleotide (O-(x-1))^{#} obtained in step (b') with the building block B-x of step (c') under conditions suitable to form a covalent bond between said free backbone hydroxyl group of the (x-1)-th cycle oligonucleotide (O-(x-1))^{#} and the phosphorus atom of said phosphorus moiety of the building block B-x, thereby obtaining a x-th cycle oligonucleotide O-x;
(e') optionally, incubating the x-th cycle oligonucleotide O-x obtained in step (d') with an oxidizing or sulfurizing agent, thereby converting any P (III) atoms within said x-th cycle oligonucleotide O-x to P (V) atoms;
wherein in at least one iteration of step (b'), said deprotection mixture M-b' is a liquid composition C as defined in claim 1.

4. The method according to any one of claims 2 and 3, wherein:
- the phosphorus moiety of the building block B-1 and each building block B-x is independently selected from the group consisting of a phosphoramidite moiety and a H-phosphonate monoester moiety;
- in each coupling cycle, in which said phosphorus moiety of the building block B-1 or the building block B-x is a phosphoramidite moiety, step (e) or (e') is carried out; and
- at least in the final coupling cycle, step (e) or (e') is carried out.

5. The method according to any one of claims 1 to 4, wherein said nucleoside or oligonucleotide, which is covalently linked to a solid support, of claim 1 and the component C-0 of claim 2 is a compound of the following Formula I: 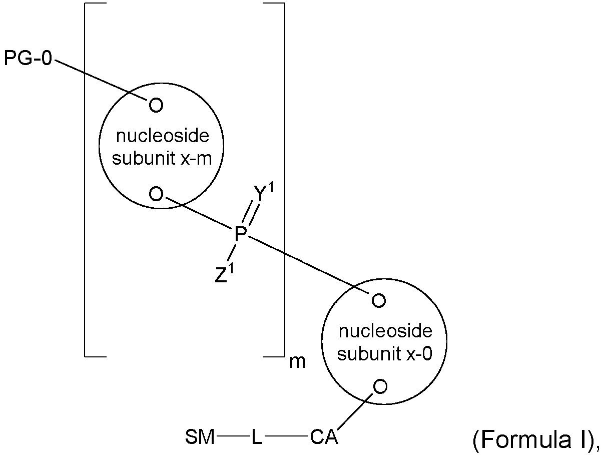 wherein in Formula I:
each oxygen atom (O) depicted within each nucleoside subunit x-0 to x-m represents the oxygen atom of a hydroxyl moiety of the respective nucleoside subunit;
each of the nucleoside subunits x-0 to x-m may be the same or different;
PG-0 is a di(p-methoxyphenyl)phenylmethyl protecting group;
m is an integer equal to or larger than 0;
Y¹ is selected independently for each repetitive unit m from the group consisting of O and S;
Z¹ is selected independently for each repetitive unit m from the group consisting of O-R^{z-1} and S-R^{z-1};
R^{z-1} is a protecting group, which may be the same or different for each repetitive unit m;
CA is a capping moiety or a covalent chemical bond;
L is a linker moiety or a covalent chemical bond; and
SM is a solid support.

6. The method according to any one of claims 2 to 5, wherein each of the building blocks B-1 and B-x is a compound of the following Formula II-1: 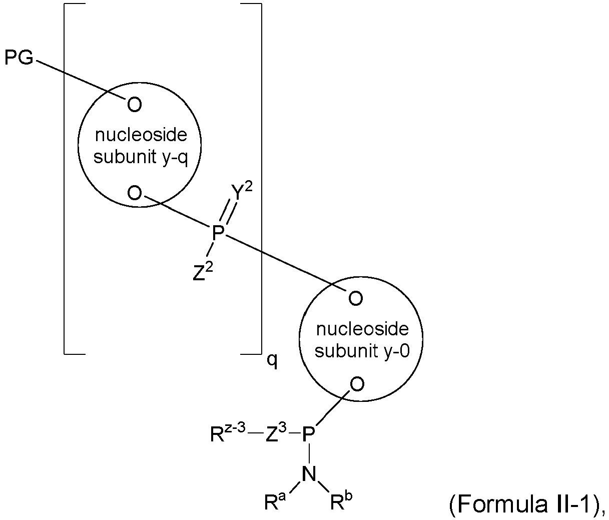 wherein in Formula II-1:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different;
PG is the protecting group PG-1 or PG-x, and is a di(*p*-methoxyphenyl)phenylmethyl protecting group;
q is an integer equal to or larger than 0;
Y² is selected independently for each repetitive unit q from the group consisting of O and S;
Z² is selected independently for each repetitive unit q from the group consisting of O-R^{z-2}, S-R^{z-2};
R^{z-2} is a protecting group, which may be the same or different for each repetitive unit q;
Z³ is selected from the group consisting of O and S; and
R^{z-3} is a protecting group;
each of R^{a} and R^{b} is independently a C₁-C₆-alkyl group, wherein R^{a} and R^{b} may be the same or different and may also bond to each other to form a 5-membered or 6-membered aliphatic cyclic amine moiety together with the nitrogen atom to which R^{a} and R^{b} are bonded;
and wherein step (e) or step (e') is carried out in each coupling cycle.

7. The method according to any one of claims 2 to 6, wherein
- the first coupling cycle further comprises a step (f) of reacting free hydroxyl groups with a blocking agent, wherein step (f) is carried out after step (d) or after step (e); and/or
- at least one iteration of the (n-1) iterations of the coupling cycle comprising steps (b') to (e') further comprises a step (f') of reacting free hydroxyl groups with a blocking agent, wherein step (f') is carried out after step (d') or after step (e').

8. The method according to any one of claims 2 and 4 to 7, wherein
- the method further comprises a step (g) of incubating the first cycle oligonucleotide O-1 with a deprotection mixture M-g, thereby cleaving the protecting group PG-1 from the first cycle oligonucleotide O-1, so as to obtain a first cycle oligonucleotide (O-1)^{#} having a free backbone hydroxyl group; and/or
- the method further comprises a step (h) of cleaving the first cycle oligonucleotide O-1 or (O-1)^{#} from the solid support;
and wherein, if both steps (g) and (h) are performed, they may be performed in any order.

9. The method according to any one of claims 3 to 7, wherein
- the method further comprises a step (g') of incubating the n-th cycle oligonucleotide O-n with a deprotection mixture M-g', thereby cleaving the protecting group PG-n from the n-th cycle oligonucleotide O-n, so as to obtain a n-th cycle oligonucleotide (O-n)^{#} having a free backbone hydroxyl group; and/or
- the method further comprises a step (h') of cleaving the n-th cycle oligonucleotide O-n or (O-n)^{#} from the solid support;
and wherein, if both steps (g') and (h') are performed, they may be performed in any order.

10. The method according to any one of claims 1 to 9, wherein at least steps (b) and (b') are carried out in a batch reactor or wherein at least steps (b) and (b') are carried out in a column reactor and the flow rate of the liquid composition C through the column reactor is below 300 cm/h.

11. The method according to any one of claims 1 to 10, wherein
- the backbone hydroxyl moiety protected by said protecting group PG-0 is part of a nucleoside moiety comprising a purine type nucleobase; and
- in at least one iteration of the coupling cycle comprising steps (b') to (e'), in which said protecting group PG-(x-1) is part of a nucleoside moiety comprising a purine type nucleobase, the deprotection mixture M-b' is a liquid composition C.

12. The method according to any one of claims 1 to 11, wherein the synthesis is carried out on a scale of at least 100 mmol of the target oligonucleotide O^{T}.

13. The method according to any one of claims 1 to 12, wherein the liquid composition C comprises said at least one alcohol according to Formula D in a molar concentration of 0.45-5.60 mol/L.

14. The method according to any one of claims 1 to 13, wherein said solvent comprised in the liquid composition C is a non-halogenated aprotic solvent.

15. The method according to any one of claims 8 and 10 to 14, wherein the method further comprises a step (i) of modifying the first cycle oligonucleotide O-1 or (O-1)^{#}.

16. The method according to any one of claims 9 to 14, wherein the method further comprises a step (i') of modifying the n-th cycle oligonucleotide O-n or (O-n)^{#}.

17. A composition comprising
- an oligonucleotide which is covalently linked to a solid support and comprises a hydroxyl moiety protected by a di(p-methoxyphenyl)phenylmethyl protecting group, and
- a liquid composition C comprising a solvent, a protic acid having a pKa equal to or smaller than 4, and at least one alcohol, in which one or more hydroxyl groups are covalently bonded directly to an optionally substituted phenyl moiety,
wherein
- said protic acid comprised in the liquid composition C is selected from the group consisting of a carboxylic acid, a sulfonic acid, a mineral acid, a protonated aliphatic, aromatic or heteroaromatic amine, whose protonated form has a pKa in the range of 1-4, and mixtures thereof; and
- each of said at least one alcohol, in which one or more hydroxyl groups are covalently bonded directly to an optionally substituted phenyl moiety, is independently an alcohol of the following Formula D: 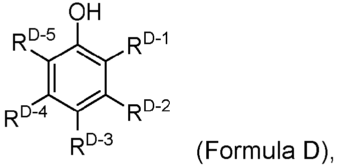 wherein in Formula D:
R^{D-1}, R^{D-2}, R^{D-3}, R^{D-4}, and R^{D-5} are independently of each other selected from the group consisting of H, OH, a C₁-C₆-alkyl group, O(C₁-C₆-alkyl), C(O)(C₁-C₆-alkyl), C(O)O(C₁-C₆-alkyl), F, Cl, Br, I, and CN;
preferably wherein the composition and/or one or more components are defined as in one or more of the preceding claims,
in particular wherein the concentration of said at least one alcohol of Formula D is defined as in claim 13, and/or said solvent is defined as in claim 14.

## Patentansprüche

1. Verfahren zur Festphasensynthese eines Zieloligonukleotids O^{T}, umfassend einen Schritt (b) des Inkubierens eines Nukleosids oder Oligonukleotids, das kovalent an einen festen Träger gebunden ist und einen Grundgerüst-Hydroxylanteil umfasst, der durch eine Di(p-methoxyphenyl)phenylmethyl-Schutzgruppe PG-0 geschützt ist, mit einer Entschützungsmischung M-b, wodurch die Schutzgruppe PG-0 von dem Nukleosid oder Oligonukleotid abgespalten wird, wobei die Entschützungsmischung M-b eine flüssige Zusammensetzung C ist, die ein Lösungsmittel, eine Protonensäure mit einem pKa gleich oder kleiner als 4 und mindestens einen Alkohol umfasst, in dem eine oder mehrere Hydroxylgruppen kovalent direkt an einen gegebenenfalls substituierten Phenylanteil gebunden sind, wobei
- die in der flüssigen Zusammensetzung C enthaltene Protonensäure ausgewählt ist aus der Gruppe bestehend aus einer Carbonsäure, einer Sulfonsäure, einer Mineralsäure, einem protonierten aliphatischen, aromatischen oder heteroaromatischen Amin, dessen protonierte Form einen pKa im Bereich von 1-4 aufweist, und Mischungen davon; und
- jeder des mindestens einen Alkohols, in dem eine oder mehrere Hydroxylgruppen kovalent direkt an einen gegebenenfalls substituierten Phenylanteil gebunden sind, unabhängig ein Alkohol der folgenden Formel D ist: 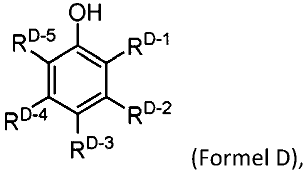
wobei in Formel D:
R^{D-1}, R^{D-2}, R^{D-3}, R^{D-4} und R^{D-5} unabhängig voneinander ausgewählt sind aus der Gruppe bestehend aus H, OH, einer C₁-C₆-Alkylgruppe, O(C₁-C₆-Alkyl), C(O)(C₁-C₆-Alkyl), C(O)O(C₁-C₆-Alkyl), F, Cl, Br, I und CN.

2. Verfahren nach Anspruch 1, wobei das Zieloligonukleotid O^{T} ein Oligonukleotid O-1 des ersten Zyklus umfasst, und das Verfahren den folgenden Schritt (a) und einen ersten Kopplungszyklus umfasst, der die folgenden Schritte (b) bis (e) umfasst:
(a) Bereitstellen einer Komponente C-0 ausgewählt aus der Gruppe bestehend aus einem Nukleosid und einem Oligonukleotid, wobei die Komponente C-0 kovalent an einen festen Träger gebunden ist und einen Grundgerüst-Hydroxylanteil umfasst, der durch eine Di(p-methoxyphenyl)phenylmethyl-Schutzgruppe PG-0 geschützt ist;
(b) Inkubieren der Komponente C-0 aus Schritt (a) mit einer Entschützungsmischung M-b, wodurch die Schutzgruppe PG-0 von der Komponente C-0 abgespalten wird, um eine Komponente C-0^{#} mit einer freien Grundgerüst-Hydroxylgruppe zu erhalten;
(c) Bereitstellen eines Bausteins B-1 ausgewählt aus der Gruppe bestehend aus einem Nukleosid und einem Oligonukleotid, wobei der Baustein B-1 einen durch eine Di(p-methoxyphenyl)phenylmethyl-Schutzgruppe PG-1 geschützten Grundgerüst-Hydroxylanteil und einen über sein Phosphoratom kovalent an ein Sauerstoffatom des Grundgerüsts des Bausteins B-1 gebundenen Phosphoranteil umfasst;
(d) Umsetzen der Komponente C-0^{#} von Schritt (b) mit dem Baustein B-1 von Schritt (c) unter Bedingungen, die geeignet sind, um eine kovalente Bindung zwischen der freien Grundgerüst-Hydroxylgruppe der Komponente C-0^{#} und dem Phosphoratom des Phosphoranteils des Bausteins B-1 zu bilden, wodurch ein Oligonukleotid O-1 des ersten Zyklus erhalten wird;
(e) gegebenenfalls Inkubieren des in Schritt (d) erhaltenen Oligonukleotids O-1 des ersten Zyklus mit einem Oxidations- oder Sulfurierungsmittel, wodurch beliebige P(III)-Atome innerhalb des Oligonukleotids O-1 des ersten Zyklus in P(V)-Atome umgewandelt werden;
wobei in Schritt (b) die Entschützungsmischung M-b eine flüssige Zusammensetzung C wie in Anspruch 1 definiert ist.

3. Verfahren nach Anspruch 2, wobei das Zieloligonukleotid O^{T} ein Oligonukleotid O-n des n-ten Zyklus umfasst, und das Verfahren ferner Durchführen von (n-1) Iterationen eines Kopplungszyklus umfasst, der die folgenden Schritte (b') bis (e') umfasst, wobei n eine ganze Zahl im Bereich von 2 bis 99 ist, die die Gesamtzahl der Kopplungszyklen angibt, die durchgeführt werden, um das Oligonukleotid O-n des n-ten Zyklus zu erhalten, und jeder individuelle Kopplungszyklus, der die folgenden Schritte (b') bis (e') umfasst, durch eine Seriennummer x identifiziert wird, die in Schritten von 1 von 2 bis n verläuft:
(b') Inkubieren des im vorherigen Kopplungszyklus erhaltenen Oligonukleotids O-(x-1) des (x-1)-ten Zyklus mit einer Entschützungsmischung M-b', wodurch die Di(p-methoxyphenyl)phenylmethyl-Schutzgruppe PG-(x-1) von dem Oligonukleotid O-(x-1) des (x-1)-ten Zyklus abgespalten wird, um ein Oligonukleotid (O-(x-1))^{#} des (x-1)-ten Zyklus mit einer freien Grundgerüst-Hydroxylgruppe zu erhalten;
(c') Bereitstellen eines Bausteins B-x ausgewählt aus der Gruppe bestehend aus einem Nukleosid und einem Oligonukleotid, wobei der Baustein B-x einen durch eine Di(p-methoxyphenyl)phenylmethyl-Schutzgruppe PG-x geschützten Grundgerüst-Hydroxylanteil und einen über sein Phosphoratom kovalent an ein Sauerstoffatom des Grundgerüsts des Bausteins B-x gebundenen Phosphoranteil umfasst;
(d') Umsetzen des in Schritt (b') erhaltenen Oligonukleotids (O-(x-1))^{#} des (x-1)-ten Zyklus mit dem Baustein B-x von Schritt (c') unter Bedingungen, die geeignet sind, um eine kovalente Bindung zwischen der freien Grundgerüst-Hydroxylgruppe des Oligonukleotids (O-(x-1)^{#}) des (x-1)-ten Zyklus und dem Phosphoratom des Phosphoranteils des Bausteins B-x zu bilden, wodurch ein Oligonukleotid O-x des x-ten Zyklus erhalten wird;
(e') gegebenenfalls Inkubieren des in Schritt (d') erhaltenen Oligonukleotids O-x des x-ten Zyklus mit einem Oxidations- oder Sulfurierungsmittel, wodurch beliebige P(III)-Atome innerhalb des Oligonukleotids O-x des x-ten Zyklus in P(V)-Atome umgewandelt werden;
wobei in mindestens einer Iteration von Schritt (b') die Entschützungsmischung M-b' eine flüssige Zusammensetzung C ist, wie sie in Anspruch 1 definiert ist.

4. Verfahren nach einem der Ansprüche 2 und 3, wobei:
- der Phosphoranteil des Bausteins B-1 und jedes Bausteins B-x unabhängig ausgewählt ist aus der Gruppe bestehend aus einem Phosphoramiditanteil und einem H-Phosphonatmonoesteranteil;
- in jedem Kopplungszyklus, in dem der Phosphoranteil des Bausteins B-1 oder des Bausteins B-x ein Phosphoramiditanteil ist, Schritt (e) oder (e') durchgeführt wird; und
- zumindest in dem letzten Kopplungszyklus Schritt (e) oder (e') durchgeführt wird.

5. Verfahren nach einem der Ansprüche 1 bis 4, wobei das Nukleosid oder Oligonukleotid, das kovalent an einen festen Träger gebunden ist, nach Anspruch 1 und die Komponente C-0 nach Anspruch 2 eine Verbindung der folgenden Formel I ist: 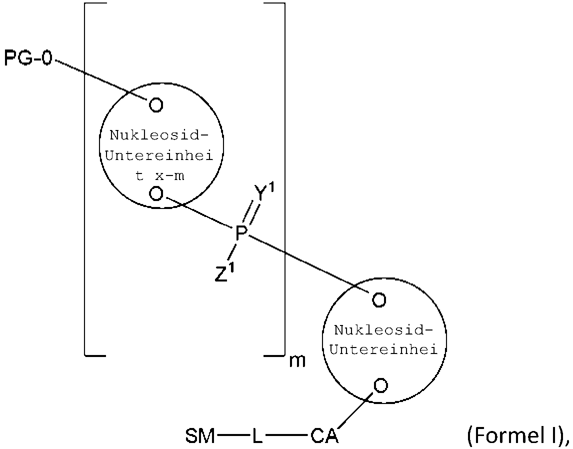 wobei in Formel I:
jedes Sauerstoffatom (O), das innerhalb jeder Nukleosiduntereinheit x-0 bis x-m dargestellt ist, für das Sauerstoffatom eines Hydroxylanteils der jeweiligen Nukleosiduntereinheit steht;
jede der Nukleosiduntereinheiten x-0 bis x-m gleich oder verschieden sein kann;
PG-0 eine Di(p-methoxyphenyl)phenylmethyl-Schutzgruppe ist;
m eine ganze Zahl gleich oder größer als 0 ist;
Y¹ unabhängig für jede sich wiederholende Einheit m aus der Gruppe bestehend aus O und S ausgewählt wird;
Z¹ unabhängig für jede sich wiederholende Einheit m aus der Gruppe bestehend aus O-R^{z-1} und S-R^{z-1} ausgewählt wird;
R^{z-1} eine Schutzgruppe ist, die für jede sich wiederholende Einheit m gleich oder verschieden sein kann;
CA ein Verkappungsanteil oder eine kovalente chemische Bindung ist;
L ein Linkeranteil oder eine kovalente chemische Bindung ist; und
SM in solider Träger ist.

6. Verfahren nach einem der Ansprüche 2 bis 5, wobei jeder der Bausteine B-1 und B-x eine Verbindung der folgenden Formel II-1 ist: 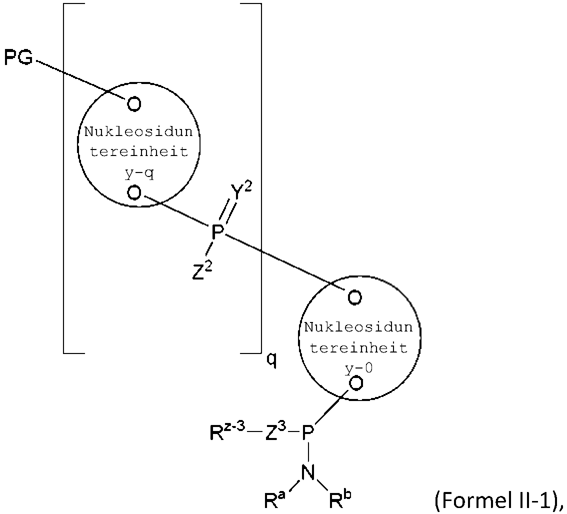 wobei in Formel II-1:
jedes Sauerstoffatom (O), das innerhalb jeder Nukleosiduntereinheit y-0 bis y-q dargestellt ist, für das Sauerstoffatom eines Hydroxylanteils der jeweiligen Nukleosiduntereinheit steht;
jede Nukleosiduntereinheit y-0 bis y-q gleich oder verschieden sein kann;
PG die Schutzgruppe PG-1 oder PG-x ist und eine Di(p-methoxyphenyl)phenylmethyl-Schutzgruppe ist;
q eine ganze Zahl gleich oder größer als 0 ist;
Y² unabhängig für jede sich wiederholende Einheit q aus der Gruppe bestehend aus O und S ausgewählt wird;
Z² unabhängig für jede sich wiederholende Einheit q aus der Gruppe bestehend aus O-R^{z-2}, S-R^{z-2} ausgewählt wird;
R^{z-2} eine Schutzgruppe ist, die für jede sich wiederholende Einheit q gleich oder verschieden sein kann;
Z³ ausgewählt ist aus der Gruppe bestehend aus O und S; und
R^{z-3} eine Schutzgruppe ist;
jedes von R^{a} und R^{b} unabhängig für eine C₁-C₆-Alkylgruppe steht, wobei R^{a} und R^{b} gleich oder verschieden sein können und auch aneinander binden können, um zusammen mit dem Stickstoffatom, an das R^{a} und R^{b} gebunden sind, einen 5-gliedrigen oder 6-gliedrigen aliphatischen cyclischen Aminrest zu bilden;
und wobei Schritt (e) oder Schritt (e') in jedem Kopplungszyklus durchgeführt wird.

7. Verfahren nach einem der Ansprüche 2 bis 6, wobei
- der erste Kopplungszyklus ferner einen Schritt (f) des Umsetzens freier Hydroxylgruppen mit einem Blockierungsmittel umfasst, wobei Schritt (f) nach Schritt (d) oder nach Schritt (e) durchgeführt wird; und/oder
- zumindest eine Iteration der (n-1) Iterationen des Kopplungszyklus, der die Schritte (b') bis (e') umfasst, ferner einen Schritt (f') des Umsetzens freier Hydroxylgruppen mit einem Blockierungsmittel umfasst, wobei Schritt (f') nach Schritt (d') oder nach Schritt (e') durchgeführt wird.

8. Verfahren nach einem der Ansprüche 2 und 4 bis 7, wobei
- das Verfahren ferner einen Schritt (g) des Inkubierens des Oligonukleotids O-1 des ersten Zyklus mit einer Entschützungsmischung M-g umfasst, wodurch die Schutzgruppe PG-1 von dem Oligonukleotid O-1 des ersten Zyklus abgespalten wird, um ein Oligonukleotid (O-1)^{#} des ersten Zyklus mit einer freien Grundgerüst-Hydroxylgruppe zu erhalten; und/oder
- das Verfahren ferner einen Schritt (h) des Abspaltens des Oligonukleotids O-1 oder (O-1)^{#} des ersten Zyklus von dem festen Träger umfasst;
und wobei, wenn beide Schritte (g) und (h) durchgeführt werden, sie in beliebiger Reihenfolge durchgeführt werden können.

9. Verfahren nach einem der Ansprüche 3 bis 7, wobei
- das Verfahren ferner einen Schritt (g') des Inkubierens des Oligonukleotids O-n des n-ten Zyklus mit einer Entschützungsmischung M-g' umfasst, wodurch die Schutzgruppe PG-n von dem Oligonukleotid O-n des n-ten Zyklus abgespalten wird, um ein Oligonukleotid (O-n)^{#} des n-ten Zyklus mit einer freien Grundgerüst-Hydroxylgruppe zu erhalten; und/oder
- das Verfahren ferner einen Schritt (h') des Abspaltens des Oligonukleotids O-n oder (O-n)^{#} des n-ten Zyklus von dem festen Träger umfasst;
und wobei, wenn beide Schritte (g') und (h') durchgeführt werden, sie in beliebiger Reihenfolge durchgeführt werden können.

10. Verfahren nach einem der Ansprüche 1 bis 9, wobei mindestens die Schritte (b) und (b') in einem Batch-Reaktor durchgeführt werden, oder wobei mindestens die Schritte (b) und (b') in einem Säulenreaktor durchgeführt werden und die Durchflussrate der flüssigen Zusammensetzung C durch den Säulenreaktor unter 300 cm/h liegt

11. Verfahren nach einem der Ansprüche 1 bis 10, wobei
- der durch die Schutzgruppe PG-0 geschützte Grundgerüst-Hydroxylanteil Teil eines Nukleosidanteils ist, der eine Nukleobase vom Purintyp umfasst; und
- in zumindest einer Iteration des Kopplungszyklus, der die Schritte (b') bis (e') umfasst, wobei die Schutzgruppe PG-(x-1) Teil eines Nukleosidanteils ist, der eine Nukleobase vom Purintyp umfasst, die Entschützungsmischung M-b' eine flüssige Zusammensetzung C ist.

12. Verfahren nach einem der Ansprüche 1 bis 11, wobei die Synthese im Maßstab von mindestens 100 mmol des Zieloligonukleotids O^{T} durchgeführt wird.

13. Verfahren nach einem der Ansprüche 1 bis 12, wobei die flüssige Zusammensetzung C den mindestens einen Alkohol gemäß Formel D in einer molaren Konzentration von 0,45-5,60 mol/l umfasst

14. Verfahren nach einem der Ansprüche 1 bis 13, wobei das in der flüssigen Zusammensetzung C enthaltene Lösungsmittel ein nicht-halogeniertes aprotisches Lösungsmittel ist.

15. Verfahren nach einem der Ansprüche 8 und 10 bis 14, wobei das Verfahren ferner einen Schritt (i) des Modifizierens des Oligonukleotids O-1 oder (O-1)^{#} des ersten Zyklus umfasst.

16. Verfahren nach einem der Ansprüche 9 bis 14, wobei das Verfahren ferner einen Schritt (i') des Modifizierens des Oligonukleotids O-n oder (O-n)^{#} des n-ten Zyklus umfasst.

17. Zusammensetzung, umfassend
- ein Oligonukleotid, das kovalent an einen festen Träger gebunden ist und einen Hydroxylanteil umfasst, der durch eine Di(p-methoxyphenyl)phenylmethyl-Schutzgruppe geschützt ist, und
- eine flüssige Zusammensetzung C, umfassend ein Lösungsmittel, eine Protonensäure mit einem pKa gleich oder kleiner als 4 und mindestens einen Alkohol, wobei eine oder mehrere Hydroxylgruppen kovalent direkt an einen gegebenenfalls substituierten Phenylanteil gebunden sind,
wobei
- die in der flüssigen Zusammensetzung C enthaltene Protonensäure ausgewählt ist aus der Gruppe bestehend aus einer Carbonsäure, einer Sulfonsäure, einer Mineralsäure, einem protonierten aliphatischen, aromatischen oder heteroaromatischen Amin, dessen protonierte Form einen pKa im Bereich von 1-4 aufweist, und Mischungen davon; und
- jeder des mindestens einen Alkohols, in dem eine oder mehrere Hydroxylgruppen kovalent direkt an einen gegebenenfalls substituierten Phenylanteil gebunden sind, unabhängig ein Alkohol der folgenden Formel D ist: 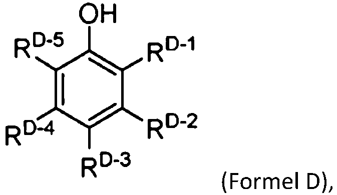
wobei in Formel D:
R^{D-1}, R^{D-2}, R^{D-3}, R^{D-4} und R^{D-5} unabhängig voneinander ausgewählt sind aus der Gruppe bestehend aus H, OH, einer C₁-C₆-Alkylgruppe, O(C₁-C₆-Alkyl), C(O)(C₁-C₆-Alkyl), C(O)O(C₁-C₆-Alkyl), F, Cl, Br, I und CN;
wobei vorzugsweise die Zusammensetzung und/oder eine oder mehrere Komponenten wie in einem oder mehreren der vorhergehenden Ansprüche definiert sind,
wobei insbesondere die Konzentration des mindestens einen Alkohols der Formel D wie in Anspruch 13 definiert ist und/oder das Lösungsmittel wie in Anspruch 14 definiert ist.

## Revendications

1. Procédé de synthèse en phase solide d'un oligonucléotide cible O^{T} comprenant une étape (b) d'incubation d'un nucléoside ou oligonucléotide, qui est lié de manière covalente à un support solide et comprend un groupement hydroxyle de squelette protégé par un groupe protecteur di(p-méthoxyphényl)phénylméthyle PG-0 avec un mélange de déprotection M-b, clivant ainsi le groupe protecteur PG-0 du nucléoside ou de l'oligonucléotide, ledit mélange de déprotection M-b étant une composition liquide C comprenant un solvant, un acide protique ayant un pKa inférieur ou égal à 4, et au moins un alcool, dans lequel un ou plusieurs groupes hydroxyle sont liés de façon covalente directement à un groupement phényle éventuellement substitué, dans lequel
- ledit acide protique compris dans la composition liquide C est choisi dans le groupe constitué par un acide carboxylique, un acide sulfonique, un acide minéral, une amine aliphatique, aromatique ou hétéroaromatique protonée, dont la forme protonée possède un pKa dans la plage de 1 à 4, et des mélanges correspondants ; et
- chacun dudit au moins un alcool, dans lequel un ou plusieurs groupes hydroxyle sont liés de façon covalente directement à un groupement phényle éventuellement substitué, est indépendamment un alcool de la Formule D suivante : 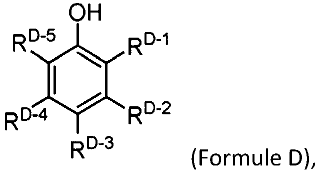
dans lequel dans la Formule D :
R^{D-1}, R^{D-2}, R^{D-3}, R^{D-4}, et R^{D-5} sont indépendamment les uns des autres choisis dans le groupe constitué par H, OH, un groupe alkyle en C₁-C₆, O(C₁-C₆-alkyle), C(O)(C₁-C₆-alkyle), C(O)O(C₁-C₆-alkyle), F, CI, Br, I, et CN.

2. Procédé selon la revendication 1, dans lequel l'oligonucléotide cible O^{T} comprend un oligonucléotide O-1 de premier cycle, et le procédé comprend l'étape suivante (a) et un premier cycle de couplage comprenant les étapes suivantes (b) à (e) :
(a) fourniture d'un composant C-0 choisi dans le groupe constitué par un nucléoside et un oligonucléotide, le composant C-0 étant lié de manière covalente à un support solide et comprenant un groupement hydroxyle de squelette protégé par un groupe protecteur di(p-méthoxyphényl)phénylméthyle PG-0 ;
(b) incubation du composant C-0 de l'étape (a) avec un mélange de déprotection M-b, clivant ainsi le groupe protecteur PG-0 du composant C-0, de façon à obtenir un composant C-0^{#} ayant un groupe hydroxyle de squelette libre ;
(c) fourniture d'un bloc constitutif B-1 choisi dans le groupe constitué par un nucléoside et un oligonucléotide, le bloc constitutif B-1 comprenant un groupe hydroxyle de squelette protégé par un groupe protecteur di(p-méthoxyphényl)phénylméthyle PG-1 et un groupe au phosphore lié de façon covalente par l'intermédiaire de son atome de phosphore à un atome d'oxygène du squelette du bloc constitutif B-1 ;
(d) mise en réaction du composant C-0^{#} de l'étape (b) avec le bloc constitutif B-1 de l'étape (c) dans des conditions appropriées pour former une liaison covalente entre ledit groupe hydroxyle de squelette libre du composant C-0^{#} et l'atome de phosphore dudit groupement au phosphore du bloc constitutif B-1, de façon à obtenir un oligonucléotide de premier cycle O-1 ;
(e) éventuellement, incubation de l'oligonucléotide O-1 de premier cycle obtenu dans l'étape (d) avec un agent oxydant ou sulfurant, convertissant ainsi tout atome de P (III) dans ledit oligonucléotide O-1 de premier cycle en atomes de P (V) ;
dans lequel dans l'étape (b), ledit mélange de déprotection M-b est une composition liquide C telle que définie selon la revendication 1.

3. Procédé selon la revendication 2, dans lequel l'oligonucléotide cible O^{T} comprend un oligonucléotide O-n de n-ième cycle, et le procédé comprend en outre la réalisation de (n-1) itérations d'un cycle de couplage comprenant les étapes suivantes (b') à (e'), où n est un entier dans la plage de 2 à 99, qui désigne le nombre total de cycles de couplage effectués pour obtenir l'oligonucléotide O-n de n-ième cycle, et chaque cycle de couplage individuel comprenant les étapes (b') à (e') suivantes est identifié par un numéro de série x, qui s'exécute par incréments de 1 de 2 à n :
(b') incubation de l'oligonucléotide O-(x-1) de (x-1)-ième cycle obtenu dans le cycle de couplage précédent avec un mélange de déprotection M-b', clivant ainsi le groupe protecteur di(p-méthoxyphényl)phénylméthyle PG-(x-1) de l'oligonucléotide O-(x-1) de (x-1)-ième cycle, de façon à obtenir un oligonucléotide O-(x-1)^{#} de (x-1)-ième cycle ayant un groupe hydroxyle de squelette libre ;
(c') la fourniture d'un bloc constitutif B-x choisi dans le groupe constitué par un nucléoside et un oligonucléotide, le bloc constitutif B-x comprenant un groupe hydroxyle de squelette protégé par un groupe protecteur di(p-méthoxyphényl)phénylméthyle PG-x et un groupement au phosphore lié de façon covalente par l'intermédiaire de son atome de phosphore à un atome d'oxygène du squelette du bloc constitutif B-x ;
(d') mise en réaction de l'oligonucléotide O-(x-1))^{#} de (x-1)-ième cycle obtenu dans l'étape (b') avec le bloc constitutif B-x de l'étape (c') dans des conditions adaptées pour former une liaison covalente entre ledit groupe hydroxyle de squelette libre de l'oligonucléotide (O-(x-1))^{#} de (x-1)-ième cycle et l'atome de phosphore dudit groupement au phosphore du bloc constitutif B-x, obtenant ainsi un oligonucléotide O-x de x-ième cycle ;
(e') éventuellement, incubation de l'oligonucléotide O-x de x-ième cycle obtenu dans l'étape (d') avec un agent oxydant ou sulfurant, convertissant ainsi tout atome de P (III) dans ledit oligonucléotide O-x de x-ième cycle en atomes de P (V) ;
dans lequel dans au moins une itération de l'étape (b'), ledit mélange de déprotection M-b' est une composition liquide C telle que définie selon la revendication 1.

4. Procédé selon l'une quelconque des revendications 2 à 3, dans lequel :
- le groupement au phosphore du bloc constitutif B-1 et chaque bloc constitutif B-x est indépendamment choisi dans le groupe constitué d'un groupement phosphoramidite et d'un groupement monoester de H-phosphonate ;
- dans chaque cycle de couplage, dans lequel ledit groupement au phosphore du bloc constitutif B-1 ou du bloc constitutif B-x est un groupement phosphoramidite, l'étape (e) ou (e') est réalisée ; et
- au moins dans le cycle de couplage final, l'étape (e) ou (e') est réalisée.

5. Procédé selon l'une quelconque des revendications 1 à 4, dans lequel ledit nucléoside ou oligonucléotide, qui est lié de manière covalente à un support solide, selon la revendication 1 et le composant C-0 selon la revendication 2 est un composé de Formule I suivante : 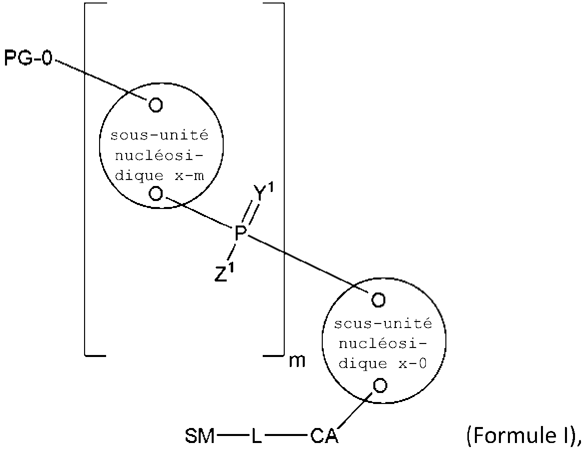 dans lequel dans la Formule I :
chaque atome d'oxygène (O) représenté dans chaque sous-unité nucléosidique x-0 à x-m représente l'atome d'oxygène d'un groupement hydroxyle de la sous-unité nucléosidique respective ;
chacune des sous-unités nucléosidiques x-0 à x-m peut être identique ou différente ;
PG-0 est un groupe protecteur di(p-méthoxyphényl)phénylméthyle ;
m est un entier égal ou supérieur à 0 ;
Y¹ est choisi indépendamment pour chaque motif répétitif m dans le groupe constitué par O et S ;
Z¹ est choisi indépendamment pour chaque motif répétitif m dans le groupe constitué par O-R^{z-1} et S-R^{z-1} ;
R^{z-1} est un groupe protecteur, qui peut être identique ou différent pour chaque motif répétitif m ;
CA est un groupement de coiffage ou une liaison chimique covalente ;
L est un groupement lieur ou une liaison chimique covalente ; et
SM est un support solide.

6. Procédé selon l'une quelconque des revendications 2 à 5, dans lequel chacun des blocs constitutifs B-1 et B-x est un composé de Formule II-1 suivante : 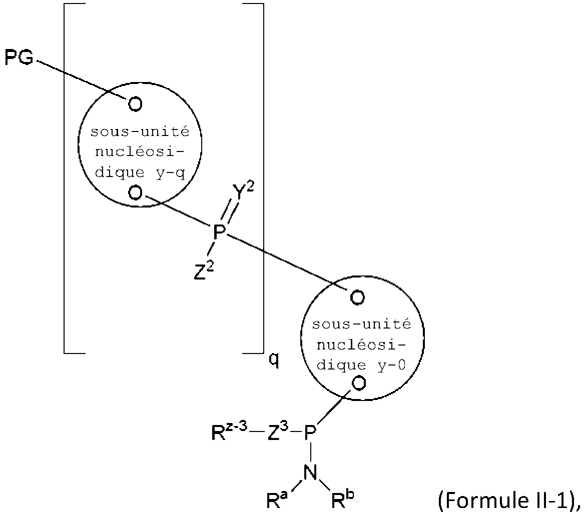 dans lequel dans la Formule II-1 :
chaque atome d'oxygène (O) représenté au sein de chaque sous-unité nucléosidique y-0 à y-q représente l'atome d'oxygène d'un groupement hydroxyle de la sous-unité nucléosidique respective ;
chaque sous-unité nucléosidique y-0 à y-q peut être identique ou différente ;
PG est le groupe protecteur PG-1 ou PG-x et est un groupe protecteur di(p-méthoxyphényl)phénylméthyle ;
q est un entier égal ou supérieur à 0 ;
Y² est sélectionné indépendamment pour chaque motif répétitif q dans le groupe constitué par O et S ;
Z² est sélectionné indépendamment pour chaque motif répétitif q dans le groupe constitué par O-R^{z-2}, S-R^{z-2} ;
R^{z-2} est un groupe protecteur, qui peut être identique ou différent pour chaque motif répétitif q ;
Z³ est choisi dans le groupe constitué par O et S ; et
R^{z-3} est un groupe protecteur ;
chacun parmi R^{a} et R^{b} est indépendamment un groupe alkyle en C₁-C₆, R^{a} et R^{b} pouvant être identiques ou différents et pouvant être également liés l'un à l'autre pour former un groupement amine cyclique aliphatique à 5 ou 6 chaînons conjointement avec l'atome d'azote auquel R^{a} et R^{b} sont liés ;
et dans lequel l'étape (e) ou l'étape (e') est réalisée dans chaque cycle de couplage.

7. Procédé selon l'une quelconque des revendications 2 à 6, dans lequel
- le premier cycle de couplage comprend en outre une étape (f) de mise en réaction de groupes hydroxyle libres avec un agent de blocage, l'étape (f) étant réalisée après l'étape (d) ou après l'étape (e) ; et/ou
- au moins une itération des (n-1) itérations du cycle de couplage comprenant les étapes (b') à (e') comprend en outre une étape (f') de mise en réaction de groupes hydroxyle libres avec un agent de blocage, l'étape (f') étant réalisée après l'étape (d') ou après l'étape (e').

8. Procédé selon l'une quelconque des revendications 2 et 4 à 7, dans lequel
- le procédé comprend en outre une étape (g) d'incubation de l'oligonucléotide O-1 de premier cycle avec un mélange de déprotection M-g, clivant ainsi le groupe protecteur PG-1 de l'oligonucléotide O-1 de premier cycle, de sorte à obtenir un oligonucléotide (O-1)^{#} de premier cycle ayant un groupe hydroxyle de squelette libre ; et/ou
- le procédé comprend en outre une étape (h) de clivage de l'oligonucléotide O-1 ou (O-1)^{#} de premier cycle, du support solide ;
et dans lequel si les deux étapes (g) et (h) sont effectuées, elles peuvent être effectuées dans n'importe quel ordre.

9. Procédé selon l'une quelconque des revendications 3 à 7, dans lequel
- le procédé comprend en outre une étape (g') d'incubation de l'oligonucléotide On de n-ième cycle avec un mélange de déprotection M-g', clivant ainsi le groupe protecteur PG-n de l'oligonucléotide O-n de n-ième cycle, de manière à obtenir un oligonucléotide (O-n)^{#} de n-ième cycle ayant un groupe hydroxyle de squelette libre ; et/ou
- le procédé comprend en outre une étape (h') de clivage de l'oligonucléotide O-n ou (O-n)^{#} de n-ième cycle du support solide ;
et dans lequel si les deux étapes (g') et (h') sont réalisées, elles peuvent être effectuées dans n'importe quel ordre.

10. Procédé selon l'une quelconque des revendications 1 à 9, dans lequel au moins les étapes (b) et (b') sont réalisées dans un réacteur discontinu, ou dans lequel au moins les étapes (b) et (b') sont réalisées dans un réacteur à colonne et le débit de la composition liquide C à travers le réacteur à colonne est inférieur à 300 cm/h.

11. Procédé selon l'une quelconque des revendications 1 à 10, dans lequel
- le groupement hydroxyle de squelette protégé par ledit groupe protecteur PG-0 fait partie d'un groupement nucléosidique comprenant une base nucléique de type purine ; et
- dans au moins une itération du cycle de couplage comprenant les étapes (b') à (e'), dans lesquelles ledit groupement protecteur PG-(x-1) fait partie d'un groupement nucléosidique comprenant une base nucléique de type purine, le mélange de déprotection M-b' est une composition liquide C.

12. Procédé selon l'une quelconque des revendications 1 à 11, dans lequel la synthèse est effectuée sur une échelle d'au moins 100 mmol de l'oligonucléotide cible O^{T}.

13. Procédé selon l'une quelconque des revendications 1 à 12, dans lequel la composition liquide C comprend ledit au moins un alcool selon la Formule D en une concentration molaire de 0,45 à 5,60 mol/L.

14. Procédé selon l'une quelconque des revendications 1 à 13, dans lequel ledit solvant compris dans la composition liquide C est un solvant aprotique non halogéné.

15. Procédé selon l'une quelconque des revendications 8 et 10 à 14, dans lequel le procédé comprend en outre une étape (i) de modification de l'oligonucléotide de premier cycle O-1 ou (O-1)^{#}.

16. Procédé selon l'une quelconque des revendications 9 à 14, dans lequel le procédé comprend en outre une étape (i') de modification de l'oligonucléotide On ou (O-n)^{#} de n-ième cycle.

17. Composition comprenant
- un oligonucléotide lié de manière covalente à un support solide et comprenant un groupement hydroxyle protégé par un groupe protecteur di(p-méthoxyphényl)phénylméthyle, et
- une composition liquide C comprenant un solvant, un acide protique ayant un pKa égal ou inférieur à 4 et au moins un alcool, dans laquelle un ou plusieurs groupes hydroxyle sont liés de manière covalente directement à un groupement phényle éventuellement substitué,
dans laquelle
- ledit acide protique compris dans la composition liquide C est choisi dans le groupe constitué par un acide carboxylique, un acide sulfonique, un acide minéral, une amine aliphatique, aromatique ou hétéroaromatique protonée, dont la forme protonée possède un pKa dans la plage de 1 à 4, et des mélanges correspondants ; et
- chacun dudit au moins un alcool, dans lequel un ou plusieurs groupes hydroxyle sont liés de façon covalente directement à un groupement phényle éventuellement substitué, est indépendamment un alcool de la Formule D suivante : 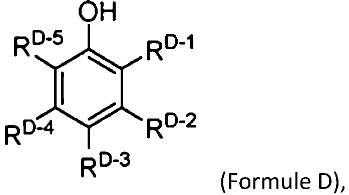
dans laquelle dans la Formule D :
R^{D-1}, R^{D-2}, R^{D-3}, R^{D-4}, et R^{D-5} sont indépendamment les uns des autres choisis dans le groupe constitué par H, OH, un groupe alkyle en C₁-C₆, O(C₁-C₆-alkyle), C(O)(C₁-C₆-alkyle), C(O)O(C₁-C₆-alkyle), F, CI, Br, I, et CN ;
de préférence dans laquelle la composition et/ou un ou plusieurs composants sont définis tels que dans une ou plusieurs des revendications précédentes,
en particulier dans laquelle la concentration dudit au moins un alcool de Formule D est définie comme selon la revendication 13 et/ou ledit solvant est défini comme selon la revendication 14.
